(19) Europäisches Patentamt
European Patent Office
Office européen des brevets

(11) EP 0 576 357 B1

(12) # FASCICULE DE BREVET EUROPEEN

(45) Date de publication et mention de la délivrance du brevet: **05.03.1997 Bulletin 1997/10**

(51) Int. Cl.$^6$: **C07D 231/14**, C07D 231/12, C07D 231/40

(21) Numéro de dépôt: **93401614.8**

(22) Date de dépôt: **23.06.1993**

(54) **Dérivés du pyrazole, procédé pour leur préparation et compositions pharmaceutiques les contenant**

Pyrazolderivate, Verfahren zu ihrer Herstellung und diese enthaltende pharmazeutische Zusammensetzungen

Pyrazole derivatives, process for their preparation and pharmaceutical compositions containing them

(84) Etats contractants désignés:
**AT BE CH DE DK ES FR GB GR IE IT LI LU NL PT SE**

(30) Priorité: **23.06.1992 FR 9207645**

(43) Date de publication de la demande:
**29.12.1993 Bulletin 1993/52**

(73) Titulaire: **SANOFI**
**75008 Paris (FR)**

(72) Inventeurs:
- **Barth, Francis,**
  **St. James - Ap.B17**
  **F-34080 Montpellier (FR)**
- **Casellas, Pierre**
  **F-34000 Montpellier (FR)**
- **Congy, Christian**
  **F-34980 Saint Gely du Fesc (FR)**
- **Martinez, Serge**
  **F-34000 Montpellier (FR)**
- **Carmona, Murielle**
  **F-34680 Saint Georges d'Orques (FR)**

(74) Mandataire: **Gillard, Marie-Louise et al**
**Cabinet Beau de Loménie**
**158, rue de l'Université**
**75340 Paris Cédex 07 (FR)**

(56) Documents cités:
**EP-A- 0 029 363**
**EP-A- 0 248 594**
**EP-A- 0 418 845**
**EP-A- 0 477 049**

- **JOURNAL OF THE CHEMICAL SOCIETY, PERKIN TRANSACTIONS 1. vol. 21, 1973, LETCHWORTH GB pages 2532 - 2535 G.V. BOYD ET AL. 'Ring Transformations of Morpholinofurans and N-(2,3-Dihydro 2-furilidene) morpholinium salts'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 576 357 B1

Printed by Rank Xerox (UK) Business Services
2.13.16/3.4

## Description

La présente invention concerne de nouveaux dérivés du pyrazole, un procédé pour leur préparation et les compositions pharmaceutiques les contenant.

De nombreux dérivés du pyrazole ont été décrits dans la littérature ; plus particulièrement EP-A-268554 et DE-A-3910248 revendiquent des pyrazoles possédant des propriétés herbicides, EP-A-430186 et JP-A-03031840 revendiquent des composés utiles pour la photographie et EP-A-418845 revendique des pyrazoles pourvus d'activité antiinflammatoire, analgésique et antithrombotique.

EP-A-477 049 décrit des dérivés du pyrazole capables de se lier au récepteur de la neurotensine substitués en position 3 du pyrazole par un groupe

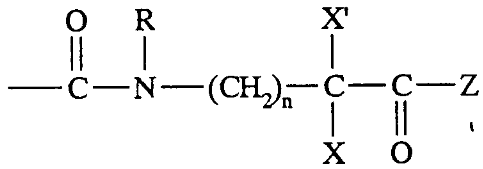

dans lequel notamment R représente l'hydrogène ou un ($C_1$-$C_4$)alkyle ; n vaut de 0 à 3, X et X' forment avec l'atome de carbone auquel ils sont liés un adamantyle et Z représente un hydroxy, un ($C_1$-$C_6$)alcoxy ou un groupe -OPg (Pg = groupe protecteur des acides carboxyliques).

On a maintenant trouvé que les pyrazoles objet de l'invention ont une bonne affinité pour le récepteur des cannabinoïdes et sont de ce fait particulièrement intéressants dans les domaines thérapeutiques où le cannabis est connu pour intervenir.

Le $\Delta^9$-tétrahydrocannabinol ou $\Delta^9$-THC est le principal constituant actif extrait de *Cannabis sativa* (Tuner, 1985 ; In Marijuana 1984, Ed. Harvey, DY, IRL Press, Oxford).

Les effets des cannabinoïdes sont dûs à une interaction avec des récepteurs spécifiques de haute affinité présents au niveau central (Devane et al., Molecular Pharmacology, 1988, *34*, 605-613) et périphérique (Nye et al., The Journal of Pharmacology and Experimental Therapeutics, 1985, *234*, 784-791 ; Kaminski et al., 1992, Molecular Pharmacology, *42*, 736-742).

La caractérisation de ce récepteur a été rendue possible par la mise au point de ligands synthétiques spécifiques tels que le CP 55,940, agoniste analogue du $\Delta^9$-THC.

Les indications thérapeutiques des cannabinoïdes concernent des domaines variés tels que le système immunitaire, le système nerveux central, le système cardiovasculaire ou endocrinien (Hollister, Pharmacological Reviews, 1986, *38*, 1-20, Renv and Sinha, Progress in Drug Research, 1991, *36*, 71-114 et Cannabinoïd receptor expression in human leucocytes, European Journal of Biochemistry, 1993, *214*, 173-180).

Plus particulièrement, les composés pourvus d'une affinité pour le récepteur des cannabinoïdes possèdent une utilité en tant qu'immunomodulateurs, psychotropes, dans les troubles thymiques, le vomissement, la myorelaxation, les neuropathies diverses, les troubles mnésiques, les dyskinésies, la migraine, l'asthme, l'épilepsie, le glaucome, encore dans la chimiothérapie anticancéreuse, dans l'ischémie et l'angor, dans l'hypotension orthostatique et dans l'insuffisance cardiaque.

Ainsi, la présente invention concerne selon un de ses aspects les composés de formule :

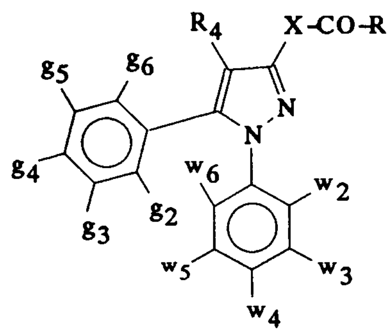

(I)

dans laquelle

- $g_2$, $g_3$, $g_4$, $g_5$, $g_6$ et $w_2$, $w_3$, $w_4$, $w_5$, $w_6$ sont identiques ou différents, et représentent indépendamment l'hydrogène,

un atome de chlore ou de brome, un ($C_1$-$C_3$) alkyle, un ($C_1$-$C_3$) alcoxy, un trifluorométhyle, un groupe nitro et $g_4$ représente éventuellement un groupe phényle;

- $R_4$ représente l'hydrogène ou un ($C_1$-$C_3$) alkyle;
- X représente soit une liaison directe soit un groupe $-(CH_2)_x-N(R_3)-$ dans lequel $R_3$ représente l'hydrogène ou un ($C_1$-$C_3$) alkyle et x représente zéro ou un;
- R représente

  . un groupe $-NR_1R_2$, dans lequel $R_1$ et $R_2$ représentent indépendamment un ($C_1$-$C_6$) alkyle; un radical carbocyclique non aromatique en ($C_3$-$C_{15}$) éventuellement substitué ; un groupe amino ($C_1$-$C_4$) alkyle dans lequel l'amino est éventuellement disubstitué par un ($C_1$-$C_3$) alkyle ; un cycloalkyl($C_1$-$C_3$)alkyle dans lequel le cycloalkyle est en ($C_3$-$C_{12}$); un phényle non substitué ou substitué une ou plusieurs fois par un halogène, par un ($C_1$-$C_5$) alkyle, ou par un ($C_1$-$C_5$) alcoxy ; un phényl ($C_1$-$C_3$) alkyle ; un diphényl ($C_1$-$C_3$) alkyle; un naphtyle ; un anthracényle ; un radical hétérocyclique saturé de 5 à 8 chaînons non substitué ou substitué par un ($C_1$-$C_3$) alkyle, un hydroxyle ou un benzyle; un 1-adamantylméthyle ; un hétérocyle aromatique non substitué ou substitué une ou plusieurs fois par un halogène, un ($C_1$-$C_5$) alkyle ou un ($C_1$-$C_5$) alcoxy ; un ($C_1$-$C_3$) alkyle substitué par un hétérocycle aromatique non substitué ou substitué une ou plusieurs fois par un halogène, un ($C_1$-$C_5$) alkyle ou un ($C_1$-$C_5$) alcoxy; ou bien $R_1$ est l'hydrogène et $R_2$ est tel que défini ci-dessus ; ou bien encore $R_1$ et $R_2$ constituent avec l'atome d'azote auquel ils sont liés, un radical hétérocyclique saturé de 5 à 8 chaînons, ledit radical hétérocyclique étant autre que morpholinyle lorsque $w_2$, $w_3$, $w_4$, $w_5$, $w_6$, $g_2$, $g_3$, $g_4$, $g_5$ et $g_6$ sont tous hydrogène;
  . un groupe $R_2$ tel que défini ci-dessus lorsque X représente $-(CH_2)_xN(R_3)-$;
  . un groupe $R_5$ lorsque X représente une liaison directe, $R_5$ étant représenté par un ($C_1$-$C_3$) alkyle ; un ($C_3$-$C_{12}$) cycloalkyle non substitué ou substitué par un ($C_1$-$C_5$) $C_5$) alkyle ; un phényl ($C_1$-$C_3$) alkyle non substitué ou substitué par un halogène ou par un ($C_1$-$C_5$) alkyle ; un cycloalkyl ($C_1$-$C_3$) alkyle dans lequel le cycloalkyle est en $C_3$-$C_{12}$ et est non substitué ou substitué par un ($C_1$-$C_5$) alkyle ; un 2-norbornylméthyle ;

ou un de leurs sels éventuels.

Les radicaux carbocycliques non aromatiques en $C_3$-$C_{15}$ comprennent les radicaux mono ou polycycliques, condensés ou pontés, saturés ou insaturés, éventuellement terpéniques. Ces radicaux sont éventuellement mono- ou poly-substitués, le ou lesdits substituants étant différents d'un groupe carbonyle substitué. Avantageusement les radicaux monocycliques sont substitués par au moins un groupe choisi parmi les groupes ($C_1$-$C_5$) alkyle, ($C_1$-$C_5$) alcoxy, les halogènes ou les radicaux hydroxy, étant entendu que dans le cas des terpènes ou des radicaux terpéniques, par exemple bornyle, menthyle ou menthényle, les groupes alkyles du terpène ne sont pas considérés comme des substituants.

Les radicaux monocycliques incluent les cycloalkyles par exemple cyclopropyle, cyclopentyle, cyclohexyle, cycloheptyle, cyclooctyle, cyclododécyle non substitués ou substitués par au moins un groupe ($C_1$-$C_5$) alkyle, un groupe ($C_1$-$C_5$) alcoxy, un halogène ou un groupe hydroxy.

Les radicaux di- ou tricycliques condensés, pontés, ou spiraniques, incluent par exemple les radicaux norbornyle, bornyle, isobornyle, noradamantyle, adamantyle, spiro[5,5]undécanyle, lesdits radicaux étant non substitués ou substitués par un ($C_1$-$C_5$) alkyle.

Par radical hétérocyclique saturé de 5 à 8 chaînons, on entend un radical hétérocyclique non aromatique mono, di ou tricyclique condensé ou ponté, l'hétéroatome étant S, O ou N ou un radical hétérocyclique non aromatique monocyclique contenant un atome d'azote et un atome d'oxygène ou de soufre, lesdits radicaux étant par exemple tétrahydrofuranyle, tétrahydrothiofuranyle, tropyle, morpholinyle, thiomorpholinyle, pipéridinyle, pipérazinyle, pyrrolidinyle, quinuclidinyle: les radicaux 1-pyrrolidinyle, 1-pipéridinyle, 1-hexahydroazépinyle, 4-morpholinyle et 4-thiomorpholinyle étant avantageux.

Les hétérocycles aromatiques peuvent être mono- ou di-cycliques comme par exemple le pyrrolyle, le pyridyle, l'indolyle, quinolinyle, thiazolyle, isoindazolyle, ces hétérocycles aromatiques étant non substitués ou substitués par exemple par des halogènes, des ($C_1$-$C_5$) alkyles, des ($C_1$-$C_5$) alcoxy. Les hétérocycles aromatiques préférés sont le pyridyle, le pyrrole, l'indole, les radicaux 2-indolyle et 3-indolyle étant particulièrement préférés.

Dans la formule (I) ci-dessus, de préférence au moins un des substituants $w_2$, $w_3$, $w_4$, $w_5$, $w_6$, $g_2$, $g_3$, $g_4$, $g_5$ et $g_6$ est autre que l'hydrogène. Dans la formule (I) ci-dessus, lorsque R représente un groupe $NR_1R_2$, de préférence:

- $R_1$ est l'hydrogène ou un groupe alkyle en $C_1$-$C_6$ et $R_2$ est tel que défini ci-dessus pour (I) ; ou
- $R_1$ et $R_2$ représentent chacun un groupe alkyle en $C_1$-$C_6$, ou un groupe cycloalkyle en $C_3$-$C_6$ ; ou
- $R_1$ est l'hydrogène ou un groupe alkyle en $C_1$-$C_6$ et $R_2$ est un groupe cycloalkyl ($C_1$-$C_3$)alkyle dans lequel le cycloalkyle est en $C_3$-$C_{12}$, un radical carbocyclique non aromatique en ($C_3$-$C_{15}$) non substitué ou substitué comme indiqué précédemment, un phényle non substitué ou substitué une ou plusieurs fois par un halogène, par un ($C_1$-

$C_3$)alkyle ou par un ($C_1$-$C_3$)alcoxy ; un phényl ($C_1$-$C_3$)alkyle ou un ($C_1$-$C_3$) alkyle substitué par un 2- ou 3-indolyle.

De façon particulièrement préférée, lorsque, dans la formule (I) R représente un groupe $-NR_1R_2$, $R_1$ est l'hydrogène ou un alkyle en ($C_1$-$C_6$) et $R_2$ est un radical carbocyclique non aromatique en ($C_3$-$C_{15}$), un cycloalkyl($C_1$-$C_3$)alkyle où le cycloalkyle est en $C_3$-$C_6$, un 2- ou 3-indolyl($C_1$-$C_3$)alkyle.

Les groupes alkyle préférés sont les groupes méthyle, éthyle, propyle et isopropyle.

Dans la formule (I) ci-dessus, R est avantageusement un groupe $-NR_1R_2$, de préférence choisi parmi les radicaux (1) à (74) ci-après.

Lorsque $R_1$ et $R_2$, avec l'atome d'azote auquel ils sont liés, représentent un radical hétérocyclique saturé, celui-ci est de préférence à 5, 6 ou 7 chainons et peut contenir un autre hétéroatome, notamment l'oxygène ou le soufre, par exemple une pyrrolidine, une pipéridine, une hexahydroazépine, une morpholine ou une thiomorpholine, avec la limitation précisée ci-dessus.

Les radicaux représentés par R tel que défini pour (I) sont de préférence des radicaux choisis parmi :

(1) propylamino
(2) butylamino
(3) isopropylamino
(4) dipentylamino
(5) 2-(N,N-diéthylamino)éthylamino
(6) benzylamino
(7) 2-phényléthylamino
(8) 3-phénylpropylamino
(9) 3,3-diphénylpropylamino
(10) phénylamino
(11) 3-chlorophénylamino
(12) 4-méthylphénylamino
(13) cyclopropylamino
(14) cyclopentylamino
(15) cyclohexylamino
(16) cycloheptylamino
(17) cyclooctylamino
(18) cyclododécylamino
(19) 2-méthylcyclohexylamino
(20) 3-méthylcyclohexylamino
(21) cis 4-méthylcyclohexylamino
(22) trans 4-méthylcyclohexylamino
(23) cis 4-tertiobutylcyclohexylamino
(24) trans 4-tertiobutylcyclohexylamino
(25) 4-hydroxycyclohexylamino
(26) 2-méthoxycyclohexylamino
(27) 4-éthylcyclohexylamino
(28) 2,6-diméthylcyclohexylamino
(29) N-méthylcyclohexylamino
(30) N,N-dicyclohexylamino
(31) endo-2-norbornylamino (ou endo-bicyclo[2.2.1]heptan-2-amino)
(32) exo-2-norbornylamino (ou exo-bicyclo[2.2.1]heptan-2-amino)
(33) 1-adamantylamino
(34) 2-adamantylamino
(35) 1-noradamantylamino
(36) (1*R*)-bornylamino
(37) (1*R*)-isobornylamino
(38) spiro[5.5]undecanylamino
(39) cyclohexylméthylamino
(40) 1-adamantylméthylamino
(41) (2-tétrahydrofuranyl)méthylamino
(42) 2-(N-méthyl-2-pyrrolyl)éthylamino
(43) 2-(2-pyridinyl)éthylamino
(44) (2-indolyl)méthylamino
(45) N-méthyl(2-indolyl)méthylamino

(46) 2-(3-indolyl)éthylamino
(47) N-méthyl 2-(3-indolyl)éthylamino
(48) 4-(N-benzylpipéridinyl)amino
(49) 3-quinuclidylamino
(50) exo bicyclo[3.2.1]octan-2-amino
(51) bicyclo[2.2.2]octan-2-amino
(52) chloro-3-bicyclo[3.2.1]oct-3-èn-2-amino
(53) bicyclo[2.2.2]oct-2-èn-5-amino
(54) exo bicyclo[3.2.1]octan-3-amino
(55) endo bicyclo[3.2.1]octan-3-amino
(56) endo oxa-7-bicyclo[2.2.1]heptan-2-amino
(57) exo oxa-7-bicyclo[2.2.1]heptan-2-amino
(58) endo-tricyclo[5.2.1.$0^{2,6}$]décan-8-amino
(59) N-éthyl-1-adamantylamino
(60) tricyclo[2.2.1.$0^{2,6}$]heptan-3-amino
(61) bicyclo[3.3.1]nonan-9-amino
(62) endo-triméthyl-1,3,3-bicyclo[2.2.1]heptan-2-amino (ou fenchylamino)
(63) (1*R*,2S-endo) (+) bicyclo[2.2.1]heptan-2-amino
(64) (1*R*,2R-exo) (-) bicyclo[2.2.1]heptan-2-amino
(65) (1*S*,2*R*-endo) (-) bicyclo[2.2.1]heptan-2-amino
(66) (1*S*,2*S*-exo) (+) bicyclo[2.2.1]heptan-2-amino
(67) 1-pipéridinylamino
(68) 1-pyrrolidinylamino
(69) 1-hexahydroazépinylamino
(70) 4-morpholinylamino
(71) 4-thiomorpholinylamino
(72) N-méthyl exo bicyclo[2.2.1]heptan-2-amino
(73) N-éthyl exo bicyclo[2.2.1]heptan-2-amino
(74) N-propyl exo bicyclo[2.2.1]heptan-2-amino

Parmi les produits de formule (I) ci-dessus, ceux répondant à la formule (Ia) ci-après:

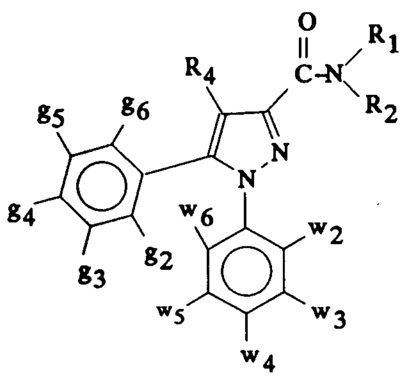

(Ia)

dans laquelle $w_2$, $w_3$, $w_4$, $w_5$, $w_6$, $g_2$, $g_3$, $g_4$, $g_5$, $g_6$ et $R_4$, $R_1$ et $R_2$ sont tels que définis pour (I) sont avantageux. Parmi ces composés de formule (Ia), les composés de formule (Ia') :

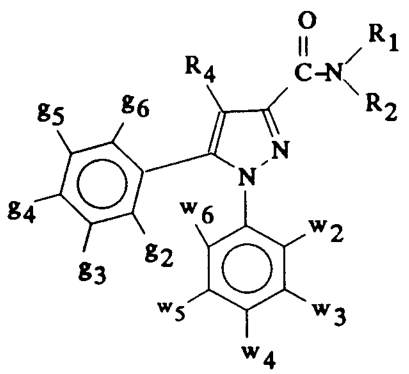

(Ia')

dans laquelle $w_2$, $w_3$, $w_4$, $w_5$, $w_6$, $g_2$, $g_3$, $g_4$, $g_5$, $g_6$ et $R_4$ sont tels que définis ci-dessus pour (I), $R_1$ est l'hydrogène ou un $C_1$-$C_6$ alkyle et $R_2$ est un radical carbocyclique non aromatique en $C_3$-$C_{15}$, ou un radical hétérocyclique saturé de 5 à 8 chaînons choisi parmi 1-pyrrolidinyle, 1-pipéridinyle, 1-hexahydroazépinyle, 4-morpholinyle et 4-thiomorpholinyle et leurs sels, sont particulièrement avantageux.

Parmi les produits de formule (I), ceux répondant aux formules (Ia), (Ib), (Ic), (Id), (Ie) et (If) ci-dessous, où au moins l'un des substituants $w_2$, $w_3$, $w_4$, $w_5$, $w_6$, $g_2$, $g_3$, $g_4$, $g_5$ et $g_6$ est autre que l'hydrogène, $R_1$ est l'hydrogène ou un alkyle en $C_1$-$C_6$, $R_2$ est tel que défini ci-dessus, $R_3$ est l'hydrogène ou un groupe alkyle en $C_1$-$C_3$ , $R_4$ est hydrogène ou méthyle et $R_5$ est un cycloalkyl($C_1$-$C_3$)alkyle, le cycloalkyle étant en $C_3$-$C_6$ ou un phényl($C_1$-$C_3$)alkyle non substitué ou substitué sur le cycle aromatique par un groupe méthyle ou un atome de fluor ou de chlore, et leurs sels éventuels, sont particulièrement avantageux.

Dans ces derniers produits particulièrement avantageux,

- lorsque $R_1$ représente un alkyle en $C_1$-$C_6$, les groupes méthyle, éthyle, propyle et isopropyle sont préférés;
- lorsque $R_3$ est un alkyle en $C_1$-$C_3$, le groupe méthyle est préféré;
- les groupes $R_2$ préférés sont les radicaux carbocycliques non aromatiques en $C_3$-$C_{15}$ non substitués ou substitués par un alkyle en $C_1$-$C_4$, notamment méthyle, éthyle, propyle, isopropyle ou t-butyle, ou par deux ou trois groupes méthyle, par exemple les radicaux méthyl-, éthyl- ou t-butylcyclohexyles ou les radicaux diméthyl-ou triméthylcyclohexyles; les radicaux cycloalkyl($C_1$-$C_3$)alkyles où le cycloalkyle est en $C_3$-$C_6$ ; les radicaux alkyle en $C_1$-$C_3$ substitués par un groupe 2- ou 3-indolyle; les radicaux 2- et 3-indolyle ; et les radicaux 1-pyrrolidinyle, 1-pipéridinyle, 1-hexahydroazépinyle, 4-morpholinyle, 4-thiomorpholinyle;
- les groupes $R_5$ préférés sont les radicaux cyclohexylméthyle, cyclohexyléthyle, benzyle, 4-méthylbenzyle et phénéthyle.

Parmi les produits de formule (I) ci-dessus, ceux représentés par la formule (i):

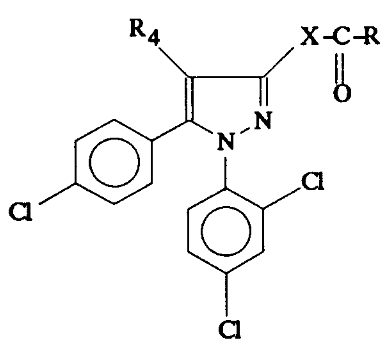

(i)

dans laquelle $R_4$, X et R sont tels que définis ci-dessus pour (I), et leurs sels, sont très avantageux, notamment lorsque $R_4$ représente l'hydrogène ou un groupe méthyle ou lorsque $R_4$ représente l'hydrogène ou un groupe méthyle et X représente une liaison directe.

Les composés de formule (i) dans laquelle $R_4$ est l'hydrogène ou un groupe méthyle, X est une liaison directe et R représente un groupe $-NR_1R_2$ dans lequel $R_1$ est l'hydrogène ou un groupe méthyle et $R_2$ est un radical carbocyclique non aromatique en $C_3$-$C_{15}$ ou un radical hétérocyclique saturé de 5 à 8 chaînons choisi parmi 1-pyrrolidinyle, 1-pipéridinyle, 1-hexahydroazépinyle, 4-morpholinyle et 4-thiomorpholinyle et leurs sels, sont particulièrement préférés.

Egalement particulièrement préférés sont les composés de formule (i) dans laquelle $R_4$ est l'hydrogène ou un

groupe méthyle, X est $-(CH_2)_x-N(R_3)-$ et R est $-NR_1R_2$, x étant zéro ou un, $R_1$ étant l'hydrogène, $R_3$ étant l'hydrogène ou un groupe méthyle et $R_2$ étant un phényle non substitué ou substitué par un ou deux atomes d'halogène, un groupe $(C_1-C_5)$alkyle ou un groupe $(C_1-C_5)$alcoxy ou un radical carbocyclique non aromatique en $C_3-C_{15}$, et leurs sels.

Parmi les composés de formule (I), ceux répondant à la formule (ii):

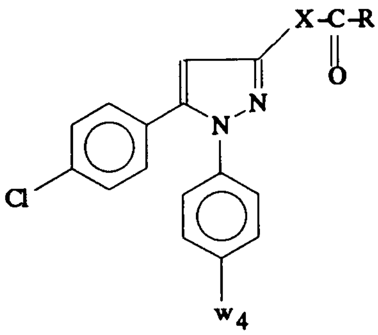

(ii)

dans laquelle X et R sont tels que définis ci-dessus pour (I) et $w_4$ est un groupe méthyle ou méthoxy, et leurs sels sont également avantageux, en particulier ceux de formule (ii) dans laquelle $w_4$ est un groupe méthyle ou méthoxy, X représente une liaison directe et R représente un groupe $-NR_1R_2$ dans lequel $R_1$ représente l'hydrogène ou un groupe méthyle et $R_2$ représente un radical carbocyclique non aromatique en $C_3-C_{15}$, et leurs sels.

Une sous-classe avantageuse comprend les composés de formule (ii) dans laquelle $w_4$ est un groupe méthyle ou méthoxy, X représente un groupe $-(CH_2)_x-N(R_3)-$ dans lequel x est zéro ou un, $R_3$ représente l'hydrogène ou un groupe méthyle, et R est un groupe $-NR_1R_2$ dans lequel $R_1$ représente l'hydrogène et $R_2$ représente un phényle non substitué ou substitué par un ou deux atomes d'halogène, un groupe $(C_1-C_5)$alkyle ou un $(C_1-C_5)$alcoxy ou un radical carbocyclique non aromatique en $C_3-C_{15}$ ou leurs sels.

D'autres composés intéressants selon la présente invention sont ceux de formule (I) dans laquelle $w_2$, $w_3$, $w_4$, $w_5$ $w_6$, $g_2$, $g_3$, $g_4$, $g_5$, $g_6$, $R_4$ et X sont tels que définis ci-dessus pour (I) et R représente un groupe $-NR_1R_2$ dans lequel $R_1$ est l'hydrogène ou un groupe $(C_1-C_6)$alkyle et $R_2$ est un groupe 2- ou 3-indolyl$(C_1-C_3)$alkyle ou un groupe 2- ou 3-indolyle et leurs sels.

Parmi ces derniers, les produits de formule (iii):

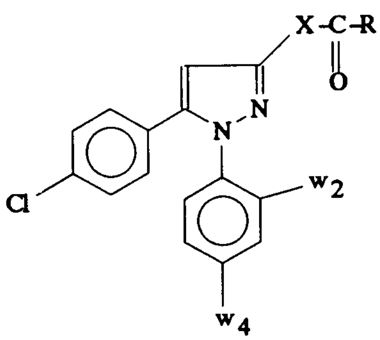

(iii)

dans laquelle X est tel que défini ci-dessus pour (I), R est un groupe $-NR_1R_2$ dans lequel $R_1$ représente l'hydrogène ou un $(C_1-C_6)$alkyle et $R_2$ est un groupe 2-ou 3-indolyl$(C_1-C_3)$alkyle ou un groupe 2- ou 3-indolyle et soit $w_2$ est l'hydrogène et $w_4$ est un groupe méthyle ou méthoxy soit $w_2$ et $w_4$ représentent un atome de chlore, et leurs sels, sont particulièrement intéressants.

Des produits inclus dans la formule (I) ci-dessus, également intéressants sont ceux de formule (iv) :

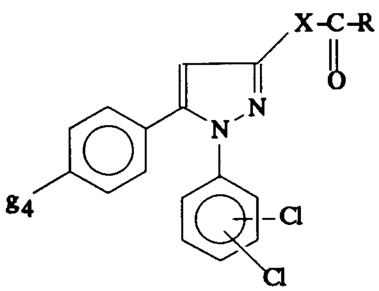

(iv).

dans laquelle X et R sont tels que définis ci-dessus pour (I) et $g_4$ représente un atome de brome, un méthyle ou un trifluorométhyle et leurs sels.

Dans les produits préférés de formule (iv), les deux atomes de chlore sont dans les positions 2,3 ; 2,4 ; 2,5 ou 3,4 et dans ces produits préférés de formule (iv), ceux dont X est une liaison directe et R est un groupe $-NR_1R_2$ où $R_1$ est l'hydrogène ou un alkyle en $C_1$-$C_6$ et $R_2$ est un radical carbocyclique non aromatique contenant de 3 à 15 atomes de carbone sont particulièrement préférés.

Les sels éventuels des composés selon la présente invention,notamment de ceux ayant les formules (I), (Ia'), (i), (ii), (iii), et (iv) ci-dessus et (Ia), (Ib), (Ic), (Id), (Ie) et (If) ci-dessous, comprennent aussi bien ceux avec des acides minéraux ou organiques qui permettent une séparation ou une cristallisation convenable des produits, tels que l'acide picrique ou l'acide oxalique, que ceux qui forment des sels pharmaceutiquement acceptables tels que le chlorhydrate, le bromhydrate, le sulfate, l'hydrogénosulfate, le dihydrogénophosphate, le méthanesulfonate, le méthylsulfate, le maléate, le fumarate, le 2-naphtalènesulfonate, le glyconate, le gluconate, le citrate, l'iséthionate.

Selon un autre de ses aspects, la présente invention concerne un procédé pour la préparation des composés (I) caractérisé en ce que l'on traite un dérivé de l'acide 3-pyrazolecarboxylique de formule:

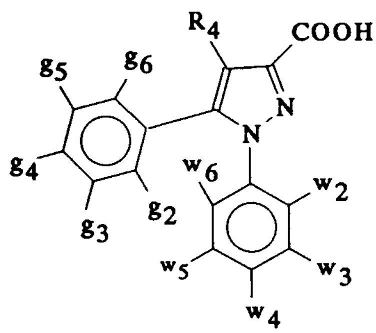

(IIa)

dans laquelle $w_2$, $w_3$, $w_4$, $w_5$ $w_6$, $g_2$, $g_3$, $g_4$, $g_5$, $g_6$ et $R_4$ sont tels que définis pour (I) ou une de ses formes activées, esters ou chlorures d'acides

. soit avec une amine de formule $HNR_1R_2$ dans laquelle $R_1$ et $R_2$ sont tels que définis pour (I) pour obtenir les amides de formule:

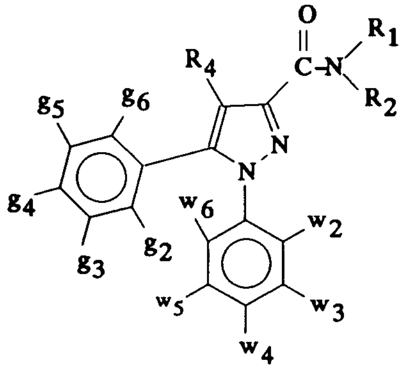

(Ia)

dans laquelle $w_2$, $w_3$, $w_4$, $w_5$ $w_6$, $g_2$, $g_3$, $g_4$, $g_5$, $g_6$, $R_4$, $R_1$ et $R_2$ sont tels que définis pour (I),

. soit éventuellement avec une amine primaire $R_3NH_2$ dans laquelle $R_3$ est tel que défini pour (I) pour obtenir les amides intermédiaires (V) de formule:

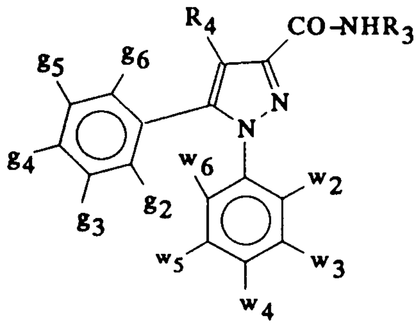

(V)

dans laquelle $w_2$, $w_3$, $w_4$, $w_5$, $w_6$, $g_2$, $g_3$, $g_4$, $g_5$, $g_6$, $R_4$ et $R_3$ sont tels que définis pour (I) pour obtenir par réduction par un hydrure métallique les amines intermédiaires de formule:

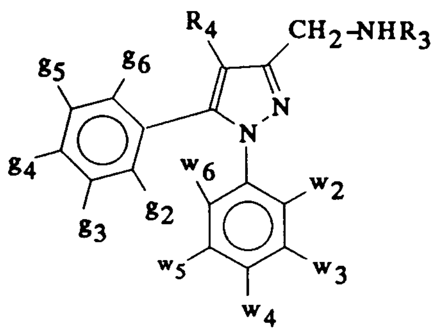

(VI)

dans laquelle $w_2$, $w_3$, $w_4$, $w_5$ $w_6$, $g_2$, $g_3$, $g_4$, $g_5$, $g_6$, $R_4$ et $R_3$ sont tels que définis pour (I), qui sont transformées en amide ou en urée de formule:

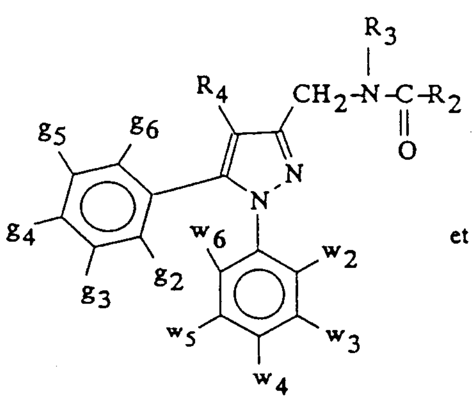

(Ib)

et

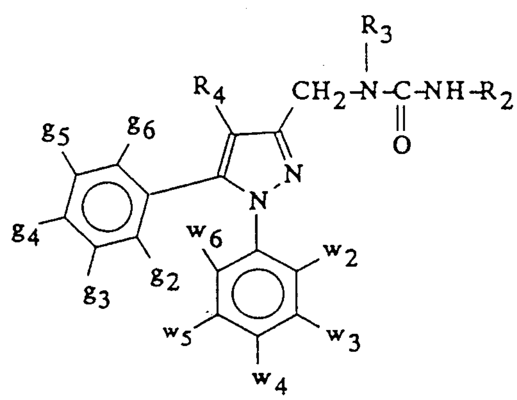

(Ic)

dans lesquelles $w_2$, $w_3$, $w_4$, $w_5$, $w_6$, $g_2$, $g_3$, $g_4$, $g_5$, $g_6$, $R_2$, $R_3$ et $R_4$ sont tels que définis pour (I) par réaction avec respectivement un chlorure d'acide de formule $R_2COCl$ ou un isocyanate de formule $R_2$-N=C=O dans lesquels $R_2$ est tel que défini pour (I),

. soit avec un dérivé de l'azidure de diphénylphosphoryle en milieu basique suivi d'un traitement acide pour obtenir l'amine intermédiaire de formule :

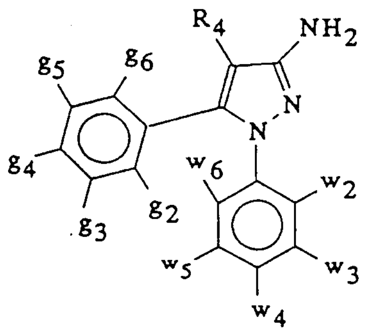

(VII)

dans laquelle $w_2$, $w_3$, $w_4$, $w_5$, $w_6$, $g_2$, $g_3$, $g_4$, $g_5$, $g_6$ et $R_4$ sont tels que définis pour (I), qui est soumis à l'action d'un chlorure d'acide $R_2COCl$ ou d'un isocyanate $R_2$-N=C=O pour obtenir respectivement des amides et les urées de formule :

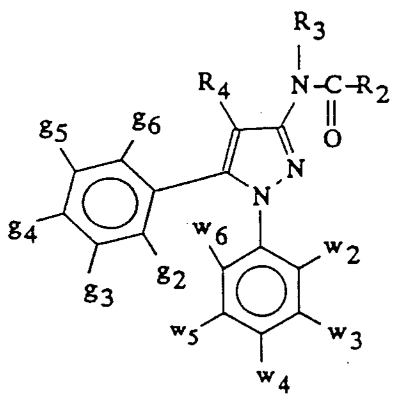

(Id)

et

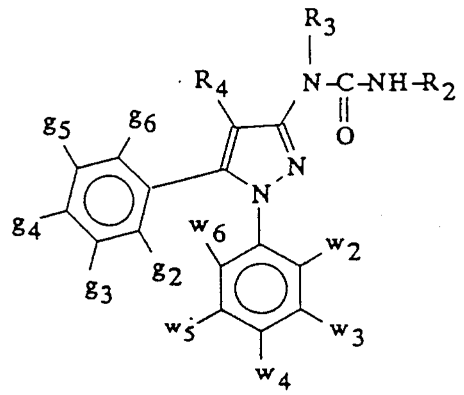

(Ie)

dans lesquelles $w_2$, $w_3$, $w_4$, $w_5$, $w_6$, $g_2$, $g_3$, $g_4$, $g_5$, $g_6$, $R_2$ et $R_4$ sont tels que définis pour (I) et $R_3$ représente l'hydrogène, les mêmes composés pour lesquels $R_3$ est différent de l'hydrogène étant préparés à partir de l'amine primaire (VII) ci-dessus transformée en amine secondaire de formule:

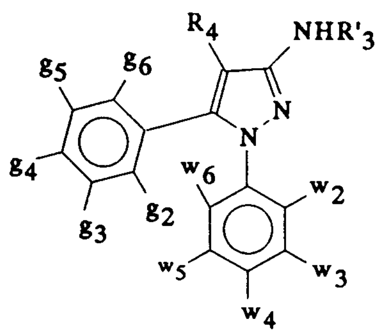

(VIIb)

dans laquelle $w_2$, $w_3$, $w_4$, $w_5$, $w_6$, $g_2$, $g_3$, $g_4$, $g_5$, $g_6$ et $R_4$ sont tels que définis pour (I) et $R'_3$ représente un $(C_1-C_2)$ alkyle, qui sont ensuite soumises à l'action d'un chlorure d'acide $R_2COCl$ ou d'un isocyanate $R_2$-N=C=O pour obtenir les amides et les urées de formule (Id) et (Ie) telles que définies ci-dessus dans lesquelles $R_3$ est différent de l'hydrogène,

. soit avec un réactif organomanganeux $R_5MnX_1$ dans lequel $R_5$ est tel que défini pour (I) et $X_1$ représente un halogène pour obtenir les dérivés cétoniques de formule:

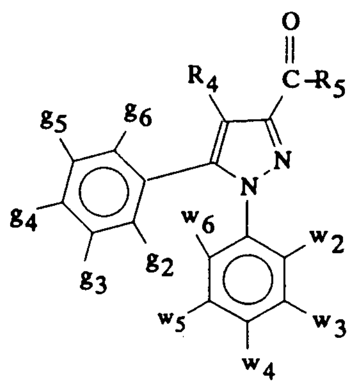

(If)

les composés ainsi obtenus étant éventuellement transformés en l'un de leurs sels.

Selon un mode opératoire préférentiel, les pyrazoles de formule (I) peuvent être synthétisés à partir des esters correspondants, par transformation de la fonction ester en amide, urée ou cétone, via l'acide et le chlorure d'acide.

Lesdits esters sont synthétisés en appliquant la méthode décrite selon Berichte, 1887, *20*, 2185.

Le schéma réactionnel de préparation des composés (I) via leur ester méthylique ou éthylique (Alk = $CH_3$ ou $C_2H_5$) est représenté par le SCHEMA 1 ci-dessous.

## SCHEMA 1

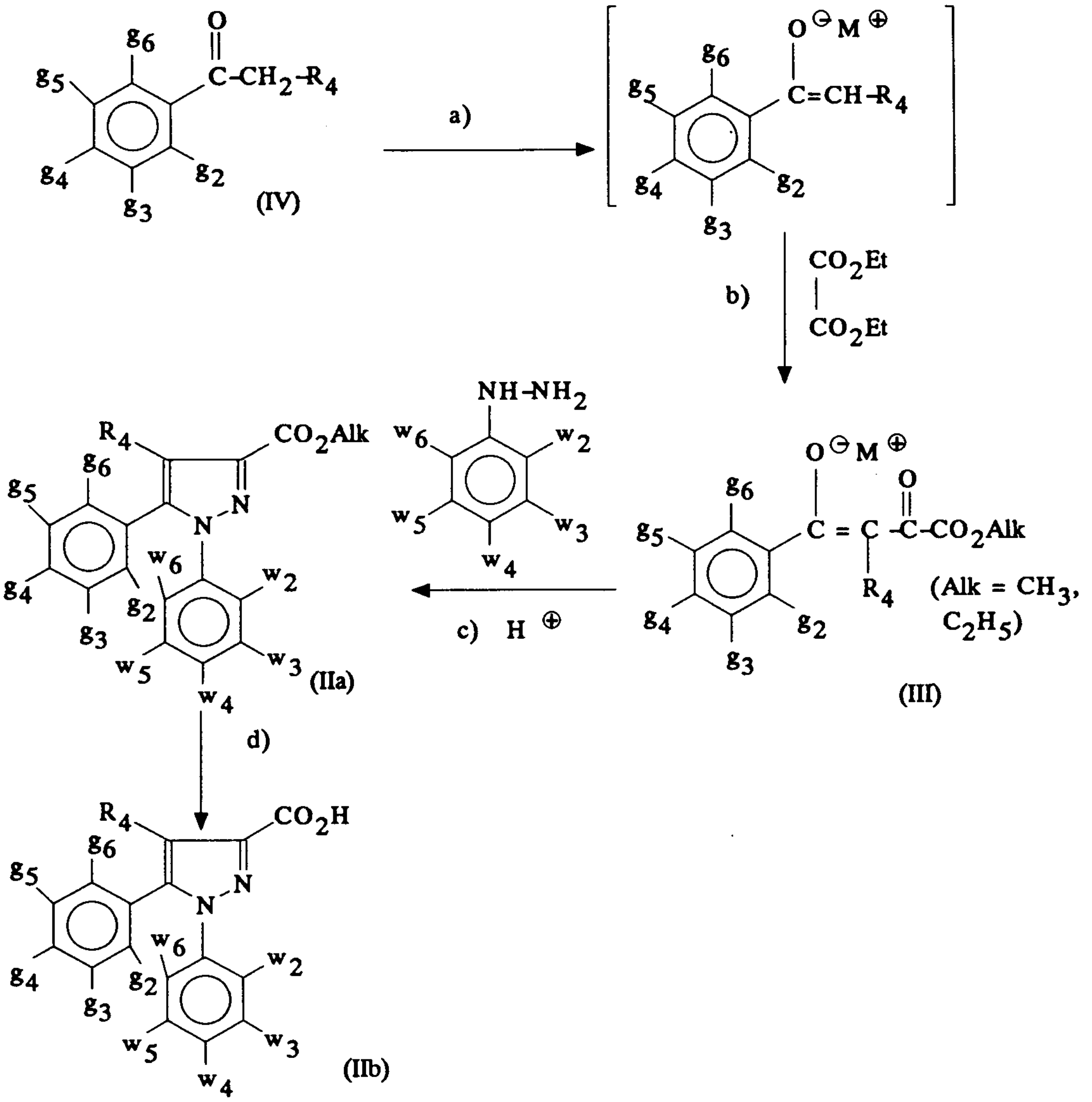


La première étape a) consiste en la préparation d'un sel de métal alcalin d'un dérivé de l'acétophénone de formule (IV) dans laquelle $R_4$ et $g_2$, $g_3$, $g_4$, $g_5$ et $g_6$ sont tels que définis ci-dessus pour (I) sur lequel est ensuite additionnée, une quantité équimolaire d'oxalate de diéthyle (étape b) pour obtenir le cétoester de formule (III).

Dans le cas où $R_4$ = H, le métal alcalin sera préférentiellement le sodium, et le sel du cétoester (III) (Alk = $CH_3$) sera obtenu selon Bull. Soc. Chim. Fr., 1947, *14,* 1098 en utilisant le méthylate de sodium dans le méthanol pour effectuer l'étape a).

Dans le cas où $R_4$ = $CH_3$, le métal alcalin sera préférentiellement le lithium, et le sel du cétoester (III) (Alk = $C_2H_5$) sera obtenu selon J. Heterocyclic. Chem. 1989, *26,* 1389 en utilisant le sel de lithium de l'hexaméthyldisilazane dans l'éther éthylique pour effectuer l'étape a).

Les sels de métal alcalin (III) ainsi préparés et un excès de dérivé de l'hydrazine sont alors chauffés au reflux de l'acide acétique (étape c). Par précipitation dans l'eau glacée on obtient ainsi les 3-pyrazole esters (IIa).

Ces esters (IIa) sont ensuite transformés en leurs acides (IIb) par action d'un agent alcalin comme par exemple l'hydroxyde de potassium puis acidification (étape d).

Dans le SCHEMA 1 ci-dessus, les esters de formule (IIa) dans laquelle $w_2$ et $w_4$ sont un atome de chlore, $w_3$, $w_5$ et $w_6$ sont l'hydrogène, $g_4$ est un atome de chlore, $g_2$, $g_3$, $g_5$ et $g_6$ sont l'hydrogène et pour lesquels Alk représente un ($C_1$-$C_5$)alkyle et les acides (IIb) correspondants, sont des intermédiaires clés nouveaux pour la préparation des composés (i), particulièrement avantageux, et représentent donc un aspect ultérieur de l'invention ; ces composés répondent aux formules (II'a) ou (II'b).

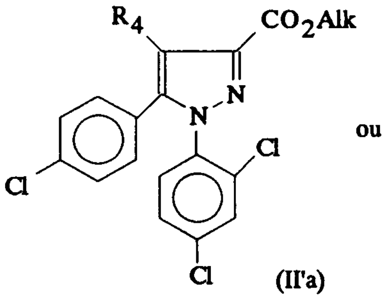
(II'a)

ou

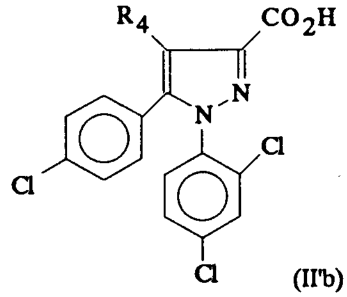
(II'b)

Lorsque X est représenté par une liaison directe, les amides selon l'invention de formule (Ia) :

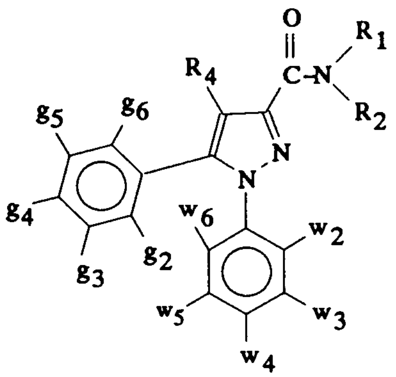

(Ia, avec X = liaison directe)

dans laquelle, $w_2$, $w_3$, $w_4$, $w_5$, $w_6$ et $g_2$, $g_3$, $g_4$, $g_5$, $g_6$, $R_1$, $R_2$ et $R_4$ sont tels que définis pour (I) sont préparés à partir d'un dérivé fonctionnel de l'acide (IIb), de préférence le chlorure, selon les méthodes habituelles afin de le substituer par une amine de formule $HNR_1R_2$, préparée selon les méthodes habituelles, pour obtenir les composés (Ia) selon l'invention.

Lorsque X est représenté par un groupe $-(CH_2)_x-N(R_3)-$ dans lequel x et $R_3$ sont tels que définis pour (I), les amides et les urées selon l'invention de formule (Ib) et (Ic) :

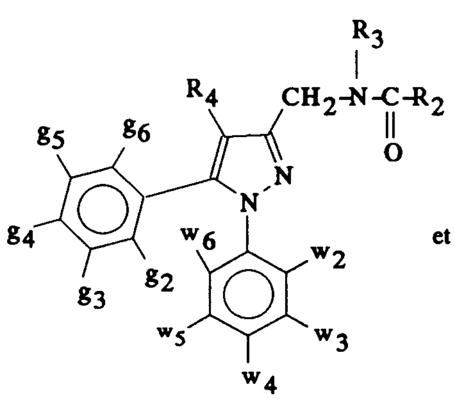

et

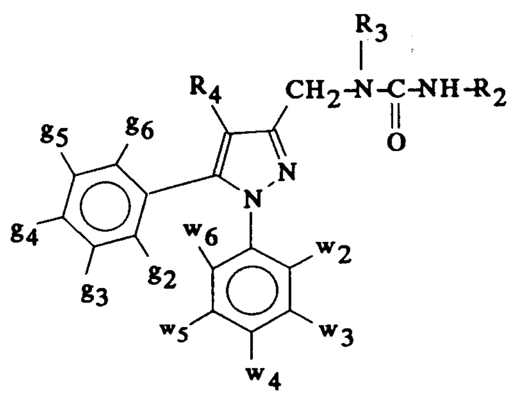

(Ib avec x = 1 et R = $R_2$) (Ic avec x = 1 et R = $NHR_2$)

dans lesquelles $w_2$, $w_3$, $w_4$, $w_5$, $w_6$ et $g_2$, $g_3$, $g_4$, $g_5$, $g_6$, $R_2$, $R_3$ et $R_4$ sont tels que définis pour (I) sont préparés à partir de l'ester (IIa) décrit précédemment selon le SCHEMA 2 suivant:

## SCHEMA 2

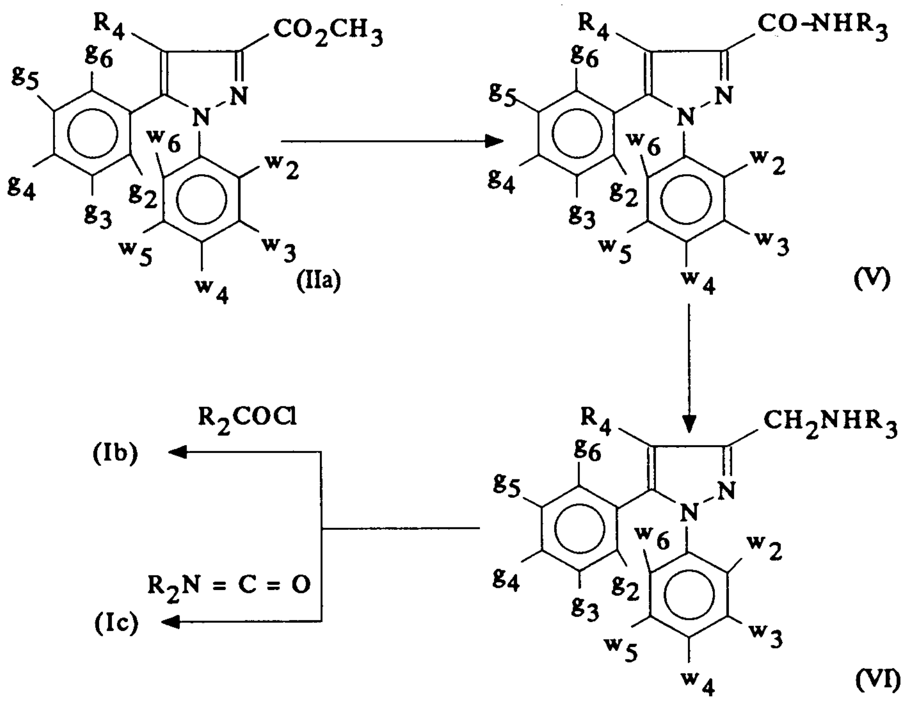


Le passage de l'ester (IIa) à l'amide intermédiaire (V) peut par exemple être effectué, via le chlorure d'acide correspondant, par réaction de celui-ci avec une amine $R_3NH_2$, dans un alcanol comme par exemple l'éthanol.

La réduction de l'amide (V) en amine (VI) est ensuite effectuée par un hydrure métallique, tel l'hydrure de lithium et d'aluminium ou préférentiellement par le complexe $BH_3$-THF en solution dans le THF chauffé au reflux. L'amine (VI) est alors transformée en amide (Ib) ou en urée (Ic) selon l'invention par les méthodes conventionnelles, par exemple respectivement par réaction avec un chlorure d'acide $R_2COCl$ ou avec un isocyanate $R_2$-N = C = O.

Les amides et les urées selon l'invention de formule (Id) et (Ie) :

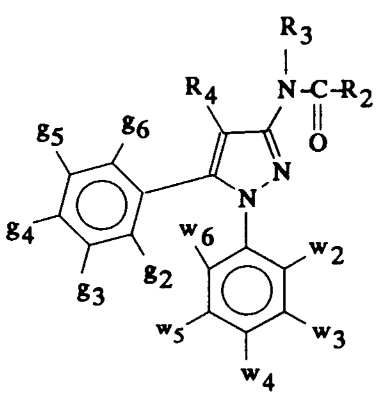

(Id, avec X = $-N(R_3)-$)

et

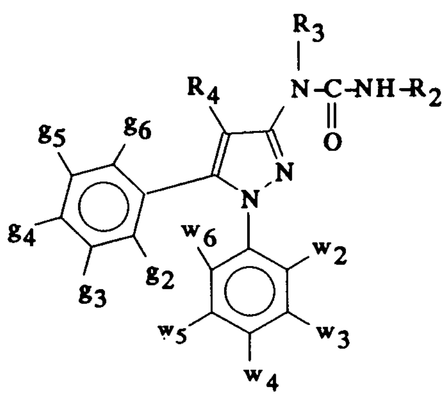

(Ie, avec X = $-N(R_3)-$ et $R_1$=H)

dans lesquelles $g_2$, $g_3$, $g_4$, $g_5$, $g_6$ et $w_2$, $w_3$, $w_4$, $w_5$, $w_6$, $R_2$, $R_3$ et $R_4$ sont tels que définis pour (I) sont préparés à partir des acides 3-pyrazolecarboxyliques obtenus précédemment selon le SCHEMA 3 suivant:

## SCHEMA 3

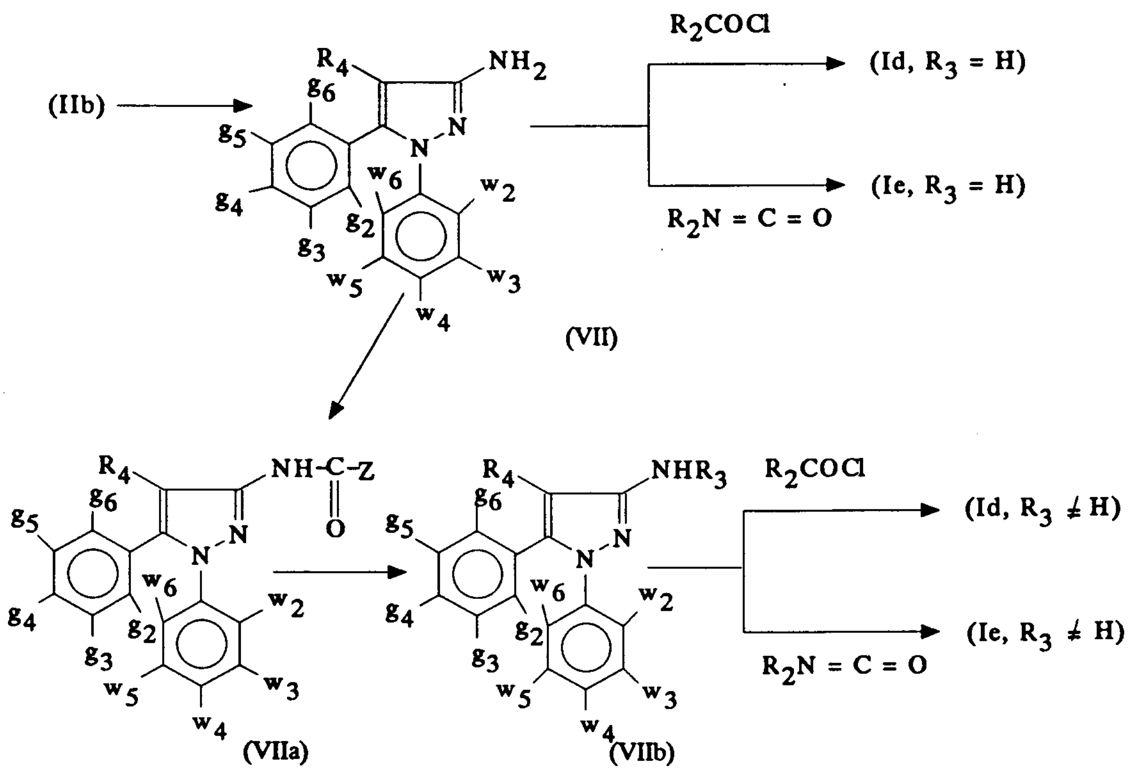


Les acides (IIb) sont transformés en amines correspondantes (VII) par réaction de Curtius, en utilisant par exemple l'azidure de diphénylphosphoryle en milieu basique suivi d'un traitement par un acide fort tel que l'acide chlorhydrique ou l'acide trifluoroacétique comme il est décrit dans Synthesis, 1990, 295. Les amines (VII) sont alors transformées en amides (Id) ou en urées (Ie) selon l'invention par les méthodes habituelles, telle que par exemple par réaction avec un chlorure d'acide $R_2COCl$ dans le cas de (Id) avec $R_3$ = H ou avec un isocyanate $R_2$-N = C = O dans le cas de (Ie) avec $R_3$ = H.

Alternativement les urées (Ie) avec $R_3$ = H peuvent être préparées par la réaction inverse : les acides (IIb) sont transformés en isocyanates correspondants (VIIc) comme décrit dans J. Org. Chem. 1961, *26,* 3511 selon le SCHEMA 4 ci-après.

La réaction des isocyanates (VIIc) avec une amine $R_2NH_2$ conduit alors directement aux urées (Ie).

## SCHEMA 4

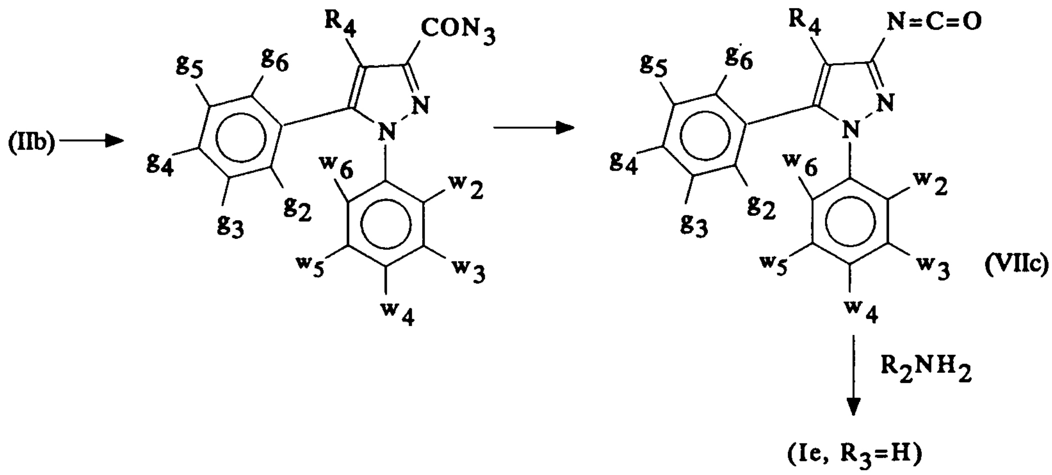


(IIb) (VIIc) (Ie, $R_3$=H)

Pour préparer les composés (Id) et (Ie) pour lesquels $R_3$ est différent de l'hydrogène, les amines primaires (VII) sont préalablement transformées en amines secondaires (VIIb) par une séquence réactionnelle telle que la réaction avec un chlorure d'acide $R'_3COCl$ (avec $R'_3$ = ($C_1$-$C_2$) alkyle), suivi d'une réduction de l'amide (VIIa) obtenu , par exemple par réaction avec $BH_3$ dans le THF. Dans le cas où $R_3$ représente un méthyle, on utilise préférentiellement la réaction des amines (VII) avec le dicarbonate de tertiobutyle, $(BOC)_2O$, ou avec un mélange d'acide formique et d'anhydride acétique qui conduisent respectivement au carbamate (VIIa, Z = O*t*Bu) ou au formamide (VIIa, Z = H), produits que l'on réduit ensuite, par exemple par $LiAlH_4$ pour obtenir les amines (VIIb, $R_3$ = $CH_3$).

Les amines secondaires (VIIb) sont ensuite transformées en amides (Id) ou en urées (Ie) selon l'invention comme décrit ci-dessus.

Les dérivés cétoniques selon l'invention de formule (If) :

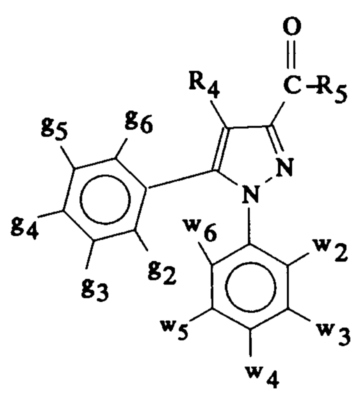

(If, X = liaison directe et R = $R_5$)

dans laquelle $g_2$, $g_3$, $g_4$, $g_5$, $g_6$ et $w_2$, $w_3$, $w_4$, $w_5$, $w_6$, $R_4$ et $R_5$ sont tels que définis pour (I) sont préparés préférentiellement à partir des acides 3-pyrazolecarboxyliques (IIb) décrits précédemment selon le SCHEMA 5 suivant:

## SCHEMA 5

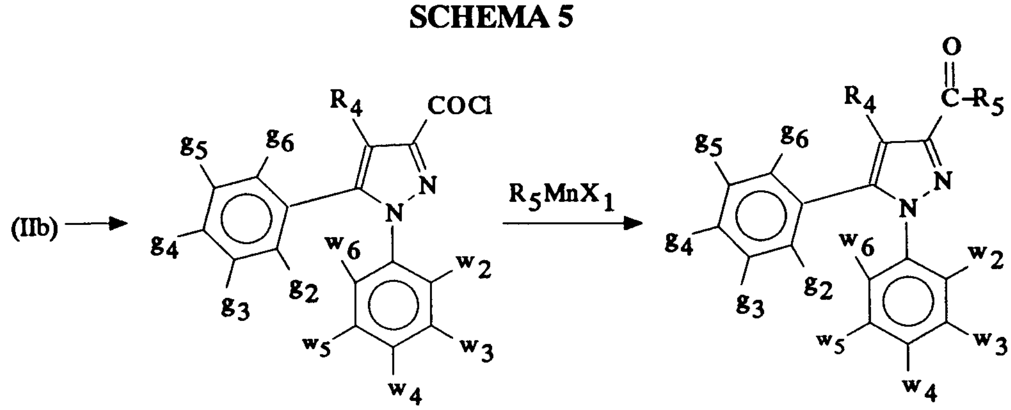


(If)

Les acides (IIb) sont transformés en chlorures d'acides selon les méthodes classiques; ces derniers sont ensuite transformés en dérivés cétoniques (If) selon l'invention par réaction avec un réactif organomanganeux approprié, $R_5MnX_1$ dans lequel $R_5$ est tel que défini pour (I) et $X_1$ représente un halogène, de préférence un atome de chlore, en utilisant par exemple la méthode décrite dans Tetrahedron Letters, 1989, *30,* 7369.

Alternativement, les dérivés cétoniques (If) peuvent être préparés à partir des acides (IIb) via les nitriles (IIc) selon le SCHEMA 6 suivant:

## SCHEMA 6

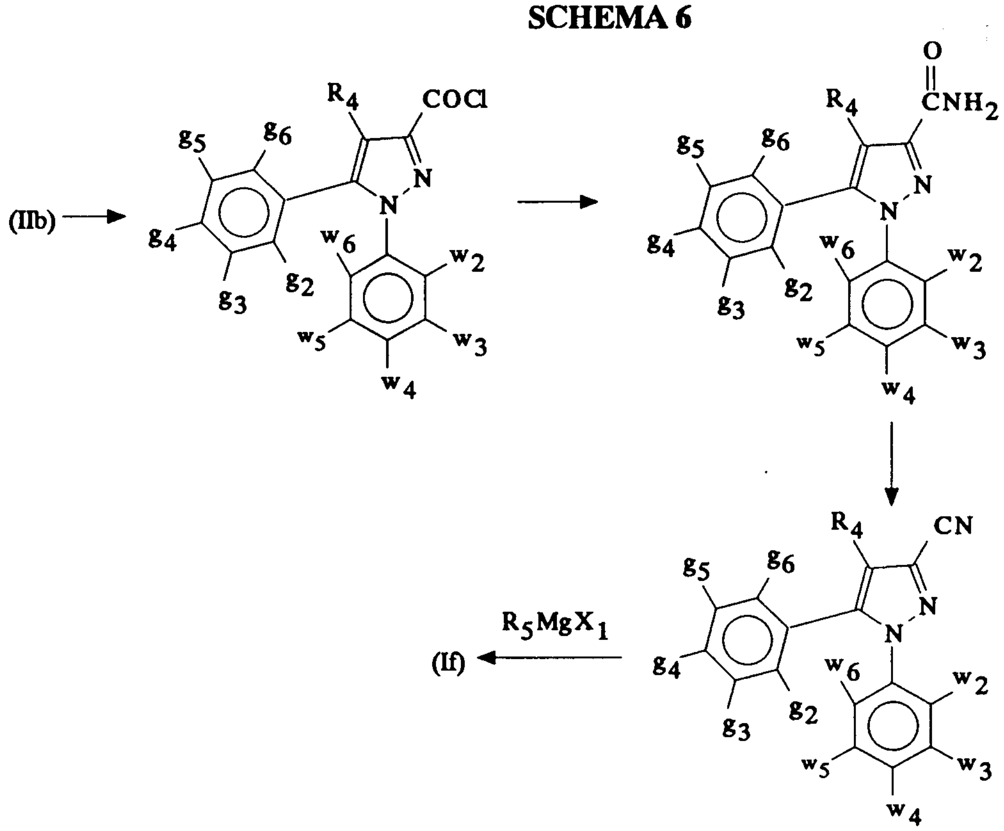


(IIc)

La transformation de (IIb) en (IIc) est effectuée par une méthode classique telle que, par exemple, la transformation en chlorure d'acide suivie d'amination ($NH_3$-THF-eau) et déshydratation de l'amide obtenu, par exemple, par traitement par $CH_3SO_2Cl$ dans la pyridine comme décrit dans J. Am. Chem. Soc., 1955, *77*, 1701.

Les nitriles (IIc) ainsi obtenus sont ensuite traités par des réactifs organométalliques préférentiellement organomagnésiens de formule $R_5MgX_1$ pour fournir, après traitement acide, les dérivés cétoniques (If).

Les composés de formule (I) ainsi obtenus sont isolés, sous forme de base libre ou éventuellement de sel, selon les techniques classiques.

Lorsque le composé de formule (I) est obtenu sous forme de base libre, la salification est effectuée par traitement avec l'acide choisi dans un solvant organique. Par traitement de la base libre, dissoute par exemple dans un alcool tel que l'isopropanol, avec une solution de l'acide choisi dans le même solvant, on obtient le sel correspondant qui est isolé selon les techniques classiques. Ainsi, on prépare par exemple le chlorhydrate, le bromhydrate, le sulfate, l'hydrogénosulfate, le dihydrogénophosphate, le méthanesulfonate, l'oxalate, le maléate, le fumarate, le 2-naphtalènesulfonate.

A la fin de la réaction, les composés de formule (I) peuvent éventuellement être isolés sous forme d'un de leurs sels, par exemple le chlorhydrate ou l'oxalate; dans ce cas, s'il est nécessaire, la base libre peut être préparée par neutralisation dudit sel avec une base minérale ou organique, telle que l'hydroxyde de sodium ou la triéthylamine ou avec un carbonate ou bicarbonate alcalin, tel que le carbonate ou bicarbonate de sodium ou de potassium.

Les amines de formule $HNR_1R_2$ sont soit disponibles commercialement, soit décrites dans la littérature ou soit préparées par des méthodes connues selon les PREPARATIONS décrites ci-après.

Parmi ces amines, les amines préférées sont par exemple celles citées ci-après:

(1) bicyclo[3.2.1]octan-2-ylamine préparée selon H. Maskill et al., J. Chem. Soc. Perkin II, 1984, 119 ;

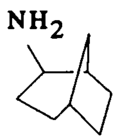

(2) bicyclo[2.2.2]octan-2-ylamine préparée selon R. Seka et al., Ber. 1942, 1379 ;

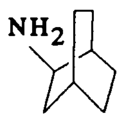

(3) endo et exo bicyclo[3.2.1.]octan-3-ylamine préparées selon H. Maskill et al., J. Chem. Soc. Perkin Trans II, 1984, 1369 ;

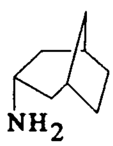

et

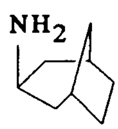

(4) endo et exo oxa-7-bicyclo[2.2.1]heptan-2-ylamine préparées selon W.L. Nelson et al., J. Heterocyclic Chem., 1972, *9*, 561 ;

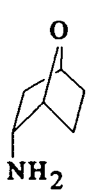

et

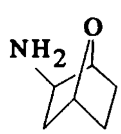

(5) endo-tricyclo[5.2.1.$0^{2,6}$]décan-8-amine préparée selon G. Buchbauer et al., Arch. Pharm., 1990, *323,* 367 ;

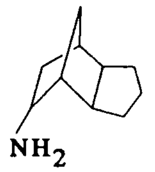

(6) endotriméthyl-1,3,3-bicyclo[2.2.1]heptan-2-ylamine préparée selon Ingersoll et al., J. Am. Chem. Soc., 1951, *73*, 3360 ;

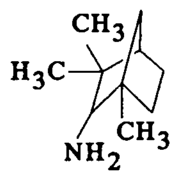

(7) 3-méthylcyclohexylamine préparée selon Smith et al., J. Org. Chem., 1952, *17,* 294 ;

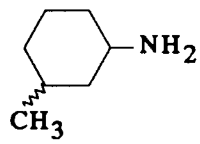

(8) 2,6-diméthylcyclohexylamine préparée selon Cornubert et al., Bull. Soc. Chim. Fr., 1945, *12,* 367 ;

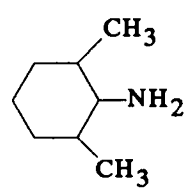

(9) 2-méthoxycyclohexylamine préparée selon Noyce et al., J. Am. Soc., 1954, *76*, 768 ;

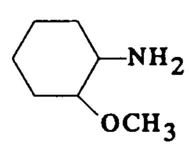

(10) 4-éthylcyclohexylamine préparée selon A. Shirahata et al., Biochem. Pharmacol., 1991, *41,* 205 ;

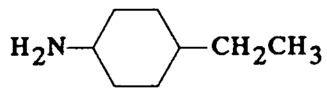

(11) bicyclo[2.2.2]oct-2-èn-5-amine préparée selon H.L. Goering et al., J. Am. Chem. Soc., 1961, *83,* 1391 ;

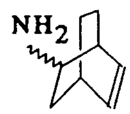

(12) N-éthyl-1-adamantylamine préparée selon V.L. Narayanan et al., J. Med. Chem., 1972, *15,* 443 ;

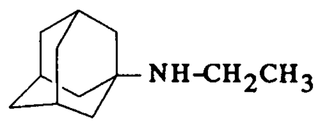

(13) tricyclo[2.2.1.0$^{2,6}$]heptan-3-ylamine préparée selon G. Muller et al., Chem. Ber., 1965, *98,* 1097;

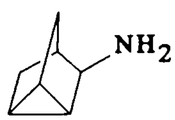

(14) N-méthyl-exo-bicyclo[2.2.1]heptan-2-ylamine préparée selon W.G. Kabalka et al., Synth. Commun., 1991, *20,* 231;

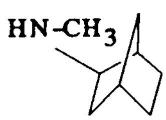

Les amines $R_3NH_2$ sont disponibles commercialement ou préparées selon des méthodes connues.

Les chlorures d'acides $R_2COCl$ sont disponibles commercialement ou préparés à partir des acides correspondants selon les méthodes connues.

Les isocyanates $R_2$-N=C=O sont également disponibles commercialement ou préparés à partir des amines correspondantes (réaction au phosgène) ou des acides correspondants (réarrangement de Curtius) selon les méthodes connues.

Les composés selon l'invention ont fait l'objet d'essais biochimiques.

Les composés (I) et leurs sels éventuels ont montré une bonne affinité *in vitro* pour les récepteurs aux cannabinoïdes dans des essais réalisés selon les conditions expérimentales décrites par Devane et al., Molecular Pharmacology,

1988, *34*, 605-613.

Les composés selon l'invention possèdent également une affinité pour les récepteurs aux cannabinoïdes présents sur des préparations d'organes isolés stimulés électriquement. Ces essais ont été réalisés sur l'iléon de cobaye et sur le vas deferens de souris selon Roselt et al., Acta Physiological, Scandinavia, 1975, *94*, 142-144 et selon Nicolau et al., Arch. Int. Pharmacodyn., 1978, *236,* 131-136.

Les composés selon l'invention sont généralement administrés en unités de dosage.

Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I) ou un de ses sels pharmaceutiquement acceptables.

Les composés de formule (I) ci-dessus et leurs sels pharmaceutiquement acceptables peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,5 à 4000 mg par jour, plus particulièrement de 2,5 à 1000 mg selon l'âge du sujet à traiter ou le type de traitement : prophylactique ou curatif.

Dans les compositions pharmaceutiques de la présente invention pour l'administration orale, sublinguale, sous-cutanée, intramusculaire, intraveineuse, transdermique, locale ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale

Dans les compositions pharmaceutiques de la présente invention, le principe actif est généralement formulé en unités de dosage contenant de 0,5 à 1000 mg, de préférence de 1 à 200 mg dudit principe actif par unité de dosage.

Lorsque l'on prépare une composition solide sous forme de comprimés, on mélange le principe actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant et en versant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Pour une administration par inhalation on utilise un aérosol contenant par exemple du trioléate de sorbitane ou de l'acide oléique ainsi que du trichlorofluorométhane, du dichlorotétrafluoroéthane ou tout autre gaz propulseur biologiquement compatible.

Le principe actif peut être formulé également sous forme de microcapsules, éventuellement avec un ou plusieurs supports ou additifs.

Le principe actif peut être également présenté sous forme de complexe avec une cyclodextrine, par exemple $\alpha$, $\beta$ ou $\gamma$ cyclodextrine, 2-hydroxypropyl-$\beta$-cyclodextrine ou méthyl-$\beta$-cyclodextrine.

Les composés de formule (I) ainsi formulés peuvent être utilisés pour le traitement de l'immunomodulation, de la migraine, de l'asthme, de l'épilepsie, du glaucome, de la maladie de Parkinson, des dyskinésies, des neuropathies, des troubles mnésiques et thymiques, des vomissements, de l'ischémie, de l'angor, de l'hypotension orthostatique ou de l'insuffisance cardiaque.

Les exemples suivants illustrent l'invention sans toutefois la limiter.

Les points de fusion ou de décomposition des produits, F, ont été mesurés en tube capillaire avec un appareil de Tottoli.

L'excès énantiomérique des amines optiquement actives, e.e., a été déterminé par RMN du $^{19}F$ après réaction avec le chlorure d'acide de Mosher S (+) selon J. Org. Chem., 1969, *34,* 2543.

Les pouvoirs rotatoires, $[\alpha]_D^{20}$ , ont été mesurés à c = 1 dans l'éthanol.

**PREPARATIONS**

A. AMINES $NHR_1R_2$

1. (1*R*,2*S*-endo) (+) bicyclo[2.2.1]heptan-2-ylamine.

L'acide (1*R*,2*S*-endo) (-) bicyclo[2.2.1]heptan-2-carboxylique est préparé selon Tetrahedron Letters, 1985, *26,* 3095.

Par une réaction de Curtius effectuée selon J. Org. Chem., 1961, *26,* 3511, il est ensuite transformé en amine correspondante (1*R*,2*S*-endo) (+).

$[\alpha]_D^{20}$ = +13,4° (c = 1, EtOH).

e.e. > 95 % δ ($CF_3$) = 6,67 ppm par rapport à $CF_3CO_2H$.

2. (1*R*,2*R*-exo) (-) bicyclo[2.2.1]heptan-2-ylamine.

L'acide (1*R*,2*S*-endo) (-) bicyclo[2.2.1]heptan-2-carboxylique préparé dans l'exemple précédent est transformé en son isomère (1*R*,2*R*-exo) (-) selon J. Am. Chem. Soc., 1983, *105,* 950 puis transformé comme décrit dans l'exemple précédent en amine correspondante (1*R*,2*R*-exo) (-).

$[\alpha]_D^{20}$ = -17,7° (c = 1,EtOH).

e.e. > 94 % (déterminé comme ci-dessus, δ ($CF_3$) = 6,81 ppm).

3. (1*S*,2*R*-endo) (-) bicyclo[2.2.1]heptan-2-ylamine.

L'acide (1*S*,2*R*-endo) (+) bicyclo[2.2.1]heptan-2-carboxylique est préparé selon Tetrahedron Letters, 1989, *30,* 5595 puis transformé comme décrit précédemment en amine correspondante (1*S*,2*R*-endo) (-).

e.e. > 95 % (déterminé comme ci-dessus, δ ($CF_3$) = 6,62 ppm).

4. (1*S*,2*S*-exo) (+) bicyclo[2.2.1]heptan-2-ylamine.

L'acide (1*S*,2*R*-endo) (+) préparé dans l'exemple précédent est transformé en son isomère (1*S*,2*S*-exo) (+) selon J. Am. Chem. Soc., 1983, *105,* 950 puis celui-ci est converti en l'amine correspondante (1*S*,2*S*-exo) (+) comme décrit précédemment.

e.e. > 94 % (déterminé comme ci-dessus, δ ($CF_3$) = 6,91 ppm).

5. exo-3-chlorobicyclo[3.2.1]oct-3-ènyl-2-amine.

Une solution de 6,1 g d'exo-3-chloro-2-azidobicyclo[3.2.1]oct-3-ène obtenu selon J. Chem. Soc. Perkin Trans II, 1984, 119 dans 600 ml d'éthanol et 60 ml de $CHCl_3$ est additionnée de 0,4 g de $PtO_2$ et hydrogénée dans un appareil de Parr à 4 bars et température ambiante jusqu'à disparition de la fonction azide. Après filtration sur célite, le mélange réactionnel est évaporé et le résidu est cristallisé dans un mélange éthanol/$CHCl_3$. On obtient 0,49 g du chlorhydrate de l'amine attendue, F > 240°C.

6. N-éthyl-exo-bicyclo[2.2.1]heptan-2-ylamine.

6.1. N-acétyl-exo-bicyclo[2.2.1]heptan-2-ylamine.

Une solution de 3,50 ml de chlorure d'acétyle dans 10 ml de $CH_2Cl_2$ est ajoutée goutte à goutte à une solution de 5,00 g d'exo-bicyclo[2.2.1]heptan-2-ylamine et 6,90 ml de triéthylamine dans 50 ml de $CH_2Cl_2$ refroidie à 0°C. Après 16 heures d'agitation à température ambiante, le mélange est versé dans 100 ml d'eau glacée, et la phase organique est séparée et lavée par une solution d'HCl à 5 % puis à l'eau puis par une solution saturée de NaCl. Après séchage sur $MgSO_4$ et évaporation des solvants on obtient 5,80 g de l'acétamide attendu, F = 128°C.

6.2. N-éthyl-exo-bicyclo[2.2.1]heptan-2-ylamine.

Une solution de 5,10 g du dérivé précédent dans 30 ml de THF est ajoutée goutte à goutte sur une suspension de 2,18 g de $LiAlH_4$ dans 30 ml de THF refroidie à 0°C puis le mélange est chauffé à reflux pendant 8 heures. Le mélange est hydrolysé à 0°C par 2,2 ml d'eau puis 2,2 ml de solution de NaOH à 15 % puis 7,5 ml d'eau. Après 15 minutes d'agitation le précipité est filtré et lavé au THF, le filtrat est évaporé puis repris dans 50 ml d'éther éthylique. Cette solution éthérée est extraite par HCl 5 % ; la phase aqueuse obtenue est neutralisée par NaOH 30 % puis extraite à l'éther éthylique. Après lavage par une solution saturée de NaCl, séchage sur $MgSO_4$ et évaporation on obtient 3,82 g de liquide jaune pâle. Par dissolution dans l'éther éthylique et traitement par une solution d'HCl gaz dans l'éther éthylique anhydre on obtient un précipité blanc que l'on filtre, lave à l'éther éthylique et sèche sous vide. On obtient ainsi 4,16 g du chlorhydrate de l'amine attendue, F = 145°C (décomposition).

7. N-(n-propyl)-exo-bicyclo[2.2.1]heptan-2-ylamine.

7.1. N-propionyl-exo-bicyclo[2.2.1]heptan-2-ylamine.

Cet amide est obtenu de la même façon que l'analogue N-acétyle décrit dans l'exemple 6 précédent, en utilisant le chlorure de propionyle au lieu du chlorure d'acétyle.

7.2. N-(n-propyl)-exo-bicyclo[2.2.1]heptan-2-ylamine.

Cette amine est obtenue au départ de l'amide précédent, de la même façon que l'analogue N-éthyl décrit dans l'exemple précédent. Par salification par $HCl/Et_2O$ dans un mélange $Et_2O/iPr_2O$ on obtient le chlorhydrate de l'amine attendue, F = 230°C (décomposition).

8. Bicyclo[3.3.1]nonan-9-ylamine.

8.1. Bicyclo[3.3.1]nonan-9-onoxime.

Une solution de 1,83 g de chlorhydrate d'hydroxylamine et de 2,95 g d'acétate de sodium dans 22 ml d'eau est ajoutée à une solution de 2,43 g de bicyclo[3.3.1]nonan-9-one dans 9 ml de méthanol, et le mélange est chauffé à reflux pendant 24 heures. Après refroidissement, le mélange est extrait à l'éther éthylique, les phases organiques sont lavées par une solution de NaCl saturée puis par une solution de $Na_2CO_3$ à 5 %, puis à l'eau, séchées sur $MgSO_4$ et évaporées. On obtient 3,00 g d'oxime, F = 151°C

8.2. Bicyclo[3.3.1]nonan-9-ylamine.

Une solution de 1,00 g d'oxime dans 250 ml d'éthanol et 4 ml de $CHCl_3$ est additionnée de 0,20 g de $PtO_2$ et hydrogénée dans un appareil de Parr à 6 bars et température ambiante pendant 18 heures. Après filtration sur célite, les solvants sont évaporés et le résidu est cristallisé dans un mélange éthanol/heptane. On obtient 0,55 g de chlorhydrate de l'amine attendue. F > 240°C.

**EXEMPLE 1**

N-(2-adamantyl)-1-(2,4-dichlorophényl)-5-(4-chlorophényl)-1*H*-pyrazole-3-carboxamide

(I):$w_2$, $w_4$ = Cl ; $g_4$ = Cl; $R_4$ = H;

-X-CO-R = -CO-NH 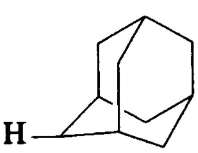

A) Sel de sodium du 4-chlorobenzoylpyruvate de méthyle.

12 g de sodium sont dissous dans 250 ml de méthanol anhydre. On ajoute ensuite un mélange de 64,6 ml de 4-chloroacétophénone et 67,1 ml d'oxalate de diéthyle dans 600 ml de méthanol en maintenant la température inférieure à 10°C. Le mélange réactionnel est ensuite agité à température ambiante pendant 3 heures puis on ajoute 1 l d'éther sec. On agite encore pendant 20 minutes, filtre, lave le précipité à l'éther et le sèche sous vide pour obtenir 74,6 g du sel de sodium attendu.

B) Ester méthylique de l'acide 1-(2,4-dichlorophényl)-5-(4-chlorophényl)-1*H*-pyrazole-3-carboxylique.

Une suspension de 26,3 g du sel de sodium obtenu précédemment et 23,5 g de chlorhydrate de 2,4-dichlorophénylhydrazine dans 250 ml d'acide acétique est chauffée à reflux pendant 4 heures. Après refroidissement, le

mélange est versé sur 250 g de glace, les cristaux obtenus sont filtrés, lavés à l'eau et séchés sous vide pour fournir 26,3 g d'ester, F=167°C.

C) Acide 1-(2,4-dichlorophényl)-5-(4-chlorophényl)-1*H*-pyrazole-3-carboxylique.

Une solution de 3,70 g de KOH dans 35 ml d'eau est ajoutée à une solution de 10,0 g d'ester obtenu précédemment dans 35 ml de méthanol. Le mélange est chauffé à reflux pendant 4 heures, refroidi à température ambiante et versé dans 100 ml d'eau puis neutralisé par une solution d'HCl à 5 %. Les cristaux obtenus sont filtrés, lavés à l'eau puis au pentane et séchés sous vide. On obtient 9,50 g d'acide, F = 185°C.

D) Chlorure de l'acide 1-(2,4-dichlorophényl)-5-(4-chlorophényl)-1*H*-pyrazole-3-carboxylique.

5,8 ml de chlorure de thionyle sont ajoutés à une suspension de 9,50 g de l'acide obtenu précédemment dans 100 ml de toluène et le mélange est chauffé à reflux pendant 3 heures. Le solvant est ensuite évaporé puis le résidu est repris dans 50 ml de toluène et le solvant est reévaporé (processus répété deux fois). On obtient 8,28 g de chlorure d'acide.

E) N-(2-adamantyl)-1-(2,4-dichlorophényl)-5-(4-chlorophényl)-1*H*-pyrazole-3-carbo-xamide

Une solution de 0,50 g du chlorure d'acide obtenu précédemment dans 10 ml de $CH_2Cl_2$ est ajoutée goutte à goutte à une solution de 0,30 g de chlorhydrate de 2-adamantaneamine et de 0,41 ml de triéthylamine dans 10 ml de $CH_2Cl_2$ refroidie à 0°C. Le mélange est ensuite agité à température ambiante pendant 16 heures puis versé dans 30 ml d'eau glacée. Le mélange est extrait au $CH_2Cl_2$ et la phase organique est lavée successivement avec une solution d'HCl à 5 %, à l'eau, par une solution de $Na_2CO_3$ à 5 % puis par une solution de NaCl saturée. Après séchage sur $MgSO_4$ et évaporation du solvant, le produit brut est cristallisé dans le benzène à chaud pour donner 0,32 g de cristaux blancs, F = 203°C.

**EXEMPLE 2**

N-(trans-4-hydroxycyclohexyl)-1-(2,4-dichlorophényl)-5-(4-chlorophényl)-1*H*-pyrazole-3-carboxamide

(I):$w_2$,$w_4$ = Cl; $g_4$ = Cl; $R_4$ = H;

$-X-CO-R = -CO-NH$ 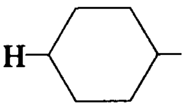 $OH$ trans.

A) trans-4-triméthylsilyloxycyclohexylamine.

Une solution de 1,85 ml de chlorotriméthylsilane dans 10 ml de $CH_2Cl_2$ est ajoutée goutte à goutte à une solution de 2,0 g de chlorhydrate de trans-4-hydroxycyclohexylamine et de 4,05 ml de triéthylamine dans 20 ml de $CH_2Cl_2$ refroidie à 0°C. Après 16 heures d'agitation à température ambiante, le mélange est hydrolysé à l'eau et extrait. La phase organique est lavée successivement à l'eau, par une solution de $Na_2CO_3$ à 5 % et de NaCl saturée. Après séchage sur $MgSO_4$ et évaporation des solvants, on obtient 1,43 g d'amine (liquide incolore).

B) N-(trans-4-hydroxycyclohexyl)-1-(2,4-dichlorophényl)-5-(4-chlorophényl)-1*H*-pyrazole-3-carboxamide

Une solution de 0,60 g de chlorure d'acide préparé précédemment selon l'exemple 1D) dans 10 ml de $CH_2Cl_2$ est ajoutée goutte à goutte à une solution de 0,35 g de trans-4-triméthylsilyloxycyclohexylamine et 0,32 ml de triéthylamine dans 10 ml de $CH_2Cl_2$ refroidie à 0°C. Après 16 heures d'agitation à température ambiante le mélange est versé dans 30 ml d'eau glacée, et extrait au $CH_2Cl_2$. La phase organique est lavée successivement par HCl à 5 % et par une solution de NaCl saturée puis séchée sur $MgSO_4$ et évaporée. Le produit brut est dissous dans 15 ml de THF ; on ajoute à la solution 15 ml d'HCl à 5 % et agite pendant 1 heure. Le mélange est alors extrait à l'éther et lavé à l'eau puis séché sur $MgSO_4$ et évaporé pour donner après cristallisation dans $CH_3OH$, 0,20 g du pyrazole attendu, F = 209°C.

En procédant selon l'EXEMPLE 1 ci-dessus, à partir, par exemple, des dérivés acides ou esters ci-dessous décrits dans le TABLEAU A, on prépare les composés décrits dans les TABLEAUX I à XII ci-après.

## TABLEAU A

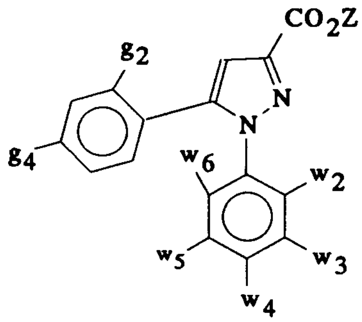

| $w_2$ | $w_3$ | $w_4$ | $w_5$ | $w_6$ | $g_2$ | $g_4$ | F ; °C Z = H | F ; °C Z = $CH_3$ |
|---|---|---|---|---|---|---|---|---|
| H | H | $CH_3$ | H | H | H | Cl | 185 | 98 |
| H | Cl | Cl | H | H | H | Cl | 162 | 147 |
| H | Cl | Cl | H | H | H | $CH_3$ | 188 | 145 |
| Cl | H | H | Cl | H | H | $CH_3$ | 232 | 132 |
| Cl | H | H | Cl | H | H | $CF_3$ | 214 | 179 |
| Cl | Cl | H | H | H | H | $CH_3$ | 214 | 101 |
| H | H | $CH_3$ | H | H | Cl | Cl | 188 | 102 |
| H | H | Cl | H | H | Cl | Cl | 224 | 118 |
| H | H | $OCH_3$ | H | H | H | Cl | 168 | - |
| Cl | H | Cl | H | Cl | H | Cl | 255 | 214 |
| Cl | H | Cl | H | H | H | 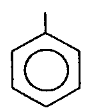 | 115 | 138 |
| Cl | H | Cl | H | H | H | Br | 188 | 177 |
| H | H | $NO_2$ | H | H | H | $CH_3$ | 106 | 166 |

# TABLEAU I

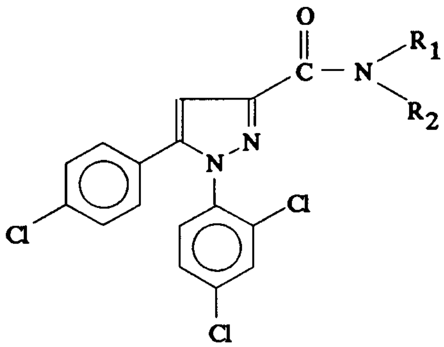

(Ia)

| Exemple n° | 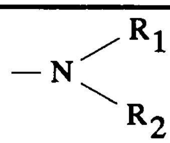 | F ; °C ou $[\alpha]^{20}_{D}$ | Sel |
|---|---|---|---|
| 3 | $-NH-(CH_2)_2-CH_3$ | 100 | |
| 4 | 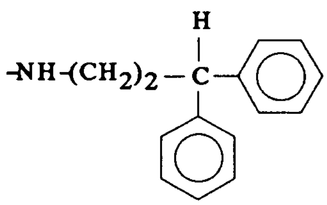 | 102 | |
| 5 | 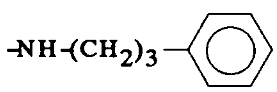 | 60 | |
| 6 | $-NH-(CH_2)_4-CH_3$ | 97 | |
| 7 | 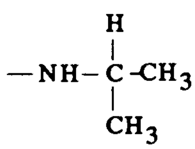 | 152 | |
| 8 | 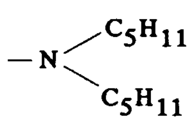 | (1) | |
| 9 | 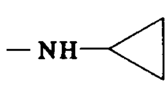 | 152 | |

TABLEAU I (suite 1)

| Exemple n° | 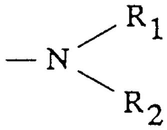 | F ; °C ou $[\alpha]_D^{20}$ | Sel |
|---|---|---|---|
| 10 | 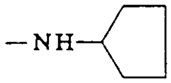 | 148 | |
| 11 | 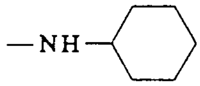 | 162 | |
| 12 | 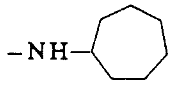 | 83 | |
| 13 | 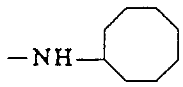 | 132 | |
| 14 | 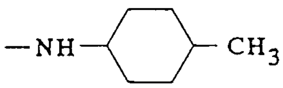 trans | 186 | |
| 15 | 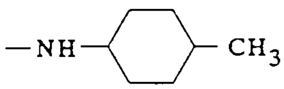 cis | 165 | |
| 16 | 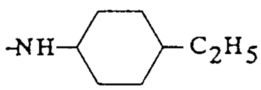 | 134 | |
| 17 | 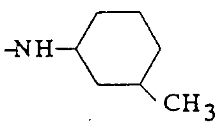 | 144 | |
| 18 | 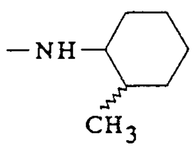 | 174 | |
| 19 | 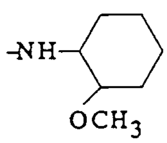 | 188 | |
| 20 | 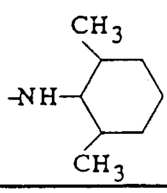 | 120 | |

## TABLEAU I (suite 2)

| Exemple n° | 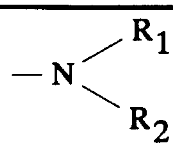 | F ; °C ou $[\alpha]_D^{20}$ | Sel |
|---|---|---|---|
| 21 | 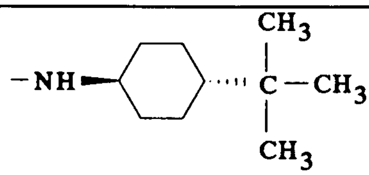 trans | 208 | |
| 22 | 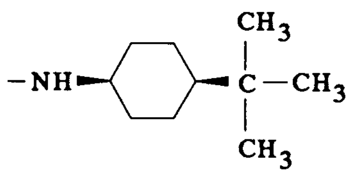 cis | 81 | |
| 23 | 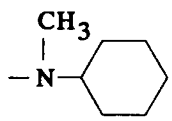 | 122 | |
| 24 | 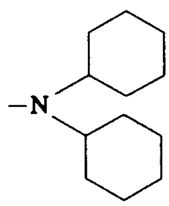 | 188 | |
| 25 | 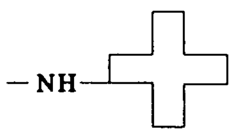 | 194 | |
| 26 | 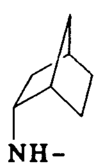 endo (+,-) | 190 | |
| 27 | 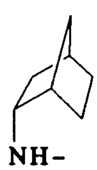 endo (+) | 183<br>+ 14,1° | |

## TABLEAU I (suite 3)

| Exemple n° | 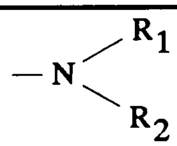 | F ; °C ou $[\alpha]_D^{20}$ | Sel |
|---|---|---|---|
| 28 | 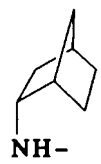 endo (–) | 182<br>– 14,1° | |
| 29 | 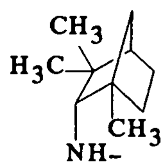 endo | 178 | |
| 30 | 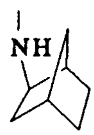 exo (+,–) | 191 | |
| 31 | 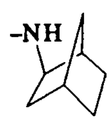 exo (+) | 185<br>+ 10,2° | |
| 32 | 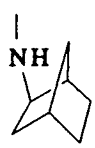 exo (–) | 184<br>– 10,6° | |
| 33 | 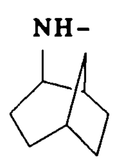 exo | 170 | |
| 34 | 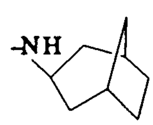 exo | 198 | |

**TABLEAU I (suite 4)**

| Exemple n° | 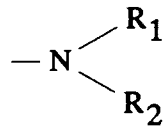 | F ; °C ou $[\alpha]^{20}_{D}$ | Sel |
|---|---|---|---|
| 35 | 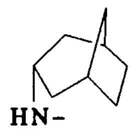 endo | 182 | |
| 36 | 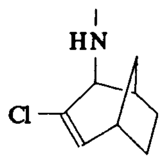 exo | 188 | |
| 37 | 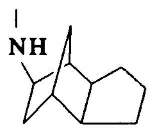 | 141 | |
| 38 | 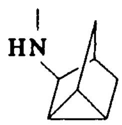 | 197 | |
| 39 | 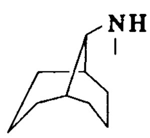 | 209 | |
| 40 | 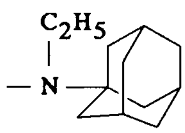 | 164 | |
| 41 | 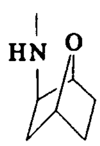 endo | 184 | |
| 42 | 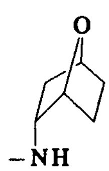 exo | 180 | |

**TABLEAU I (suite 5)**

| Exemple n° | 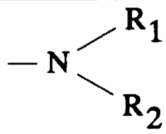 | F ; °C ou $[\alpha]_D^{20}$ | Sel |
|---|---|---|---|
| 43 | 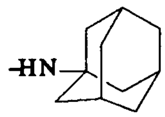 | 233 | |
| 44 | 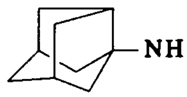 | 220 | |
| 45 | 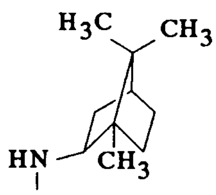 (+) | 156<br>+11,7° | |
| 46 | 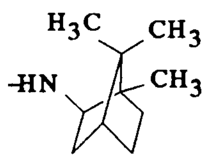 (-) | 151<br>-61,6° | |
| 47 | 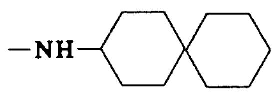 | 168 | |
| 48 | 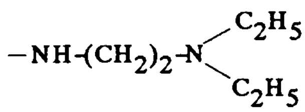 | 108 | |
| 49 | 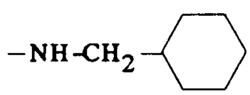 | 161 | |
| 50 | 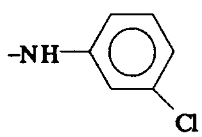 | 154 | |
| 51 | 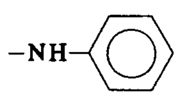 | 112 | |

TABLEAU I (suite 6)

| Exemple n° | 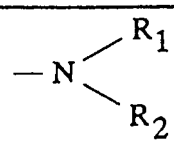 | F ; °C ou $[\alpha]^{20}_{D}$ | Sel |
|---|---|---|---|
| 52 | 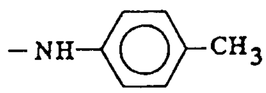 | 159 | |
| 53 | 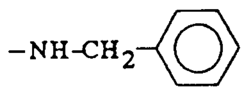 | 149 | |
| 54 | 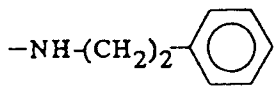 | 125 | |
| 55 | 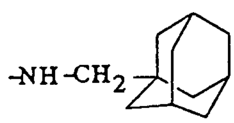 | 220 | |
| 57 | 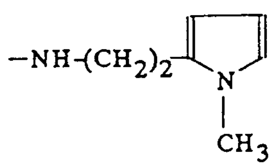 | 158 | |
| 58 | 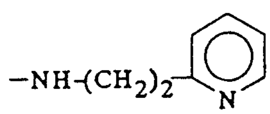 | 234 | HCl |
| 59 | 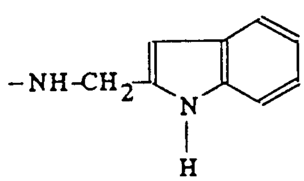 | 96 | |
| 60 | 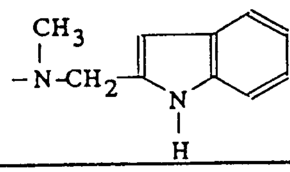 | 95 | |

## TABLEAU I (suite 7)

| Exemple n° | 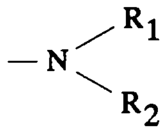 | F ; °C ou $[\alpha]_D^{20}$ | Sel |
|---|---|---|---|
| 61 | 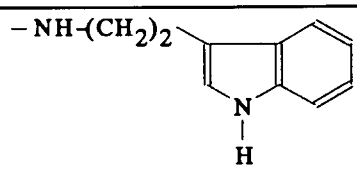 | 179 | |
| 62 | 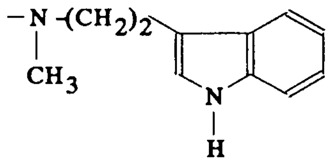 | 172 | |
| 63 | 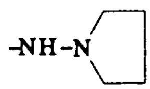 | 215 (déc) | HCl |
| 64 | 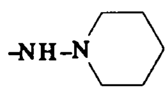 | 184 | |
| 65 | 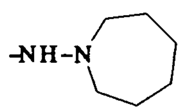 | 195(déc) | HCl |
| 66 | 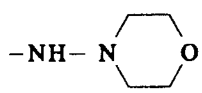 | 158 | |
| 67 | 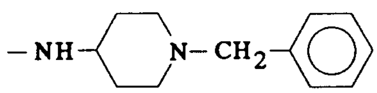 | 147 | |
| 68 | 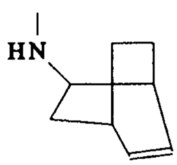 | 186 | |
| 69 | 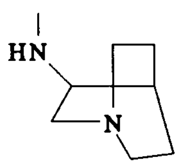 | 205 | HCl |

## TABLEAU I (suite 8)

| Exemple n° | 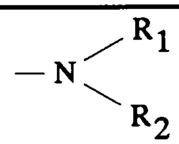 | F ; °C ou $[\alpha]_D^{20}$ | Sel |
|---|---|---|---|
| 70 | 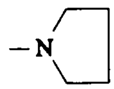 | 136 | |
| 71 | 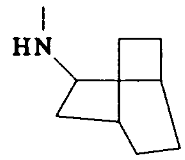 | 208 | |
| 72 | 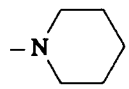 | 162 | |
| 73 | 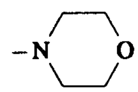 | 139 | |

(1) Spectre de RMN du composé de l'exemple 8 ; (200MHz, DMSO $d^6$) : 0,74 (3H, t J = 5Hz, $CH_3$) ; 0,91 (3H, t J = 5Hz, $CH_3$) ; 1,41–1,69 (12H, m, $6CH_2$) ; 3,43 (2H, t, $NCH_2$) ; 3,66 (2H, s, $NCH_2$) ; 7,06 (1H, s, *H* pyrazole) ; 7,29 (2H, d J = 8Hz, *H*ar) ; 7,49 (2H, d J = 8Hz, *H*ar) ; 7,62–7,77 (2H, m, *H*ar) ; 7,92 (1H, d J = 2Hz, *H*ar).

## TABLEAU II

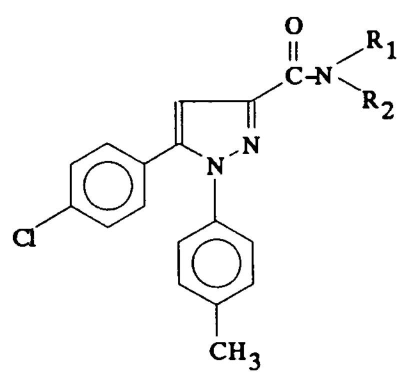

(Ia)

| Exemple n° | 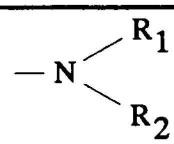 | F ; °C |
|---|---|---|
| 74 | $-NH-(CH_2)_2-CH_3$ | 95 |

## TABLEAU II (suite)

| Exemple n° | 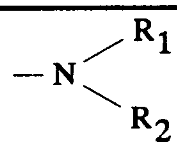 | F ; °C |
|---|---|---|
| 75 | 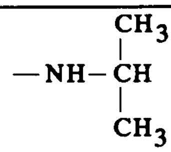 | 114 |
| 76 | 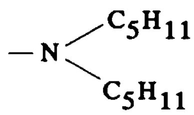 | 59 |
| 77 | 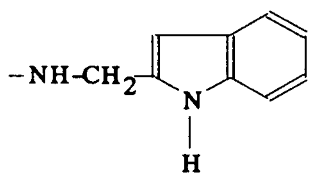 | 175 |
| 78 | 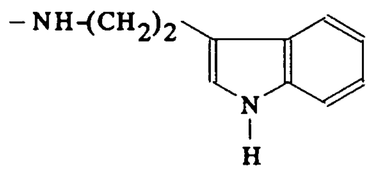 | 178 |
| 79 | 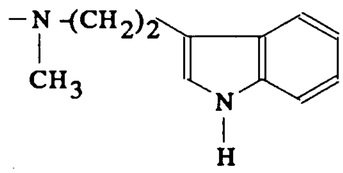 | 175 |
| 80 | 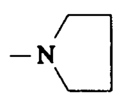 | 147 |

## TABLEAU III

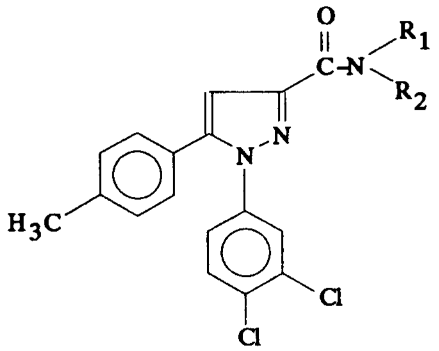

(Ia)

| Exemple n° | 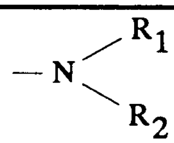 | F ; °C |
|---|---|---|
| 81 | 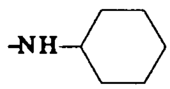 | 144 |
| 82 | 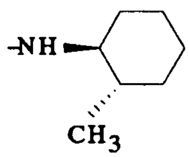 trans | 165 |
| 83 | 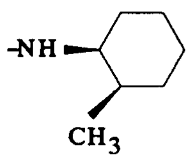 cis | 143 |
| 84 | 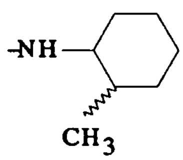 | 155 |
| 85 | 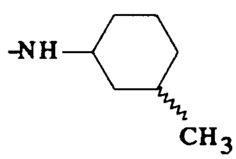 | 153 |
| 86 | 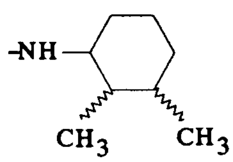 | 129 |

**TABLEAU III (suite 1)**

| Exemple n° | 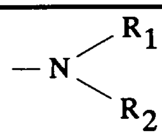 | F ; °C |
|---|---|---|
| 87 | 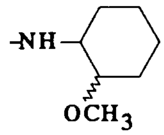 | 140 |
| 88 | 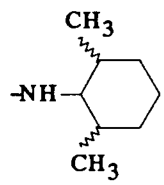 | 148 |
| 89 | 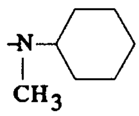 | 137 |
| 90 | 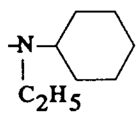 cis + trans | 63 |
| 91 | 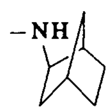 exo (+,-) | 156 |
| 92 | 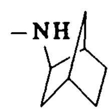 exo (-) | 149<br>- 15,1° |
| 93 | 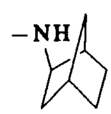 exo (+) | 149<br>+ 15,1° |
| 94 | 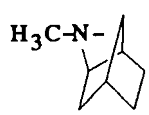 exo | (2) |
| 95 | 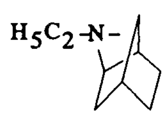 exo | 48 |

## TABLEAU III (suite 2)

| Exemple n° | 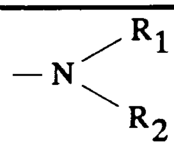 | F ; °C |
|---|---|---|
| 96 | 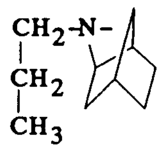 exo | 57 |
| 97 | 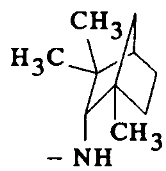 endo | 157 |
| 98 | 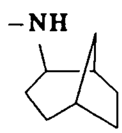 exo | 168 |
| 99 | 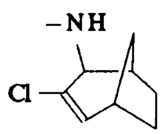 exo | 156 |
| 100 | 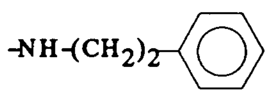 | 112 |

(2) Spectre de RMN du composé de l'exemple 94 (200MHz, DMSO $d^6$) : 1,14–1,80 (10H, m, norbornyle) 2,34 (3H, s, $CH_3$ tolyle) ; 3,12 (3H, se, $NCH_3$) ; 4,40 (1H, t, N-*CH* norbornyle) ; 6,90 (1H, s, *H* pyrazole) ; 7,23–7,31 (2H, m, *H*ar) ; 7,71–7,77 (5H, m, *H*ar).

# TABLEAU IV

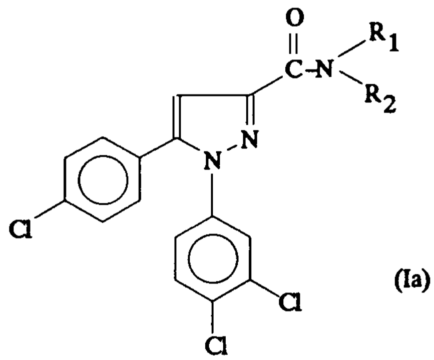

(Ia)

| Exemple n° | 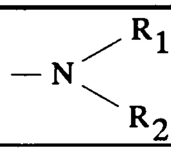 | F ; °C |
|---|---|---|
| 101 | 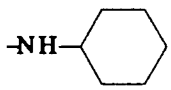 | 154 |
| 102 | 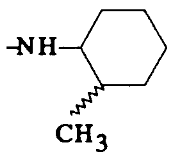 | 149 |
| 103 | 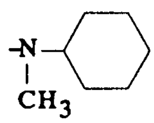 | 136 |
| 104 | 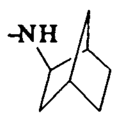 exo | 165 |
| 105 | 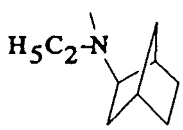 exo | 134 |

## TABLEAU V

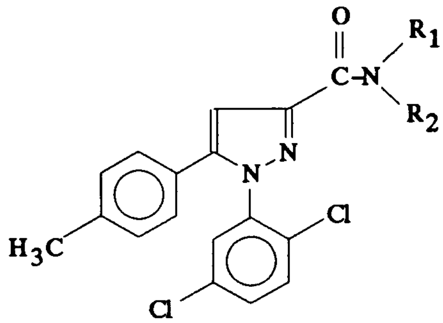

(Ia)

| Exemple n° | 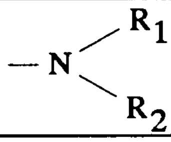 | F ; °C |
|---|---|---|
| 106 | 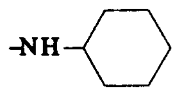 | 205 |
| 107 | 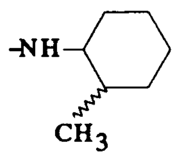 | 175 |
| 108 | 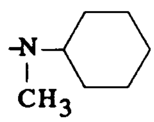 | 214 |
| 109 | 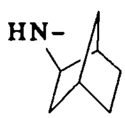 exo | 240 |
| 110 | 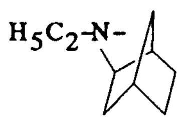 exo | 124 |
| 111 | 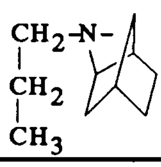 exo | 124 |

# TABLEAU VI

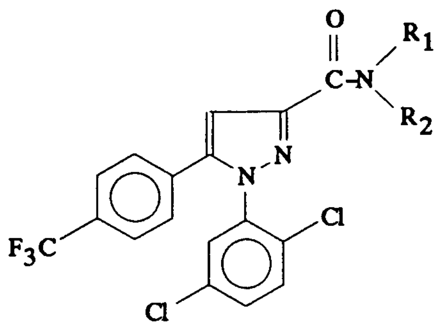

(Ia)

| Exemple n° | 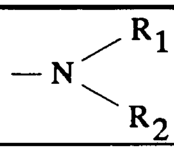 | F ; °C |
|---|---|---|
| 112 | 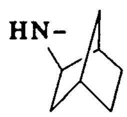 exo | 215 |
| 113 | 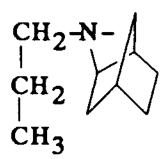 exo | 55 |
| 114 | 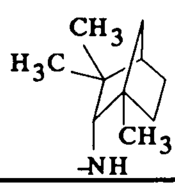 | 168 |

## TABLEAU VII

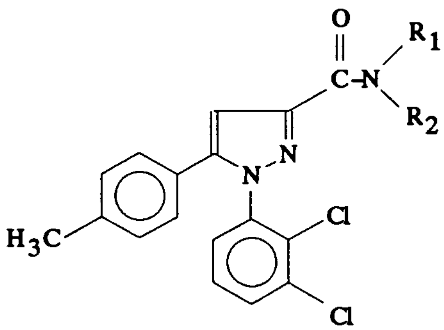

(Ia)

| Exemple n° | 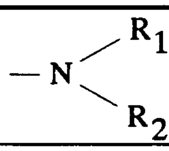 | F ; °C |
|---|---|---|
| 115 | 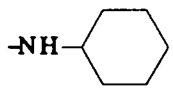 | 193 |
| 116 | 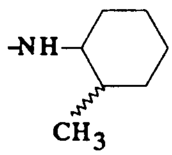 | 168 |
| 117 | 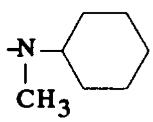 | 152 |
| 118 | 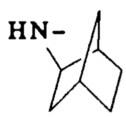 exo | 216 |
| 119 | 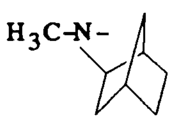 exo | 154 |
| 120 | 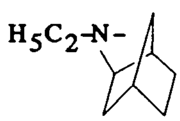 exo | 102 |

## TABLEAU VIII

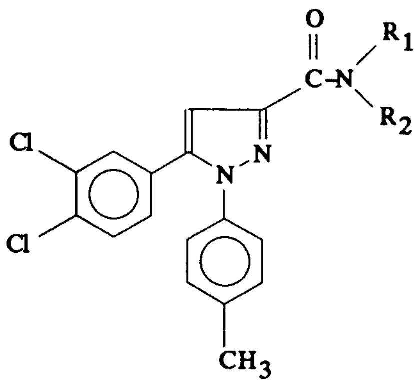

(Ia)

| Exemple n° | 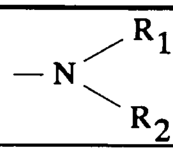 | F ; °C |
|---|---|---|
| 121 | 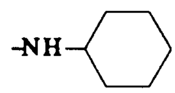 | 146 |
| 122 | 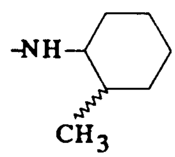 | 115 |
| 123 | 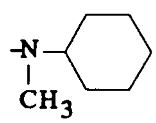 | 119 |
| 124 | 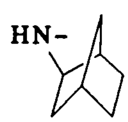 exo | 115 |
| 125 | 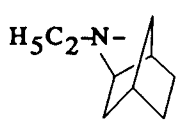 exo | 112 |

## TABLEAU IX

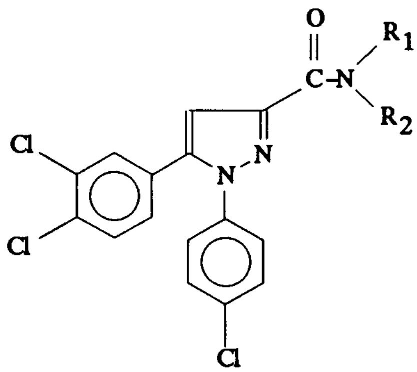

(Ia)

| Exemple n° | 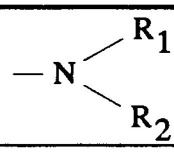 | F ; °C |
|---|---|---|
| 126 | 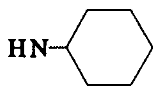 | 150 |
| 127 | 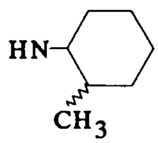 | 142 |
| 128 | 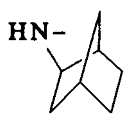 exo | 159 |
| 129 | 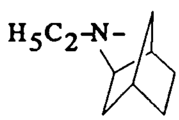 exo | 108 |

# TABLEAU X

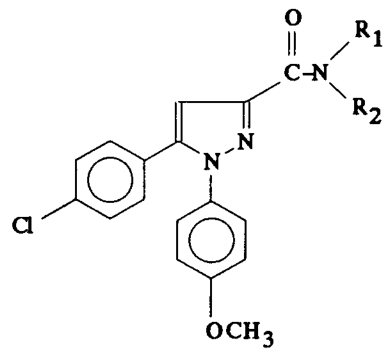

(Ia)

| Exemple n° | $-N(R_1)(R_2)$ | F ; °C |
|---|---|---|
| 130 | $-NH-(CH_2)_2-CH_3$ | 144 |
| 131 | 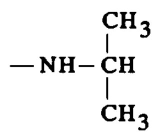 | 115 |
| 132 | 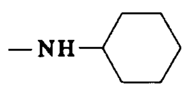 | 123 |
| 133 | 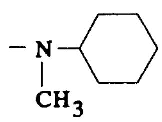 | 108 |
| 134 | 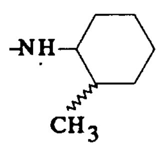 | 120 |
| 135 | 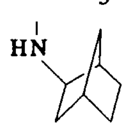 exo | 169 |
| 136 | 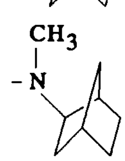 exo | 68 |

## TABLEAU X (suite)

| Exemple n° | 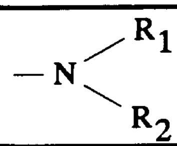 | F ; °C |
|---|---|---|
| 137 | 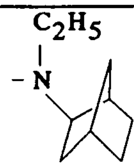 exo | 58 |
| 138 | 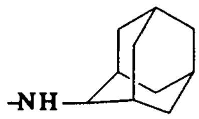 | 182 |
| 139 | 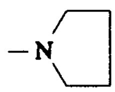 | 152 |

# TABLEAU XI

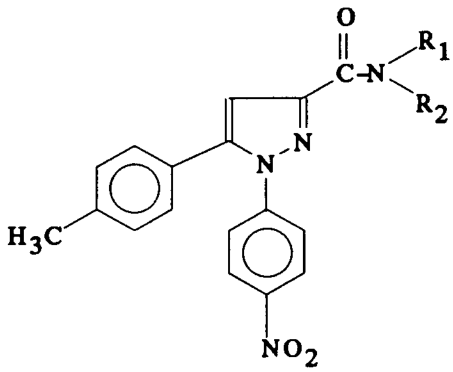

| Exemple n° | 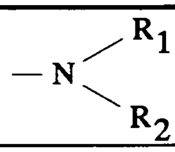 | F ; °C |
|---|---|---|
| 140 | 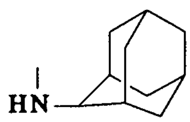 | 260 |
| 141 | 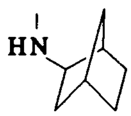 exo | 191 |
| 142 | 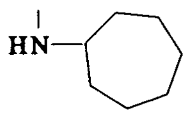 | 182 |

## TABLEAU XII

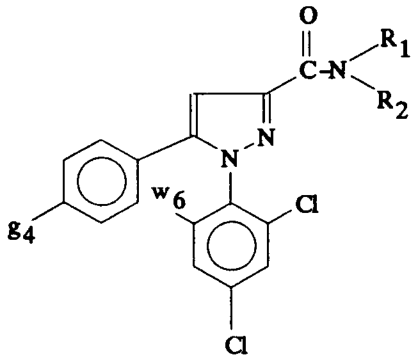

(Ia)

| Exemple n° | 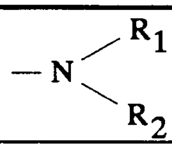 | g4 | w6 | F ; °C |
|---|---|---|---|---|
| 143 | 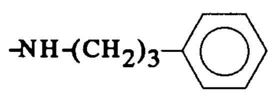 | Br | H | 130 |
| 144 | 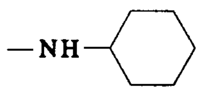 | Cl | Cl | 224 |
| 145 | 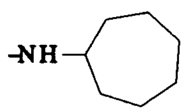 | Br | H | 148 |
| 146 | 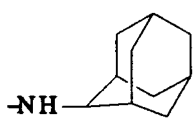 | Cl | Cl | 245 |
| 147 | 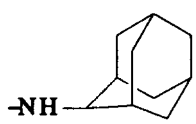 | Br | H | 206 |
| 148 | 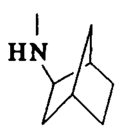 exo | Cl | Cl | 231 |
| 149 | 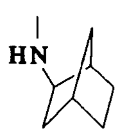 exo | Br | H | 201 |

**TABLEAU XII (suite)**

| Exemple n° | $-N(R_1)(R_2)$ | $g_4$ | $w_6$ | F ; °C |
|---|---|---|---|---|
| 150 | 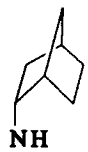 exo | 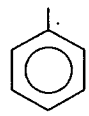 | H | 165 |
| 151 | 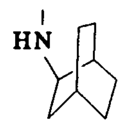 | Br | H | 209 |
| 152 | 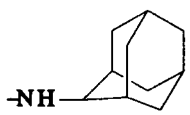 | 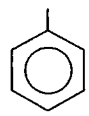 | H | 204 |

**EXEMPLE 153**

N-(2-adamantyl)-1-(2,4-dichlorophényl)-4-méthyl-5-(4-chlorophényl)-1*H*-pyrazole-3-carboxamide.

(I): $g_4$ = Cl; $w_2$, $w_4$ = Cl; $R_4$ = $CH_3$;

-X-CO-R = 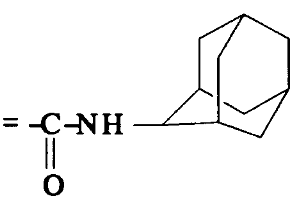

A) Sel de lithium du 2,4-dioxo-(4-chlorophényl)butanoate d'éthyle.

60 ml de solution du sel de lithium de l'hexaméthyldisilazane 1,0 M dans le THF sont introduits dans 240 ml d'éther anhydre. Le mélange est refroidi à -78°C et une solution de 10,12 g de 4-chloropropiophénone dans 50 ml d'éther est introduite goutte à goutte. Après 30 minutes d'agitation à -78°C, on introduit rapidement une solution de 9,16 ml d'oxalate de diéthyle dans 50 ml d'éther puis on laisse remonter la température et on agite pendant 5 heures à température ambiante. Le précipité jaune pâle formé est filtré, lavé à l'éther et séché sous vide. On obtient 6,32 g du sel attendu.

B) Ester éthylique de l'acide 1-(2,4-dichlorophényl)-4-méthyl-5-(4-chlorophényl)-1*H*-pyrazole-3-carboxylique.

Cet ester est obtenu de la même façon que dans l'exemple 1B) à partir du sel de lithium obtenu précédemment, et purifié par recristallisation dans l'éther isopropylique. F = 105°C.

C) Composé 153.

Cet amide est obtenu à partir de l'ester précédent de la même façon que dans l'exemple 1C), 1D) et 1E), par transformation de l'ester en chlorure d'acide et réaction de celui-ci avec la 2-adamantaneamine et purification par recristallisation dans l'éther isopropylique, F = 190°C.

En procédant selon l'exemple 153 ci-dessus on prépare les amides décrits dans le TABLEAU XIII ci-après.

## TABLEAU XIII

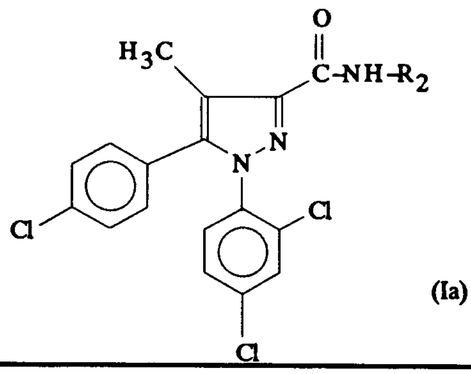

(Ia)

| Exemple n° | NH-$R_2$ | F ; °C |
|---|---|---|
| 154 | 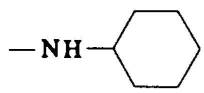 | 78 |
| 155 | 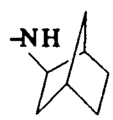 exo | 85 |
| 156 | 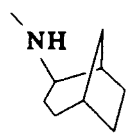 | 148 |
| 157 | 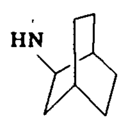 | 155 |
| 158 | 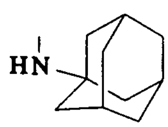 | 201 |

**EXEMPLE 159** N-[1-(para-tolyl)-5-(4-chlorophényl)-1*H*-pyrazol-3-ylméthyl]-N-méthylcyclo-hexylcarboxamide.

(I) : $g_4$ = Cl ; $w_4$ = $CH_3$ : $R_4$ = H ;

X-CO-R = 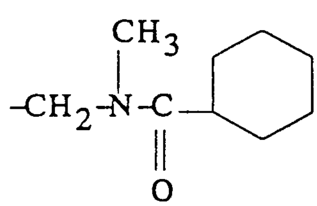

A) N-méthyl-1-(p-tolyl)-5-(4-chlorophényl)-1*H*-pyrazole-3-carboxamide.

0,50 g de chlorure de l'acide 1-(4-méthylphényl)-5-(4-chlorophényl)-3-pyrazolecarboxylique en solution dans 5 ml de $CH_2Cl_2$ sont ajoutés goutte à goutte à 100 ml d'une solution de méthylamine à 33 % dans l'éthanol. Après 2 heures d'agitation à température ambiante on concentre sous vide, reprend le résidu à l'aide d'un mélange $Na_2CO_3$ 5 % + AcOEt, décante, lave la phase organique avec une solution saturée de NaCl, sèche sur $MgSO_4$ et évapore les solvants. Le résidu est repris dans l'éther isopropylique, les cristaux obtenus sont filtrés et séchés sous vide. On obtient 0,44 g de l'amide attendu, F = 138°C.

B) N-méthyl-[1-(p-tolyl)-5-(4-chlorophényl)-1*H*-pyrazol-3-yl]méthylamine.

8,76 d'amide obtenu précédemment dissous dans 25 ml de THF anhydre sont ajoutés goutte à goutte à une température comprise entre 0 et 5°C à 75 ml d'une solution 1,0M de $BH_3$ dans le THF. Après retour à température ambiante, on chauffe le mélange réactionnel à reflux pendant 16 heures puis on coule en refroidissant au bain de glace 18 ml d'HCl 6N. On agite pendant 1 heure et demie à température ambiante puis distille le THF et concentre sous vide. Le mélange réactionnel est alors alcalinisé par NaOH en pastille jusqu'à pH = 9-10 ; on extrait à l'acétate d'éthyle, sèche sur $MgSO_4$, évapore les solvants et purifie le brut obtenu par chromatographie sur gel de silice (300 g), éluant : $CH_2Cl_2/CH_3OH$ 97/3 (v/v). On obtient 6,0 g d'amine, F = 85°C.

C) Composé 159

A une solution de 0,46 g d'amine précédente dans 10 ml de $CH_2Cl_2$ on ajoute successivement 0,62 ml de triéthylamine puis une solution de 0,23 g de chlorure d'acide cyclohexanoïque dans 5 ml de $CH_2Cl_2$. Après 15 minutes d'agitation à température ambiante, le mélange réactionnel est concentré sous vide et le résidu est repris par 30 ml d'eau et extrait à l'acétate d'éthyle. La phase organique est lavée successivement par $Na_2CO_3$ 5 %, à l'eau puis avec une solution saturée de NaCl, séchée sur $MgSO_4$ puis les solvants sont évaporés. Le produit brut est purifié par chromatographie sur gel de silice (25 g), éluant : toluène/AcOEt 70/30 (v/v). Les fractions de produit pur sont concentrées sous vide et le résidu est recristallisé dans l'éther isopropylique. On obtient 0,38 g d'amide, F=124°C.

En procédant selon l'EXEMPLE 159 ci-dessus, on prépare les amides décrits dans les TABLEAUX XIV et XV ci-dessous.

## TABLEAU XIV

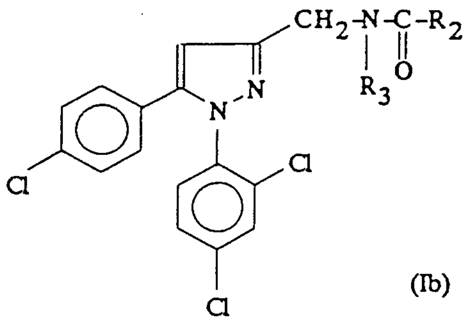

(Ib)

| Exemple n° | 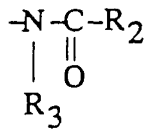 | F ; °C |
|---|---|---|
| 160 | 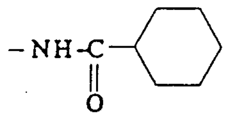 | 178 |
| 161 | 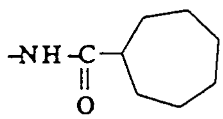 | 148 |
| 162 | 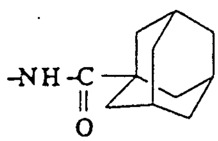 | 148 |
| 163 | 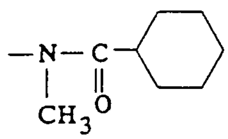 | 123 |
| 164 | 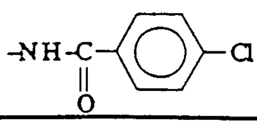 | 142 |

## TABLEAU XIV (suite)

| Exemple n° | 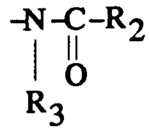 | F ; °C |
|---|---|---|
| 165 | 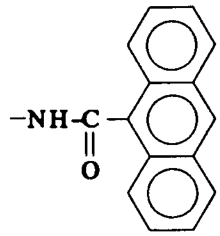 | 175 |
| 166 | 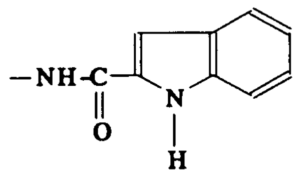 | 225 |
| 167 | 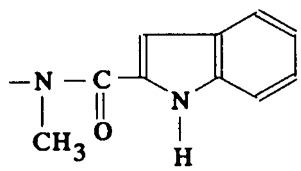 | 155 |
| 168 | 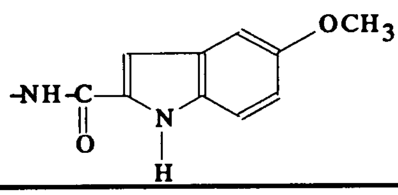 | 228 |

## TABLEAU XV

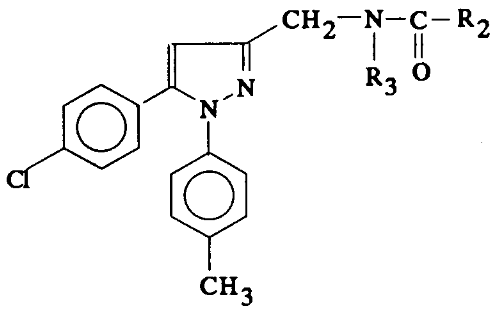

(Ib)

| Exemple n° | 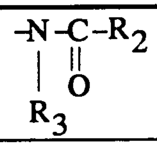 | F ; °C |
|---|---|---|
| 169 | 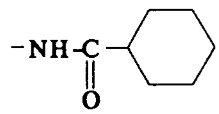 | 103 |
| 170 | 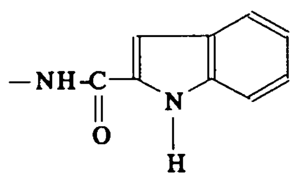 | 166 |
| 171 | 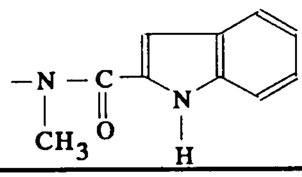 | 165 |

**EXEMPLE 172**

N-[1-(2,4-Dichlorophényl)-5-(4-chlorophényl)-1*H*-pyrazol-3-yl-méthyl]-N'-(4-chlorophényl)urée.

(I): $g_4$ = Cl; $w_2$, $w_4$ = Cl; $R_4$ = H;

-X-CO-R = 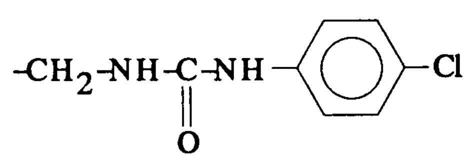

A) 1-(2,4-Dichlorophényl)-5-(4-chlorophényl)-1*H*-pyrazole-3-carboxamide.

Cet amide est obtenu de la même façon que l'exemple 159A par réaction du chlorure d'acide décrit dans l'exemple 1D avec une solution saturée d'ammoniac dans l'éthanol. F = 178°C.

B) [1-(2,4-Dichlorophényl)-5-(4-chlorophényl)-1*H*-pyrazol-3-yl]méthylamine.

Cette amine est obtenue de la même façon que l'exemple 159B par réduction de l'amide précédemment obtenue avec $BH_3$ dans le THF.

C) Composé 172.

0,20 g de 4-chlorophénylisocyanate sont additionnés à 0,45 g d'amine précédente en solution dans 10 ml de toluène et le mélange réactionnel est agité à température ambiante pendant 16 heures. Le solvant est évaporé et le résidu est repris dans 20 ml d'acétate d'éthyle, lavé à l'eau puis séché sur $MgSO_4$ et les solvants sont évaporés. Le résidu est purifié par chromatographie sur gel de silice (20 g), éluant : toluène/AcOEt 60/40 (v/v). La concentration des fractions de produit pur fournit un résidu qui est recristallisé dans un mélange isopropanoVéther isopropylique. On obtient 0,18 g d'urée attendue, F = 172°C.

En procédant selon l'EXEMPLE 172 ci-dessus, on prépare les urées décrites dans le TABLEAU XVI ci-après.

## TABLEAU XVI

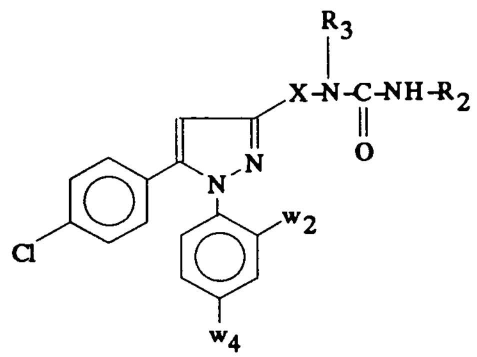

(Ic)

| Exemple n° | 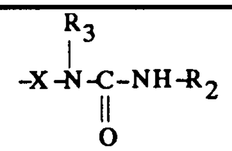 | $w_2$ | $w_4$ | F ; °C |
|---|---|---|---|---|
| 173 | 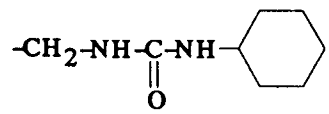 | Cl | Cl | 122 |
| 174 | 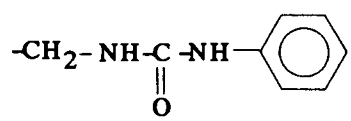 | Cl | Cl | 88 |

## TABLEAU XVI (suite)

| Exemple n° | 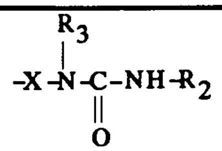 | $w_2$ | $w_4$ | F ; °C |
|---|---|---|---|---|
| 175 | 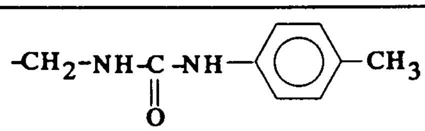 | Cl | Cl | 120 |
| 176 | 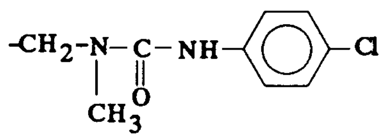 | Cl | Cl | 157 |
| 177 | 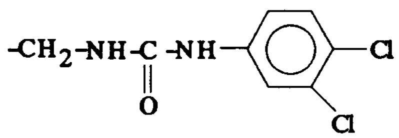 | Cl | Cl | 157 |
| 178 | 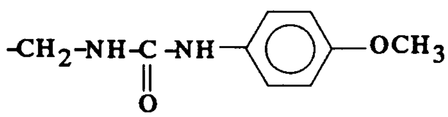 | Cl | Cl | 138 |
| 179 | 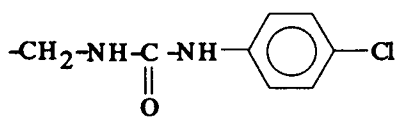 | H | $CH_3$ | 183 |
| 180 | 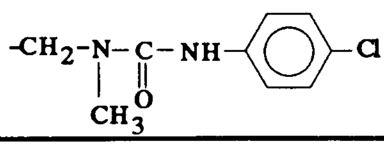 | H | $CH_3$ | 148 |

**EXEMPLE 181**

N-[1-(2,4-dichlorophényl)-5-(4-chlorophényl)-1*H*-pyrazol-3-yl]cyclohexylcarboxamide.

(I): $g_4$ = Cl; $w_2$, $w_4$ = Cl; $R_4$ = H;

-X-CO-R = 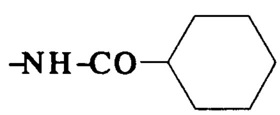

A) N-(Tertiobutoxycarbonyl)-[1-(2,4-dichlorophényl)-5-(4-chlorophényl)-1*H*-pyrazol-3-yl]amine.

A une solution de 2,05 ml d'azidure de diphénylphosphoryle dans 40 ml de t-butanol anhydre, on ajoute 3,25 g de l'acide 1-(2,4-dichlorophényl)-5-(4-chlorophényl)-3-pyrazolecarboxylique obtenu selon l'exemple 1C) puis 1,32 ml de triéthylamine et on chauffe le mélange réactionnel à reflux sous azote pendant 12 heures. Après refroidissement, le mélange réactionnel est traité par une solution saturée de $NaHCO_3$ et extrait à l'acétate d'éthyle. Après lavage à l'eau puis par une solution de NaCl saturée, séchage sur $MgSO_4$ et évaporation des solvants, le produit brut est purifié par chromatographie sur gel de silice 70-230 mesh, éluant : $CH_3OH/CH_2Cl_2$ 1/99 (v/v). On obtient 1,09 g du produit attendu.

B) Chlorhydrate de 1-(2,4-dichlorophényl)-5-(4-chlorophényl)- 1*H*-pyrazol-3-yl-ammonium.

1,09 g du produit précédent sont dissous dans 20 ml d'une solution saturée d'HCl dans EtOH diluée à 50 % et le mélange réactionnel est chauffé à reflux pendant 2 heures. Le solvant est ensuite évaporé et le résidu est trituré dans l'acétate d'éthyle à reflux puis filtré et séché sous vide. On obtient 0,55 g du chlorhydrate.

C) Composé 181.

Une solution de 0,11 ml de chlorure de l'acide cyclohexanecarboxylique dans 2 ml de $CH_2Cl_2$ est ajoutée goutte à goutte à une solution de 0,20 g du chlorhydrate obtenu dans l'exemple précédent et 0,19 ml de triéthylamine dans 5 ml de $CH_2Cl_2$. Après 24 heures d'agitation à température ambiante le mélange est lavé successivement avec une solution d'HCl 5 %, à l'eau, avec une solution de $Na_2CO_3$ 5 % puis avec une solution de NaCl saturée, séché sur $MgSO_4$, puis les solvants sont évaporés. Le produit brut est cristallisé dans $iPr_2O$. On obtient 0,12 g de l'amide attendu, F = 213°C.

**EXEMPLE 182**

N-méthyl-N-[1-(2,4-dichlorophényl)-5-(4-chlorophényl)-1*H*-pyrazol-3-yl]adamantyl-1-carboxamide.

(I):$w_2 = w_4 = Cl$; $g_4 = Cl$; $R_4 = H$;

$-X-CO-R =$ 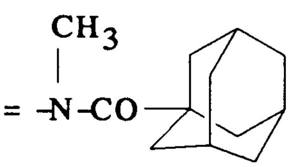

A) N-[1-(2,4-dichlorophényl)-5-(4-chlorophényl)-1*H*-pyrazol-3-yl]formamide.

Dans un mélange de 4 ml d'acide formique et 0,5 ml d'anhydride acétique refroidi au bain de glace on ajoute par petites portions 0,50 g de 1-(2,4-dichlorophényl)-5-(4-chlorophényl)-1*H*-pyrazol-3-ylamine obtenu dans l'exemple précédent. Après 30 mn d'agitation les solvants sont évaporés sous vide et le résidu est repris dans l'éther isopropylique. Le solide blanc obtenu est filtré, lavé à l'éther isopropylique et séché sous vide. On obtient 0,49 g du formamide attendu, F = 181°C.

B) N-méthyl-[1-(2,4-dichlorophényl)-5-(4-chlorophényl)-1*H*-pyrazol-3-yl]amine.

Une solution de 1,15 g du formamide obtenu dans l'exemple précédent dans 10 ml de THF anhydre est ajoutée goutte à goutte à température ambiante sur une suspension de 0,24 g de $LiAlH_4$ dans 40 ml de THF anhydre. Le mélange est ensuite chauffé à reflux pendant 20 minutes, refroidi à 0°C et hydrolysé par 0,24 ml d'eau puis 0,24 ml de NaOH à 15 % puis 0,72 ml d'eau. Après 20 minutes d'agitation à température ambiante, on filtre, lave au THF et évapore le filtrat à sec. Le résidu est repris dans l'éther isopropylique, filtré et séché sous vide. On obtient 1,02 g d'amine attendue, F = 157°C.

C) Composé 182

En procédant comme dans l'exemple 181C, la réaction de l'amine précédemment obtenue avec le chlorure de l'acide adamantane-1-carboxylique conduit à l'amide attendu qui est purifié par chromatographie sur colonne de silice ; éluant : AcOEt/toluène 7:93, F = 65°C.

## TABLEAU XVII

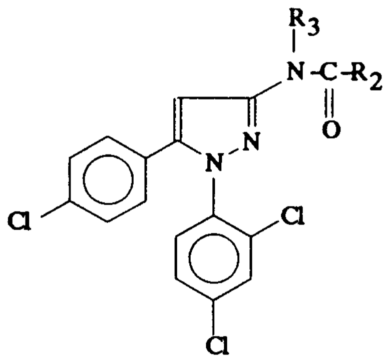

(Id)

| Exemple n° | $R_3$ | $R_2$ | F; °C |
|---|---|---|---|
| 183 | H | 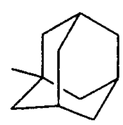 | 284 |
| 184 | H | 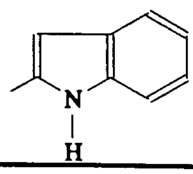 | 291 |

## TABLEAU XVII (suite)

| Exemple n° | $R_3$ | $R_2$ | F; °C |
|---|---|---|---|
| 185 | H | 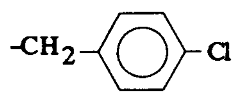 | 164 |
| 186 | $CH_3$ | 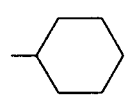 | 127 |

**EXEMPLE 187**

N-méthyl-[1-(2,4-dichlorophényl)-5-(4-chlorophényl)-1*H*-pyrazol-3-yl]-N'-(4-chlorophényl)urée.

(I):$w_2$=$w_4$=Cl;$g_4$=Cl;$R_4$=H;

-X-CO-R = -NH-CO-NH 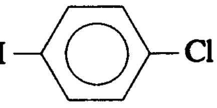

A une suspension de 0,40 g de 1-(2,4-dichlorophényl)-5-(4-chlorophényl)-1*H*-pyrazol-3-ylamine obtenue par neutralisation du chlorhydrate obtenu dans l'exemple 181B dans 15 ml de toluène on ajoute 225 mg de 4-chlorophénylisocyanate et on chauffe le mélange à 40°C pendant 1 heure puis on laisse réagir à température ambiante pendant 16 heures. Le précipité obtenu est filtré, lavé au toluène et séché sous vide. On obtient 0,46 g de l'urée attendue, F = 215°C.

**EXEMPLE 188**

N-[1-(2,4-dichlorophényl)-5-(4-chlorophényl)-1*H*-pyrazol-3-yl]-N'-(1-adamantyl)urée.

(I):$w_2=w_4=Cl;g_4=Cl;R_4=H;$

-X-CO-R = NH-CO-NH 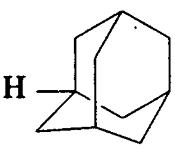

A) A une solution de 10,0 g du chlorure d'acide obtenu selon l'exemple 1D dans 320 ml d'acétone refroidie à 0°C on ajoute une solution de 2,54 g d'azoture de sodium dans 10 ml d'eau. Après 1 heure d'agitation à 0°C, le précipité obtenu est filtré et lavé à l'acétone puis séché sous vide. On obtient 9,86 g de l'azoture d'acyle attendu.

B) N-[1-(2,4-dichlorophényl)-5-(4-chlorophényl)-1*H*-pyrazol-3-yl]-N'-(1-adamantyl)-urée.

Une solution de 1,00 g d'azoture d'acyle obtenu dans l'exemple précédent dans 5 ml de toluène est chauffée à reflux pendant 30 minutes. Après retour à température ambiante on ajoute à la solution d'isocyanate ainsi obtenue 0,39 g de 1-adamantanamine et agite le mélange pendant 1 heure et demie. Le précipité obtenu est filtré, lavé au toluène puis à l'éther isopropylique puis purifié par triturage dans un mélange acétone/méthanol. Après séchage sous vide, on obtient 0,48 g de l'urée attendue, F = 244°C.

# TABLEAU XVIII

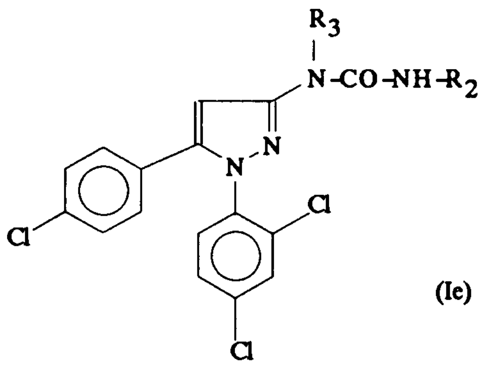

(Ie)

| Exemple n° | $R_3$ | $R_2$ | F; °C |
|---|---|---|---|
| 189 | H | 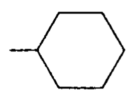 | 227 |
| 190 | $CH_3$ | 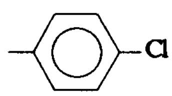 | 144 |

**EXEMPLE 191**

1-Cyclohexylméthyl-[1-(2,4-dichlorophényl)-5-(4-chlorophényl)-1*H*-pyrazol-3-yl]-cétone.

(I):$g_4$=Cl;$w_2$,$w_4$=Cl;$R_4$=H;

-X-CO-R = 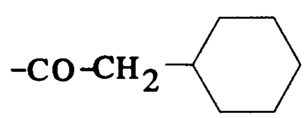

2,5 ml d'une solution 0,625 M de $MnLi_2Cl_4$ dans le THF (Tetrahedron 1989, *45,* 4163) sont refroidis à 0°C et on y ajoute goutte à goutte 3,12 ml d'une solution 0,50M de bromure de méthylcyclohexylmagnésium dans le THF puis le mélange réactionnel est agité à 0°C pendant 2 heures. Le mélange réactionnel est alors refroidi à - 10°C et une solution de 0,50 g du chlorure d'acide préparé selon l'exemple 1D), dans 8 ml de THF, est ajoutée goutte à goutte. Le mélange est agité à température ambiante pendant 5 heures puis hydrolysé par une solution saturée de $NH_4Cl$, extrait à l'éther, lavé à l'eau puis par une solution saturée de NaCl. Après séchage sur $MgSO_4$ et évaporation des solvants, le produit brut est purifié par chromatographie sur gel de silice 230-400 mesh, éluant : AcOEt/hexane 5/95 (v/v). On obtient ainsi 0,09 g de cétone attendue, F = 118°C.

**EXEMPLE 192**

1-[1-(2,4-Dichlorophényl)-5-(4-chlorophényl)-1*H*-pyrazol-3-yl]-2-(méthyl-4-phényl)-1-éthanone.

(I):$g_4$=Cl;$w_2$,$w_4$=Cl;$R_4$=H;

-X-CO-R = 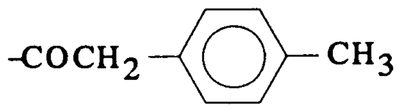

A) 1-(2,4-Dichlorophényl)-3-cyano-5-(4-chlorophényl)-pyrazole.

Une solution de 0,70 g de 1-(2,4-dichlorophényl)-5-(4-chlorophényl)-1*H*-pyrazole-3-carboxamide obtenu selon l'exemple 172A et 0,74 ml de chlorure de mésyle dans 6 ml de pyridine est chauffée à 50°C pendant 8 heures. Le solvant est évaporé sous vide et le résidu dissous dans 20 ml de $CH_2Cl_2$. On lave successivement avec une solution d'HCl à 5 % puis à l'eau, puis par une solution saturée de NaCl, on sèche sur $MgSO_4$ puis évapore le solvant. Le résidu est cristallisé dans l'éther isopropylique. On obtient 0,66 g du nitrile attendu, F = 123°C.

B) Composé 192

6,3 ml d'une solution de chlorure de méthyl-4-benzylmagnésium, 1,0M dans l'éther éthylique sont ajoutés goutte à goutte sur une solution de 0,73 g du nitrile précédent dans 20 ml d'éther éthylique. Après 2 heures de réaction à température ambiante le mélange est hydrolysé par 50 ml d'acide chlorhydrique à 5 % et le mélange biphasique résultant est agité pendant 30 minutes. Le précipité rose formé est filtré, lavé à l'eau et à l'éther éthylique puis dissous dans 100 ml de $CH_2Cl_2$ et agité 30 minutes en présence d'environ 10 g de silice humide. La silice est ensuite filtrée, le filtrat évaporé et le résidu cristallisé dans un mélange $CH_2Cl_2/iPr_2O$. On obtient 0,37 g de la cétone attendue. F = 175°C.

## TABLEAU XIX

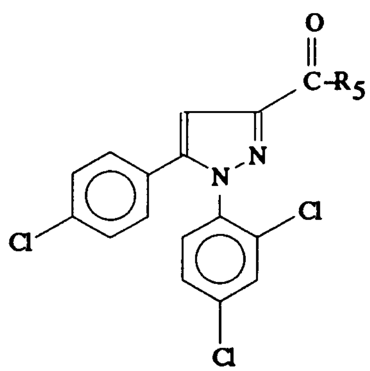

(If)

| Exemple n° | $R_5$ | F ; °C |
|---|---|---|
| 193 | $-CH_2-CH_2-$ 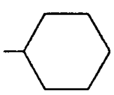 | 129 |
| 194 | $-CH_2-$ 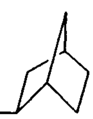 | 152 |

## Revendications

1. Composé de formule :

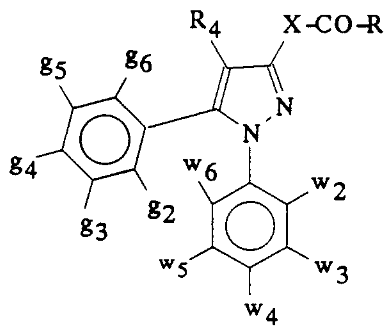

(I)

dans laquelle

- $g_2$, $g_3$, $g_4$, $g_5$, $g_6$ et $w_2$, $w_3$, $w_4$, $w_5$, $w_6$ sont identiques ou différents, et représentent indépendamment l'hydrogène, un atome de chlore ou de brome, un ($C_1$-$C_3$) alkyle, un ($C_1$-$C_3$) alcoxy, un trifluorométhyle, un groupe nitro et $g_4$ représente éventuellement un groupe phényle ;
- $R_4$ représente l'hydrogène ou un ($C_1$-$C_3$) alkyle ;
- X représente soit une liaison directe soit un groupe $-(CH_2)_x-N(R_3)-$dans lequel $R_3$ représente l'hydrogène ou

un $(C_1-C_3)$ alkyle et x représente zéro ou un ;

- R représente

  . un groupe $-NR_1R_2$, dans lequel $R_1$ et $R_2$ représentent indépendamment un $(C_1-C_6)$ alkyle ; un radical carbocyclique non aromatique en $(C_3-C_{15})$ éventuellement substitué, le(s)dit(s) substituant(s) étant différent(s) d'un carbonyle substitué ; un groupe amino $(C_1-C_4)$ alkyle dans lequel l'amino est éventuellement disubstitué par un $(C_1-C_3)$ alkyle ; un cycloalkyl$(C_1-C_3)$alkyle dans lequel le cycloalkyle est en $(C_3-C_{12})$; un phényle non substitué ou substitué une ou plusieurs fois par un halogène, par un $(C_1-C_5)$ alkyle, ou par un $(C_1-C_5)$ alcoxy ; un phényl $(C_1-C_3)$ alkyle ; un diphényl $(C_1-C_3)$ alkyle ; un naphtyle ; un anthracényle ; un radical hétérocyclique saturé de 5 à 8 chaînons non substitué ou substitué par un $(C_1-C_3)$ alkyle, un hydroxyle ou un benzyle; un 1-adamantylméthyle ; un hétérocyle aromatique non substitué ou substitué une ou plusieurs fois par un halogène, un $(C_1-C_5)$ alkyle ou un $(C_1-C_5)$ alcoxy ; un $(C_1-C_3)$ alkyle substitué par un hétérocycle aromatique non substitué ou substitué une ou plusieurs fois par un halogène, un $(C_1-C_5)$ alkyle ou un $(C_1-C_5)$ alcoxy ; ou bien $R_1$ est l'hydrogène et $R_2$ est tel que défini ci-dessus ; ou bien encore $R_1$ et $R_2$ constituent avec l'atome d'azote auquel ils sont liés, un radical hétérocyclique saturé de 5 à 8 chaînons, ledit radical hétérocyclique étant autre que morpholinyle lorsque $w_2$, $w_3$, $w_4$, $w_5$, $w_6$, $g_2$, $g_3$, $g_4$, $g_5$ et $g_6$ sont tous hydrogène ;
  . un groupe $R_2$ tel que défini ci-dessus lorsque X représente $-(CH_2)_xN(R_3)-$ ;
  . un groupe $R_5$ lorsque X représente une liaison directe, $R_5$ étant représenté par un $(C_1-C_3)$ alkyle ; un $(C_3-C_{12})$ cycloalkyle non substitué ou substitué par un $(C_1-C_5)$ alkyle ; un phényl $(C_1-C_3)$ alkyle non substitué ou substitué par un halogène ou par un $(C_1-C_5)$ alkyle ; un cycloalkyl $(C_1-C_3)$ alkyle dans lequel le cycloalkyle est en $C_3-C_{12}$ et est non substitué ou substitué par un $(C_1-C_5)$ alkyle ; un 2-norbornylméthyle ;

ou un de ses sels.

2. Composé selon la revendication 1 de formule :

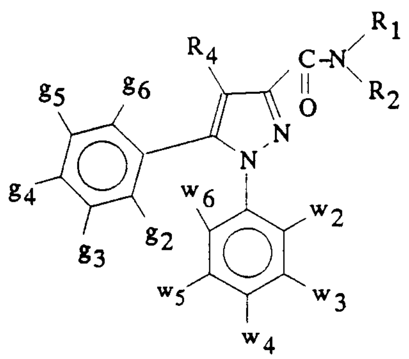

(Ia')

dans laquelle $w_2$, $w_3$, $w_4$, $w_5$, $W_6$, $g_2$, $g_3$, $g_4$, $g_5$, $g_6$ et $R_4$ sont tels que définis pour (I) dans la revendication 1, $R_1$ est l'hydrogène ou un $C_1-C_6$ alkyle et $R_2$ est un radical carbocyclique non aromatique en $C_3-C_{15}$ ou un radical hétérocyclique saturé de 5 à 8 chaînons choisi parmi 1-pyrrolidinyle, 1-pipéridinyle, 1-hexahydroazépinyle, 4-morpholinyle et 4-thiomorpholinyle ou un de ses sels.

3. Composé selon la revendication 1 de formule :

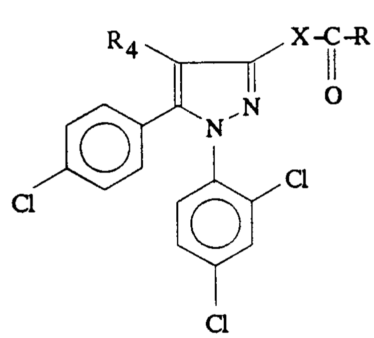

(i)

dans laquelle $R_4$, X et R sont tels que définis pour (I) dans la revendication 1 ou un de ses sels.

**4.** Composé de formule (i) selon la revendication 3 dans laquelle $R_4$ représente l'hydrogène ou un groupe méthyle ou un de ses sels.

**5.** Composé de formule (i) selon la revendication 4 dans laquelle $R_3$ est hydrogène ou méthyle et X représente une liaison directe ou un de ses sels.

**6.** Composé de formule (i) selon la revendication 3 dans laquelle $R_4$ est hydrogène ou méthyle, X est liaison directe et R représente un groupe - $NR_1R_2$ dans lequel $R_1$ est l'hydrogène ou un groupe méthyle et $R_2$ est un radical carbocyclique non aromatique en $C_3$-$C_{15}$ ou un radical hétérocyclique saturé de 5 à 8 chaînons choisi parmi 1-pyrrolidinyle, 1-pipéridyle, 1-hexahydroazépinyle, 4-morpholinyle et 4-thiomorpholinyle ou un de ses sels.

**7.** Composé de formule (i) selon la revendication 3 dans lequel $R_4$ est hydrogène ou méthyle, X est $-(CH_2)_x-N(R_3)-$, R est $-NR_1R_2$, x étant zéro ou un, $R_1$ étant l'hydrogène, $R_3$ étant l'hydrogène ou un groupe méthyle et $R_2$ étant un phényle non substitué ou substitué par un ou deux atomes d'halogène, un groupe ($C_1$-$C_5$)alkyle ou un groupe ($C_1$-$C_5$)alcoxy ou un radical carbocyclique non aromatique en $C_3$-$C_{15}$ ou un de ses sels.

**8.** Composé selon la revendication 1 de formule :

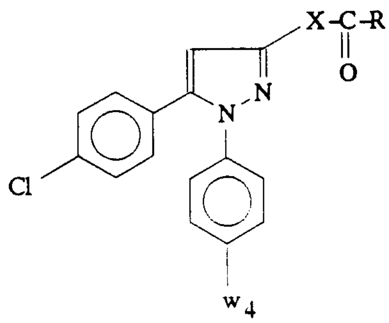

(ii)

dans laquelle X et R sont tels que définis dans la revendication 1, et $w_4$ représente un groupe méthyle ou un groupe méthoxy ou un de ses sels.

**9.** Composé de formule (ii) selon la revendication 8 dans laquelle $w_4$ est méthyle ou méthoxy, X représente une liaison directe et R représente un groupe $-NR_1R_2$ dans lequel $R_1$ représente l'hydrogène ou un groupe méthyle et $R_2$ représente un radical carbocyclique non aromatique en $C_3$-$C_{15}$ ou un de ses sels.

**10.** Composé selon la revendication 8 dans lequel $w_4$ est méthyle ou méthoxy, X représente un groupe $-(CH_2)_x-N(R_3)-$ dans lequel x est zéro ou un et $R_3$ représente l'hydrogène ou un groupe méthyle et R représente un groupe $-NR_1R_2$ où $R_1$ représente l'hydrogène et $R_2$ représente un phényle non substitué ou substitué par un ou deux atomes d'halogène, un groupe ($C_1$-$C_5$) alkyle ou un ($C_1$-$C_5$) alcoxy ou un radical carbocyclique non aromatique en $C_3$-$C_{15}$, ou un de ses sels.

**11.** Composé selon la revendication 1 de formule (I) dans laquelle $w_2$, $w_3$, $w_4$, $w_5$, $w_6$, $g_2$, $g_3$, $g_4$, $g_5$, $g_6$, $R_4$ et X sont tels que définis dans la revendication 1 et R représente un groupe $-NR_1R_2$ dans lequel $R_1$ est l'hydrogène ou un groupe ($C_1$-$C_6$) alkyle et $R_2$ est un groupe 2- ou 3-indolyl ($C_1$-$C_3$) alkyle ou un groupe 2- ou 3-indolyle ou un de ses sels.

**12.** Composé selon la revendication 1 de formule :

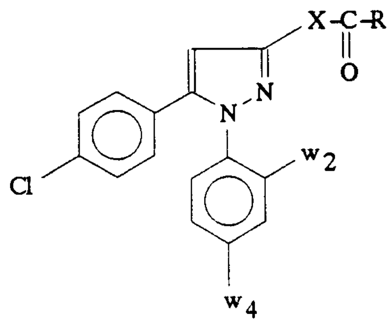

(iii)

dans laquelle X est tel que défini dans la revendication 1, R est un groupe $-NR_1R_2$ dans lequel $R_1$ représente l'hydrogène ou un $(C_1-C_6)$ alkyle et $R_2$ est un groupe 2- ou 3-indolyl $(C_1-C_3)$ alkyle ou un groupe 2- ou 3-indolyle et soit $w_2$ est l'hydrogène et $w_4$ est un groupe méthyle ou méthoxy soit $w_2$ et $w_4$ représentent un atome de chlore, ou un de ses sels.

**13.** Composé selon la revendication 1 de formule :

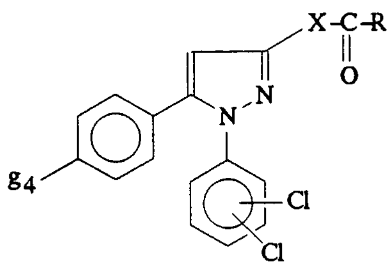

(iv)

dans laquelle X et R sont tels que définis dans la revendication 1 et $g_4$ représente un atome de brome, un groupe méthyle ou trifluorométhyle ou un de ses sels.

**14.** Procédé pour la préparation des composés de formule (I) selon la revendication 1, caractérisé en ce que on traite un dérivé de l'acide 3-pyrazolecarboxylique de formule :

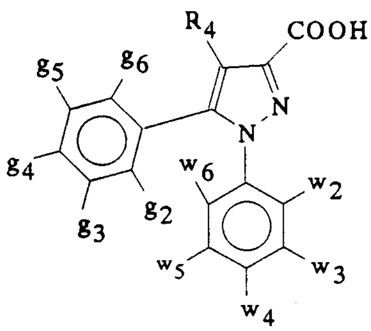

(IIa)

dans laquelle $w_2$, $w_3$, $w_4$, $w_5$ $w_6$, $g_2$, $g_3$, $g_4$, $g_5$, $g_6$ et $R_4$ sont tels que définis pour (I) dans la revendication 1 ou une de ses formes activées, un de ses esters ou chlorures d'acides avec

. soit avec une amine de formule $HNR_1R_2$ dans laquelle $R_1$ et $R_2$ sont tels que définis pour (I) dans la revendication 1 pour obtenir les amides de formule :

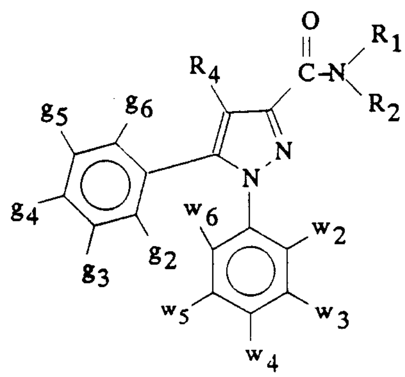

(Ia)

dans laquelle $w_2$, $w_3$, $w_4$, $w_5$, $w_6$, $g_2$, $g_3$, $g_4$, $g_5$, $g_6$, $R_4$, $R_1$ et $R_2$, sont tels que définis pour (I),

. soit éventuellement avec une amine primaire $R_3NH_2$ dans laquelle $R_3$ est tel que défini pour (I) dans la revendication 1 pour obtenir les amides intermédiaires (V) de formule :

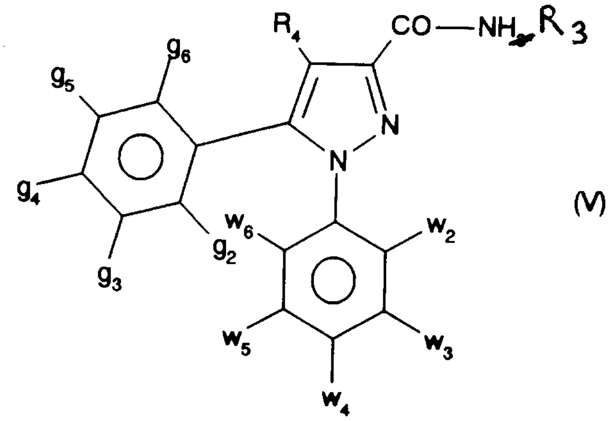

(V)

dans laquelle $w_2$, $w_3$, $w_4$, $w_5$, $w_6$, $g_2$, $g_3$, $g_4$, $g_5$, $g_6$, $R_4$ et $R_3$ sont tels que définis pour (I) pour obtenir par réduction par un hydrure métallique les amines intermédiaires de formule :

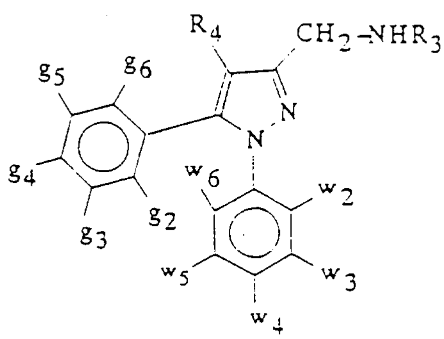

(VI)

dans laquelle $w_2$, $w_3$, $w_4$, $w_5$, $w_6$, $g_2$, $g_3$, $g_4$, $g_5$, $g_6$, $R_4$ et $R_3$ sont tels que définis pour (I),
qui sont transformées en amide ou en urée de formule :

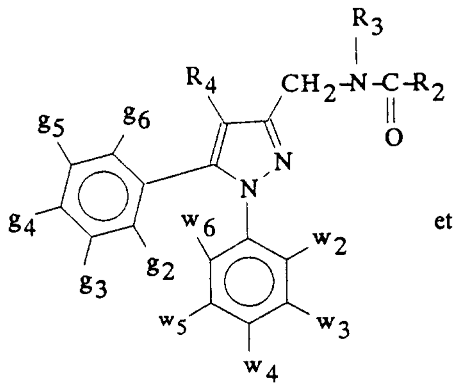

(Ib)

et

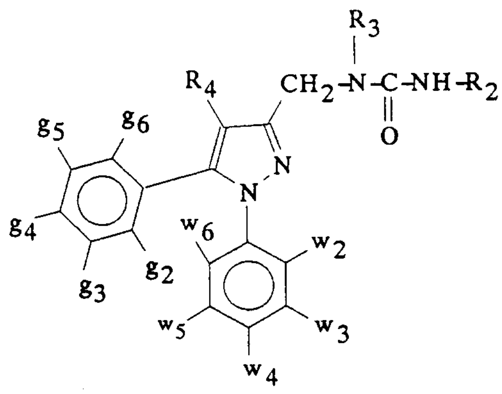

(Ic)

dans lesquelles $w_2$, $w_3$, $w_4$, $w_5$, $w_6$, $g_2$, $g_3$, $g_4$, $g_5$, $g_6$, $R_2$, $R_3$ et $R_4$ sont tels que définis pour (I) par réaction avec respectivement un chlorure d'acide de formule $R_2COCl$ ou un isocyanate de formule $R_2$-N=C=O dans lesquels $R_2$ est tel que défini pour (I),

. soit avec un dérivé de l'azidure de diphénylphosphoryle en milieu basique suivi d'un traitement acide en milieu alcoolique pour obtenir l'amine intermédiaire de formule :

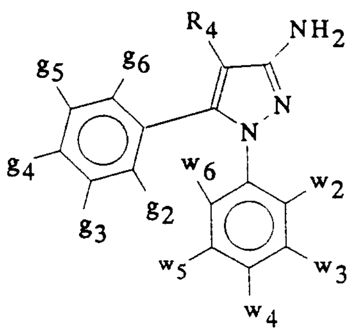

(VII)

dans laquelle $w_2$, $w_3$, $w_4$, $w_5$, $w_6$, $g_2$, $g_3$, $g_4$, $g_5$, $g_6$ et $R_4$ sont tels que définis pour (I), qui est soumis à l'action d'un chlorure d'acide $R_2COCl$ ou d'un isocyanate

$R_2$-N=C=O pour obtenir respectivement des amides et les urées de formule :

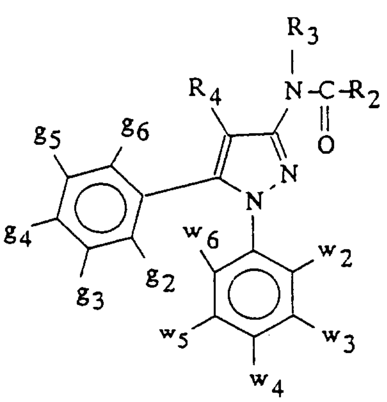

(Id)

et

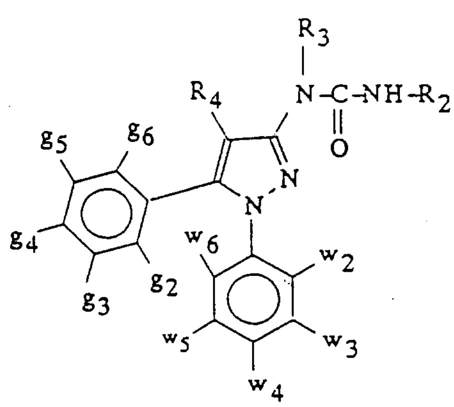

(Ie)

dans lesquelles $w_2$, $w_3$, $w_4$, $w_5$, $w_6$, $g_2$, $g_3$, $g_4$, $g_5$, $g_6$ et $R_4$ sont tels que définis pour (I) et $R_3$ représente l'hydrogène, les mêmes composés pour lesquels $R_3$ est différent de l'hydrogène étant préparés à partir de l'amine

primaire (VII) ci-dessus transformée en amine secondaire de formule :

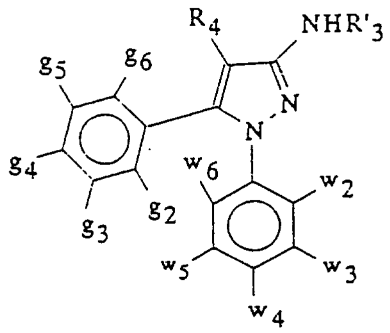

(VIIb)

dans laquelle $w_2$, $w_3$, $w_4$, $w_5$, $w_6$, $g_2$, $g_3$, $g_4$, $g_5$, $g_6$, $R_2$ et $R_4$ sont tels que définis pour (I) et $R'_3$ représente un ($C_1$-$C_2$) alkyle, qui sont ensuite soumises à l'action d'un chlorure d'acide $R_2COCl$ ou d'un isocyanate $R_2$-N=C=O pour obtenir les amides et les urées de formule (Id) et (Ie) telles que définies ci-dessus dans lesquelles $R_3$ est différent de l'hydrogène,

. soit avec un réactif organomanganeux $R_5MnX_1$ dans lequel $R_5$ est tel que défini pour (I) dans la revendication 1 et $X_1$ représente un halogène pour obtenir les dérivés cétoniques de formule :

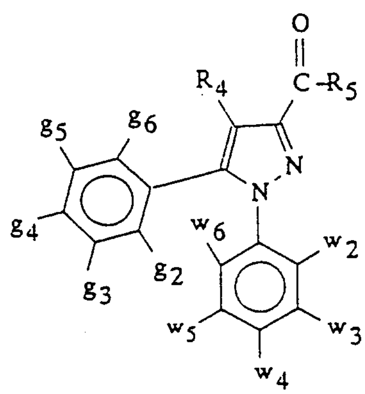

les composés ainsi obtenus étant ensuite éventuellement transformés en l'un de leurs sels.

**15.** Composés de formule :

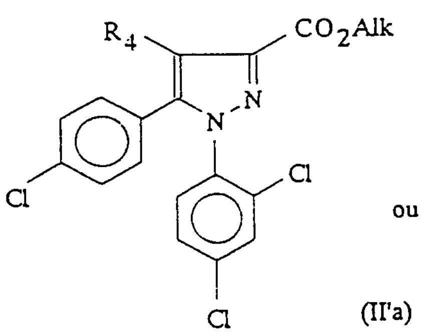

(II'a)

ou

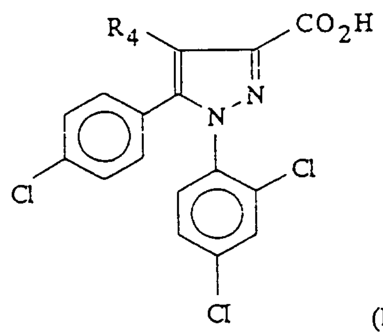

(II'b)

dans laquelle $R_4$ est tel que défini dans la revendication 1 et Alk représente un ($C_1$-$C_5$) alkyle.

**16.** Composition pharmaceutique contenant, en tant que principe actif, un composé de formule (I) selon l'une des revendications 1 à 13.

**17.** Composition pharmaceutique selon la revendication 16, sous forme d'unité de dosage, dans laquelle le principe actif est mélangé à au moins un excipient pharmaceutique.

**18.** Composition selon la revendication 17 contenant de 0,5 à 1000 mg de principe actif.

## Claims

**1.** Compound of the formula

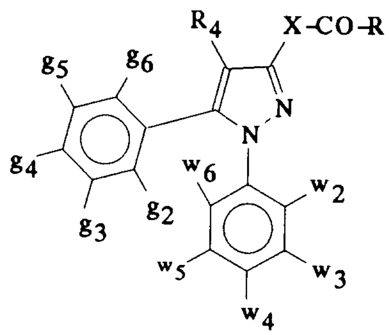

(I)

in which

- $g_2$, $g_3$, $g_4$, $g_5$, $g_6$ and $w_2$, $w_3$, $w_4$, $w_5$, $w_6$ are identical or different and are independently hydrogen, a chlorine or bromine atom, a ($C_1$-$C_3$) alkyl, a ($C_1$-$C_3$) alkoxy, a trifluoromethyl or a nitro group and $g_4$ is optionally a phenyl group;
- $R_4$ is hydrogen or a ($C_1$-$C_3$) alkyl;
- X is either a direct bond or a group $-(CH_2)_x-N(R_3)-$, in which $R_3$ is hydrogen or a ($C_1$ -$C_3$) alkyl and x is zero or one;
- R is

  . a group $-NR_1R_2$ in which $R_1$ and $R_2$ are independently a ($C_1$-$C_6$) alkyl; a non-aromatic ($C_3$-$C_{15}$) carbocyclic radical which is optionally substituted, said substituent(s) being other than a substituted carbonyl; an amino ($C_1$-$C_4$) alkyl group in which the amino is optionally disubstituted by a ($C_1$-$C_3$) alkyl; a cycloalkyl ($C_1$-$C_3$) alkyl in which the cycloalkyl is $C_3$-$C_{12}$; a phenyl which is unsubstituted or monosubstituted or polysubstituted by a halogen, by a ($C_1$-$C_5$) alkyl or by a ($C_1$-$C_5$) alkoxy; a phenyl ($C_1$-$C_3$) alkyl; a dipheny ($C_1$-$C_3$) alkyl; a naphthyl; an anthracenyl; a saturated 5- to 8-membered heterocyclic radical which is unsubstituted or substituted by a ($C_1$-$C_3$) alkyl, by a hydroxyl or by a benzyl; a 1-adamantylmethyl; an aromatic heterocycle which is unsubstituted or monosubstituted or polysubstituted by a halogen, by a ($C_1$-$C_5$) alkyl or by a ($C_1$-$C_5$) alkoxy; a ($C_1$-$C_3$) alkyl which is substituted by an aromatic heterocycle which is unsubstituted or monosubstituted or polysubstituted by a halogen, by a ($C_1$-$C_5$) alkyl or by a ($C_1$-$C_5$) alkoxy; or else $R_1$ is hydrogen and $R_2$ is as defined above; or else $R_1$ and $R_2$ form a saturated 5- to 8-membered heterocyclic radical with the nitrogen atom to which they are bonded, said heterocyclic radical being other than morpholine when $w_2$, $w_3$, $w_4$, $w_5$, $w_6$, $g_2$, $g_3$, $g_4$, $g_5$, and $g_6$ are all hydrogen;

  . a group $R_2$ as defined above when X is $-(CH_2)_xN(R_3)-$;

  . a group $R_5$ when X is a direct bond, $R_5$ being a ($C_1$-$C_3$) alkyl; a ($C_3$-$C_{12}$) cycloalkyl which is unsubstituted or substituted by a ($C_1$-$C_5$) alkyl; a phenyl ($C_1$-$C_3$) alkyl which is unsubstituted or substituted by a halogen or by a ($C_1$-$C_5$) alkyl; a cycloalkyl ($C_1$-$C_3$) alkyl in which the cycloalkyl is $C_3$-$C_{12}$ and is unsubstituted or substituted by a ($C_1$-$C_5$) alkyl; or a 2-norbornylmethyl;

or one of its salts.

**2.** Compound according to claim 1 of the formula

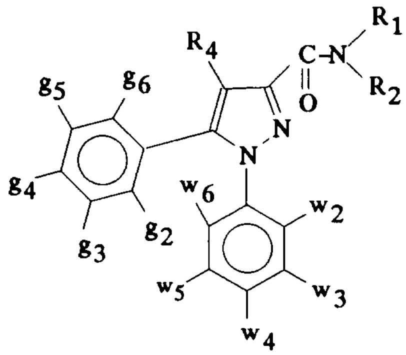

(Ia')

in which $w_2$, $w_3$, $w_4$, $w_5$, $w_6$, $g_2$, $g_3$, $g_4$, $g_5$, $g_6$ and $R_4$ are as defined for (I) in claim 1, $R_1$ is hydrogen or a ($C_1$-$C_6$) alkyl and $R_2$ is a non-aromatic $C_3$-$C_{15}$ carbocyclic radical or a saturated 5- to 8-membered heterocyclic radical selected from 1-pyrrolidinyl, 1-piperidinyl, 1-hexahydroazepinyl, 4-morpholinyl and 4-thiomorpholinyl, or one of its salts.

**3.** Compound according to claim 1 of the formula

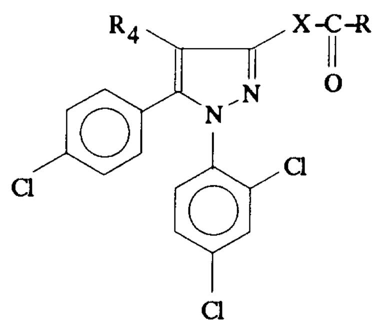

(i)

in which $R_4$, X and R are as defined for (I) in claim 1, or one of its salts.

**4.** Compound of formula (i) according to claim 3 in which $R_4$ is hydrogen or a methyl group, or one of its salts.

**5.** Compound of formula (i) according to claim 4 in which $R_3$ is hydrogen or methyl and X is a direct bond, or one of its salts.

**6.** Compound of formula (i) according to claim 3 in which $R_4$ is hydrogen or methyl, X is a direct bond and R is a group $-NR_1R_2$ in which $R_1$ is hydrogen or a methyl group and $R_2$ is a non-aromatic $C_3$-$C_{15}$ carbocyclic radical or a saturated 5- to 8-membered heterocyclic radical selected from 1-pyrrolidinyl, 1-piperidinyl, 1-hexahydroazepinyl, 4-morpholinyl and 4-thiomorpholinyl, or one of its salts.

**7.** Compound of formula (i) according to claim 3 in which $R_4$ is hydrogen or methyl, X is $-(CH_2)_x-N(R_3)-$ and R is $-NR_1R_2$, x being zero or one, $R_1$ being hydrogen, $R_3$ being hydrogen or a methyl group and $R_2$ being a phenyl which is unsubstituted or substituted by one or two halogen atoms, a ($C_1$-$C_5$) alkyl group or a ($C_1$-$C_5$) alkoxy group, or a non-aromatic $C_3$-$C_{15}$ carbocyclic radical, or one of its salts.

**8.** Compound according to claim 1 of the formula

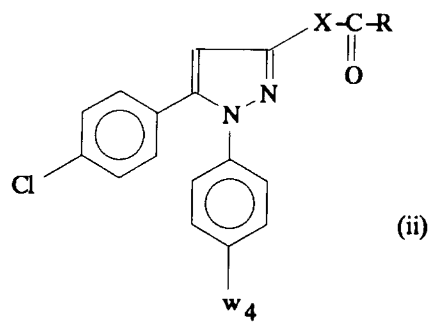

(ii)

in which X and R are as defined in claim 1 and $w_4$ is a methyl group or a methoxy group, or one of its salts.

9. Compound of formula (ii) according to claim 8 in which $w_4$ is methyl or methoxy, X is a direct bond and R is a group $-NR_1R_2$ in which $R_1$ is hydrogen or a methyl group and $R_2$ is a non-aromatic $C_3$-$C_{15}$ carbocyclic radical, or one of its salts.

10. Compound according to claim 8 in which $w_4$ is methyl or methoxy, X is a group $-(CH_2)_x-N(R_3)-$ in which x is zero or one and $R_3$ is hydrogen or a methyl group, and R is a group $-NR_1R_2$ in which $R_1$ is hydrogen and $R_2$ is a phenyl which is unsubstituted or substituted by one or two halogen atoms, a $(C_1$-$C_5)$ alkyl group or a $(C_1$-$C_5)$ alkoxy, or a non-aromatic $C_3$-$C_{15}$ carbocyclic radical, or one of its salts.

11. Compound according to claim 1 of formula (I) in which $w_2$, $w_3$, $w_4$, $w_5$, $w_6$, $g_2$, $g_3$, $g_4$, $g_5$, $g_6$, $R_4$ and X are as defined in claim 1 and R is a group $-NR_1R_2$ in which $R_1$ is hydrogen or a $(C_1$-$C_6)$ alkyl group and $R_2$ is a 2- or 3-indolyl $(C_1$-$C_3)$ alkyl group or a 2- or 3-indolyl group, or one of its salts.

12. Compound according to claim 1 of the formula

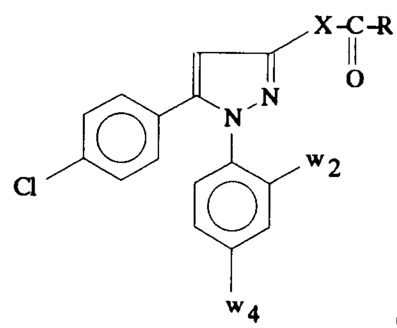

(iii)

in which X is as defined in claim 1, R is a group $-NR_1R_2$ in which $R_1$ is hydrogen or a $(C_1$-$C_6)$ alkyl and $R_2$ is a 2- or 3-indolyl $(C_1$-$C_3)$ alkyl group or a 2- or 3-indolyl group, and either $w_2$ is hydrogen and $w_4$ is a methyl or methoxy group or $w_2$ and $w_4$ are a chlorine atom, or one of its salts.

13. Compound according to claim 1 of the formula

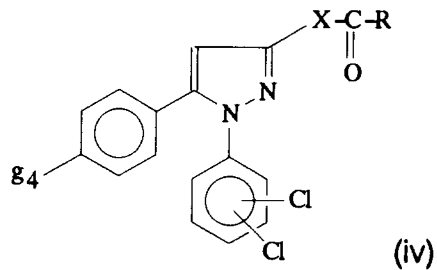

(iv)

in which X and R are as defined in claim 1 and $g_4$ is a bromine atom or a methyl or trifluoromethyl group, or one of its salts.

**14.** Method of preparing the compounds of formula (I) according to claim 1, characterized in that a pyrazole-3-carboxylic acid derivative of the formula

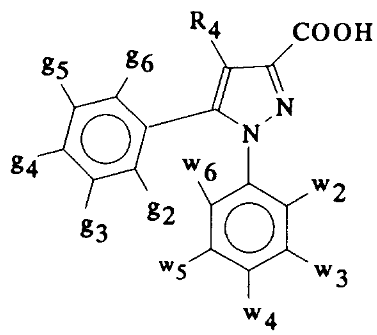

(IIa)

in which $w_2$, $w_3$, $w_4$, $w_5$, $w_6$, $g_2$, $g_3$, $g_4$, $g_5$, $g_6$ and $R_4$ are as defined for (I) in claim 1, or one of its activated forms, namely one of its esters or acid chlorides, is treated

. either with an amine of the formula $HNR_1R_2$, in which $R_1$ and $R_2$ are as defined for (I) in claim 1, to give the amides of the formula

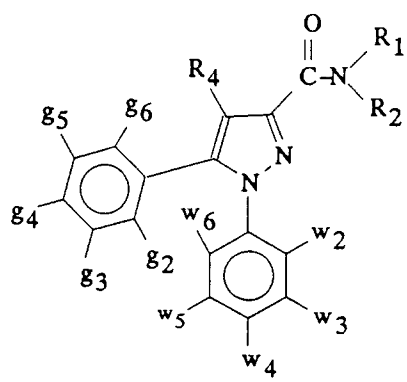

(Ia)

in which $w_2$, $w_3$, $w_4$, $w_5$, $w_6$, $g_2$, $g_3$, $g_4$, $g_5$, $g_6$, $R_4$, $R_1$ and $R_2$ are as defined for (I),

. or optionally with a primary amine $R_3NH_2$, in which $R_3$ is as defined for (I) in claim 1, to give the intermediate amides (V) of the formula

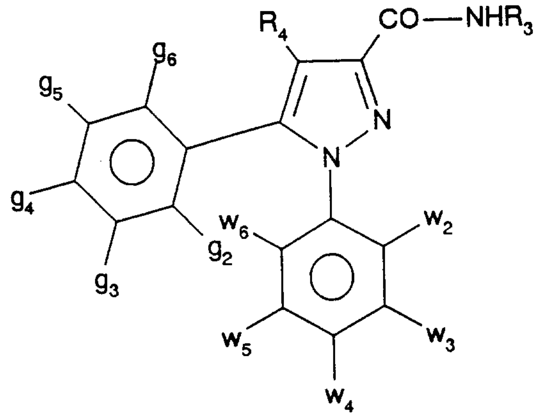

(V)

in which $w_2$, $w_3$, $w_4$, $w_5$, $w_6$, $g_2$, $g_3$, $g_4$, $g_5$, $g_6$, $R_4$ and $R_3$ are as defined for (I), to give, by reduction with a metal hydride, the intermediate amines of the formula

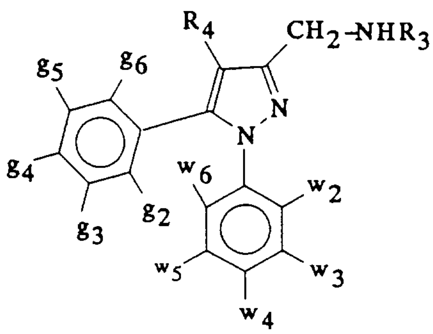

(VI)

in which $w_2$, $w_3$, $w_4$, $w_5$, $w_6$, $g_2$, $g_3$, $g_4$, $g_5$, $g_6$, $R_4$ and $R_3$ are as defined for (I),
which are converted to the amide or urea of the formula

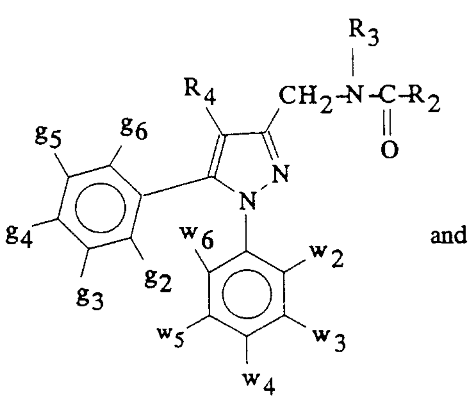

and

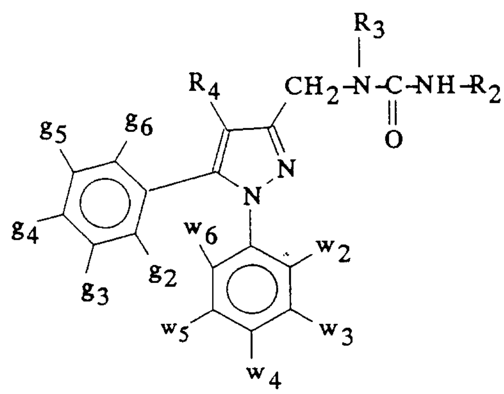

(Ib) (Ic)

in which $w_2$, $w_3$, $w_4$, $w_5$, $w_6$, $g_2$, $g_3$, $g_4$, $g_5$, $g_6$, $R_2$, $R_3$ and $R_4$ are as defined for (I), by reaction with an acid chloride of the formula $R_2COCl$ or, respectively, an isocyanate of the formula $R_2$-N=C=O, in which $R_2$ is as defined for (I),
or with a diphenylphosphorylazide derivative in a basic medium, followed by an acid treatment in an alcoholic medium, to give the intermediate amine of the formula

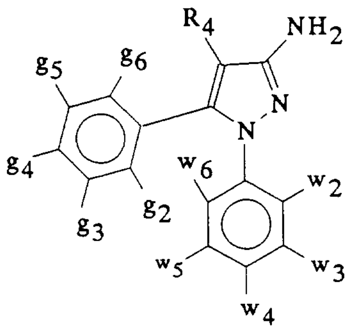

(VII)

in which $w_2$, $w_3$, $w_4$, $w_5$, $w_6$, $g_2$, $g_3$, $g_4$, $g_5$, $g_6$ and $R_4$ are as defined for (I), which is reacted with an acid chloride $R_2COCl$ or an isocyanate $R_2$-N=C=O to give respectively the amides and ureas of the formulae

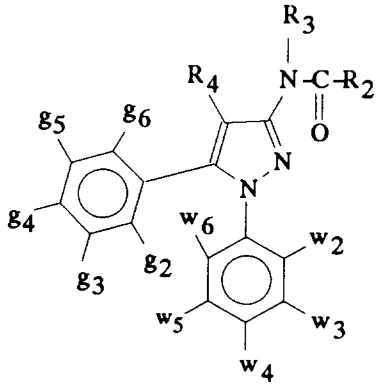

and

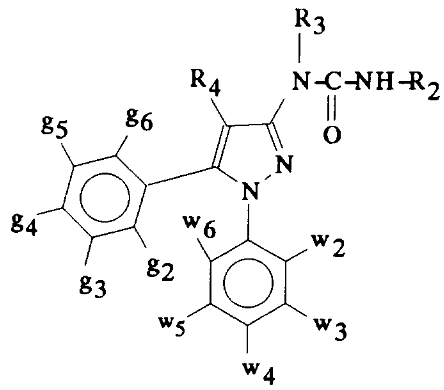

(Id) (Ie)

in which $w_2$, $w_3$, $w_4$, $w_5$, $w_6$, $g_2$, $g_3$, $g_4$, $g_5$, $g_6$, $R_2$ and $R_4$ are as defined for (I) and $R_3$ is hydrogen, the same compounds in which $R_3$ is other than hydrogen being prepared from the above primary amine (VII) converted to a secondary amine of the formula

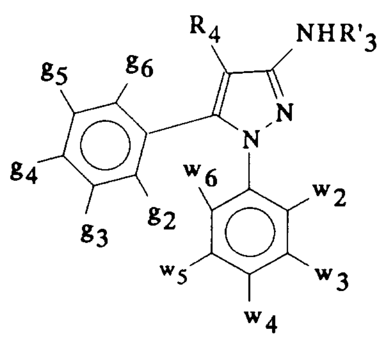

(VIIb)

in which $w_2$, $w_3$, $w_4$, $w_5$, $w_6$, $g_2$, $g_3$, $g_4$, $g_5$, $g_6$, and $R_4$ are as defined for (I) and $R'_3$ is a $(C_1-C_2)$ alkyl, which are then reacted with an acid chloride $R_2COCl$ or an isocyanate $R_2$-N=C=O to give the amides and ureas of formulae (Id) and (Ie) as defined above in which $R_3$ is other than hydrogen,

. or with an organomanganous reagent $R_5MnX_1$, in which $R_5$ is as defined for (I) in claim 1 and $X_1$ is a halogen, to give the ketone derivatives of the formula

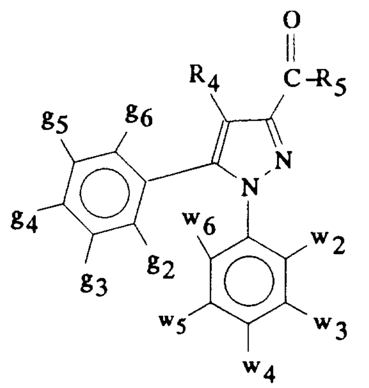

(If)

the resulting compounds then being optionally converted to one of their salts.

**15.** Compounds of the formulae

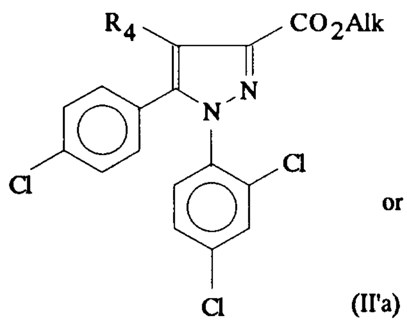

or

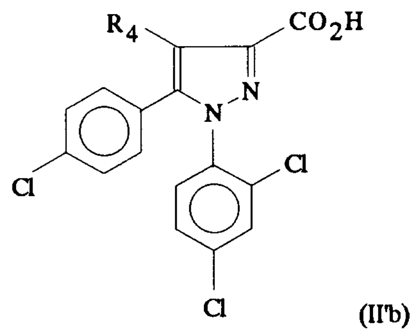

(II'a) (II'b)

in which $R_4$ is as defined in claim 1 and Alk is a ($C_1$-$C_5$) alkyl.

**16.** Pharmaceutical composition containing a compound of formula (I) according to one of claims 1 to 13 as the active principle.

**17.** Pharmaceutical composition according to claim 16 in the form of a dosage unit in which the active principle is mixed with at least one pharmaceutical excipient.

**18.** Composition according to claim 17 containing from 0.5 to 1000 mg of active principle.

**Patentansprüche**

**1.** Verbindung der Formel:

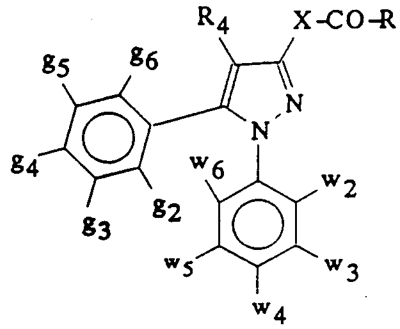

(I),

worin

- $g_2$, $g_3$, $g_4$, $g_5$, $g_6$ und $w_2$, $w_3$, $w_4$, $w_5$, $w_6$ gleich oder verschieden sind und unabhängig Wasserstoff, ein Chlor- oder Bromatom, $C_1$-$C_3$-Alkyl, $C_1$-$C_3$-Alkoxy, Trifluormethyl, eine Nitro-Gruppe bedeuten, und $g_4$ gegebenenfalls eine Phenyl-Gruppe darstellt;
- $R_4$ Wasserstoff oder $C_1$-$C_3$-Alkyl ist;
- X entweder eine direkte Bindung oder eine Gruppe $-(CH_2)_x-N(R_3)-$ darstellt, wobei $R_3$ Wasserstoff oder $C_1$-$C_3$-Alkyl bedeutet, und x Null oder 1 ist;
- R bedeutet:
  - eine Gruppe $-NR_1R_2$, wobei $R_1$ und $R_2$ unabhängig darstellen: $C_1$-$C_6$-Alkyl; einen gegebenenfalls substituierten, nicht-aromatischen $C_3$-$C_{15}$-cyclischen Kohlenwasserstoffrest, wobei der (die) Substituent(en) von Substituiertem Carbonyl verschieden ist (sind); eine Amino-$C_1$-$C_4$-alkyl-Gruppe, wobei Amino gegebenenfalls disubstituiert ist durch $C_1$-$C_3$-Alkyl; Cycloalkyl-$C_1$-$C_3$-alkyl, wobei Cycloalkyl $C_3$-$C_{12}$ ist; Phenyl, unsubstituiert oder substituiert einmal oder mehrmals durch ein Halogen, durch $C_1$-$C_5$-Alkyl oder durch $C_1$-$C_5$-Alkoxy; Phenyl-$C_1$-$C_3$-alkyl; Diphenyl-$C_1$-$C_3$-alkyl; Naphthyl; Anthracenyl; einen gesättigten heterocyclischen Rest mit 5 bis 8 Gliedern, unsubstituiert oder substituiert durch $C_1$-$C_3$-Alkyl, Hydroxyl oder Benzyl; 1-Adamantylmethyl; einen aromatischen Heterocyclus, unsubstituiert oder substituiert einmal oder mehrmals durch Halogen, $C_1$-$C_5$-Alkyl oder $C_1$-$C_5$-Alkoxy; $C_1$-$C_3$-Alkyl, substituiert durch einen aromatischen Heterocyclus, unsubstituiert oder substituiert einmal oder mehrmals durch Halogen, $C_1$-$C_5$-Alkyl oder $C_1$-$C_5$-Alkoxy; oder $R_1$ auch Wasserstoff darstellt, und $R_2$ wie oben definiert ist; oder auch $R_1$ und $R_2$ mit dem Stickstoffatom, an das sie gebunden sind, einen gesättigten heterocyclischen Rest mit 5 bis 8 Gliedern darstellen, wobei der heterocyclische Rest von Morpholinyl verschieden ist, wenn $w_2$, $w_3$, $w_4$, $w_5$, $w_6$, $g_2$, $g_3$, $g_4$, $g_5$, $g_6$ alle Wasserstoff bedeuten;
  - eine Gruppe $R_2$, wie oben definiert, wenn X $-(CH_2)_xN(R_3)-$ darstellt;
  - eine Gruppe $R_5$, wenn X eine direkte Bindung darstellt, wobei $R_5$ bedeutet: $C_1$-$C_3$-Alkyl; $C_3$-$C_{12}$-Cycloalkyl, unsubstituiert oder substituiert durch $C_1$-$C_5$-Alkyl; Phenyl-$C_1$-$C_3$-alkyl, unsubstituiert oder substituiert durch Halogen oder durch $C_1$-$C_5$-Alkyl; Cycloalkyl-$C_1$-$C_3$-alkyl, wobei Cycloalkyl $C_3$-$C_{12}$ ist und unsubstituiert oder substituiert ist durch $C_1$-$C_5$-Alkyl; 2-Norbornylmethyl;

oder eines ihrer Salze.

**2.** Verbindung nach Anspruch 1 der Formel:

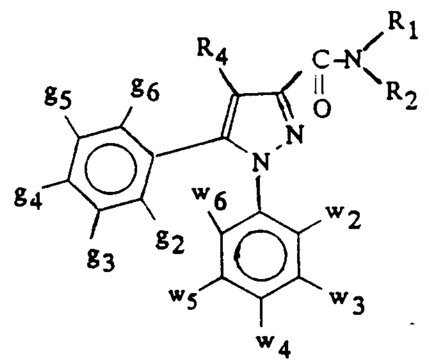

(Ia'),

worin $w_2$, $w_3$, $w_4$, $w_5$, $w_6$, $g_2$, $g_3$, $g_4$, $g_5$, $g_6$ und $R_4$ wie für (I) in Anspruch 1 definiert sind, $R_1$ Wasserstoff oder $C_1$-$C_6$-Alkyl bedeutet, und $R_2$ darstellt: einen nicht-aromatischen $C_3$-$C_{15}$-cyclischen Kohlenwasserstoffrest oder einen gesättigten heterocyclischen Rest mit 5 bis 8 Gliedern, ausgewählt aus 1-Pyrrolidinyl, 1-Piperidinyl, 1-Hexahydroazepinyl, 4-Morpholinyl und 4-Thiomorpholinyl, oder eines ihrer Salze.

3. Verbindung nach Anspruch 1 der Formel:

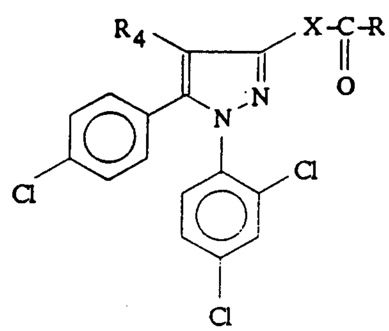

(i),

worin $R_4$, X und R wie für (I) in Anspruch 1 definiert sind, oder eines ihrer Salze.

4. Verbindung der Formel (i) nach Anspruch 3, worin $R_4$ Wasserstoff oder eine Methyl-Gruppe bedeutet, oder eines ihrer Salze.

5. Verbindung der Formel (i) nach Anspruch 4, worin $R_3$ Wasserstoff oder Methyl bedeutet, und X eine direkte Bindung darstellt, oder eines ihrer Salze.

6. Verbindung der Formel (i) nach Anspruch 3, worin $R_4$ Wasserstoff oder Methyl ist, X eine direkte Bindung darstellt, und R bedeutet: eine Gruppe $-NR_1R_2$, wobei $R_1$ Wasserstoff oder eine Methyl-Gruppe darstellt, und $R_2$ bedeutet: einen nicht-aromatischen $C_3$-$C_{15}$-cyclischen Kohlenwasserstoffrest oder einen gesättigten heterocyclischen Rest mit 5 bis 8 Gliedern, ausgewählt aus 1-Pyrrolidinyl, 1-Piperidinyl, 1-Hexahydroazepinyl, 4-Morpholinyl und 4-Thiomorpholinyl, oder eines ihrer Salze.

7. Verbindung der Formel (i) nach Anspruch 3, worin $R_4$ Wasserstoff oder Methyl ist, X $-(CH_2)_x-N(R_3)-$ darstellt, R $-NR_1R_2$ ist, wobei x Null oder 1 ist, $R_1$ Wasserstoff bedeutet, $R_3$ Wasserstoff oder eine Methyl-Gruppe darstellt, und $R_2$ bedeutet: Phenyl, unsubstituiert oder substituiert durch ein oder zwei Halogenatome, eine $C_1$-$C_5$-Alkyl-Gruppe oder eine $C_1$-$C_5$-Alkoxy-Gruppe oder einen nicht-aromatischen $C_3$-$C_{15}$-cyclischen Kohlenwasserstoffrest, oder eines ihrer Salze.

8. Verbindung nach Anspruch 1 der Formel:

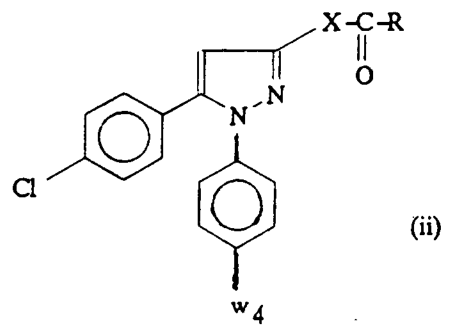

(ii),

worin X und R wie in Anspruch 1 definiert sind, und $w_4$ eine Methyl-Gruppe oder eine Methoxy-Gruppe bedeutet, oder eines ihrer Salze.

9. Verbindung der Formel (ii) nach Anspruch 8, worin $w_4$ Methyl oder Methoxy bedeutet, X eine direkte Bindung darstellt, und R eine Gruppe $-NR_1R_2$ ist, wobei $R_1$ Wasserstoff oder eine Methyl-Gruppe darstellt und $R_2$ einen nicht-aromatischen $C_3$-$C_{15}$-cyclischen Kohlenwasserstoffrest bedeutet, oder eines ihrer Salze.

10. Verbindung nach Anspruch 8, worin $w_4$ Methyl oder Methoxy bedeutet, X eine Gruppe $-(CH_2)_x-N(R_3)-$ darstellt, wobei x Null oder 1 ist, und $R_3$ Wasserstoff oder eine Methyl-Gruppe darstellt, und R eine Gruppe $-NR_1R_2$ bedeutet, wobei $R_1$ Wasserstoff darstellt, und $R_2$ bedeutet: Phenyl, unsubstituiert oder substituiert durch ein oder zwei Halogenatome, eine $C_1$-$C_5$-Alkyl-Gruppe oder eine $C_1$-$C_5$-Alkoxy-Gruppe oder einen nicht-aromatischen $C_3$-$C_{15}$-cyclischen Kohlenwasserstoffrest bedeutet, oder eines ihrer Salze.

11. Verbindung nach Anspruch 1 der Formel (I), worin $w_2$, $w_3$, $w_4$, $w_5$, $w_6$, $g_2$, $g_3$, $g_4$, $g_5$, $g_6$, $R_4$ und X wie in Anspruch 1 definiert sind, und R eine Gruppe $-NR_1R_2$ ist, wobei $R_1$ Wasserstoff oder eine $C_1$-$C_6$-Alkyl-Gruppe darstellt, und $R_2$ eine 2- oder 3-Indolyl-$C_1$-$C_3$-alkyl-Gruppe oder eine 2- oder 3-Indolyl-Gruppe bedeutet, oder eines ihrer Salze.

12. Verbindung nach Anspruch 1 der Formel:

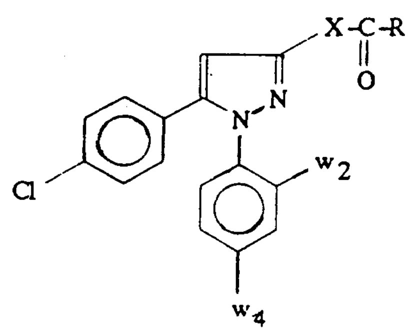

(iii),

worin X wie in Anspruch 1 definiert ist, R eine Gruppe $-NR_1R_2$ bedeutet, wobei $R_1$ Wasserstoff oder $C_1$-$C_6$-Alkyl darstellt, und $R_2$ eine 2- oder 3-Indolyl-$C_1$-$C_3$-alkyl-Gruppe oder eine 2- oder 3-Indolyl-Gruppe ist, und entweder $w_2$ Wasserstoff bedeutet, und $w_4$ eine Methyl- oder Methoxy-Gruppe darstellt, oder $w_2$ und $w_4$ ein Chloratom sind, oder eines ihrer Salze.

13. Verbindung nach Anspruch 1 der Formel:

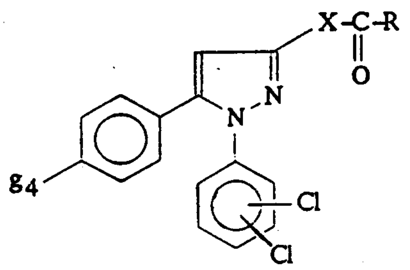

(iv),

worin X und R wie in Anspruch 1 definiert sind, und $g_4$ ein Bromatom, eine Methyl- oder Trifluormethyl-Gruppe bedeutet, oder eines ihrer Salze.

**14.** Verfahren zur Herstellung von Verbindungen der Formel (I) nach Anspruch 1, dadurch gekennzeichnet, daß ein Derivat von Pyrazol-3-carbonsäure der Formel:

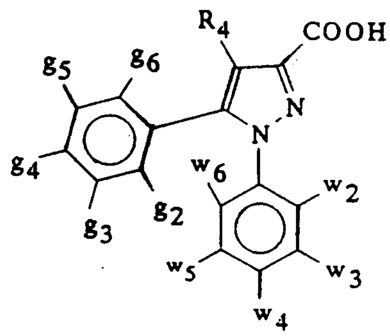

(IIa),

worin $w_2$, $w_3$, $w_4$, $w_5$, $w_6$, $g_2$, $g_3$, $g_4$, $g_5$, $g_6$ und $R_4$ wie für (I) in Anspruch 1 definiert sind, oder eine ihrer aktivierten Formen, d.h. einer ihrer Ester oder Säurechloride, behandelt wird mit

- entweder einem Amin der Formel $HNR_1R_2$, worin $R_1$ und $R_2$ wie für (I) in Anspruch 1 definiert sind, wobei Amide der Formel erhalten werden:

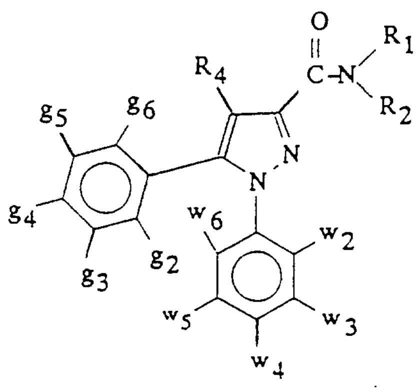

(Ia),

worin $w_2$, $w_3$, $w_4$, $w_5$, $w_6$, $g_2$, $g_3$, $g_4$, $g_5$, $g_6$, $R_4$, $R_1$ und $R_2$ wie für (I) definiert sind,
- oder gegebenenfalls einem primären Amin $R_3NH_2$, worin $R_3$ wie für (I) in Anspruch 1 definiert ist, wobei Zwischen-Amide (V) der Formel erhalten werden:

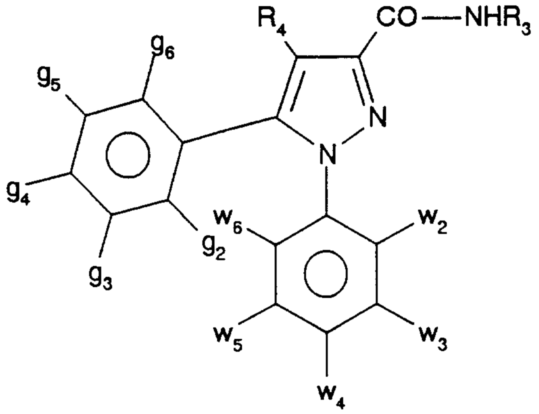

(V),

worin $w_2$, $w_3$, $w_4$, $w_5$, $w_6$, $g_2$, $g_3$, $g_4$, $g_5$, $g_6$, $R_4$ und $R_3$ wie für (I) definiert sind, wobei durch Reduktion mit einem Metallhydrid Zwischen-Amine der Formel erhalten werden:

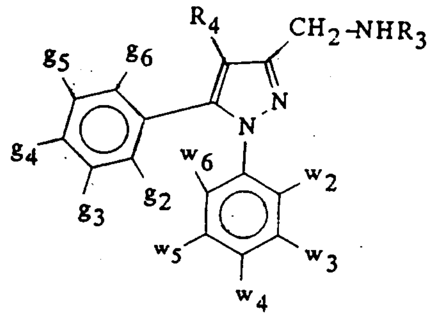

(VI),

worin $w_2$, $w_3$, $w_4$, $w_5$, $w_6$, $g_2$, $g_3$, $g_4$, $g_5$, $g_6$, $R_4$ und $R_3$ wie für (I) definiert sind,
die in ein Amid oder einen Harnstoff der Formel transformiert werden:

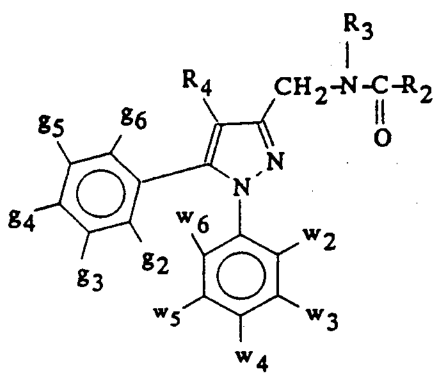

(Ib)

und

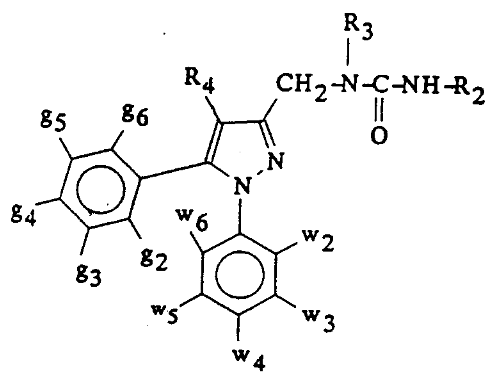

(Ic)

,

worin $w_2$, $w_3$, $w_4$, $w_5$, $w_6$, $g_2$, $g_3$, $g_4$, $g_5$, $g_6$, $R_2$, $R_3$ und $R_4$ wie für (I) definiert sind, durch Umsetzen mit einem Säurechlorid der Formel $R_2COCl$ bzw. ein Isocyanat der Formel $R_2$-N=C=O, wobei $R_2$ wie für (I) definiert ist,

- oder einem Derivat von Diphenylphosphorylazid in einem basischen Medium, gefolgt von einer Säurebehandlung in einem alkoholischen Medium, wobei das Zwischen-Amin der Formel erhalten wird:

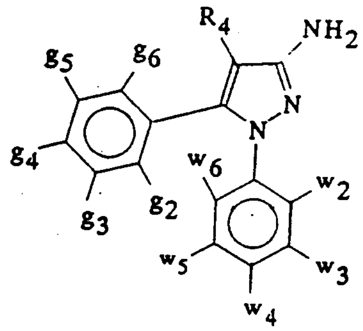

(VII),

worin $w_2$, $w_3$, $w_4$, $w_5$, $w_6$, $g_2$, $g_3$, $g_4$, $g_5$, $g_6$ und $R_4$ wie für (I) definiert sind, das der Einwirkung eines Säurechlorids $R_2COCl$ oder eines Isocyanats $R_2$-N=C=O unterworfen wird, wobei Amide bzw. Harnstoffe der Formel erhalten werden:

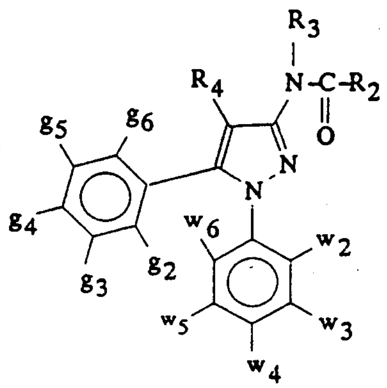

(Id)

und

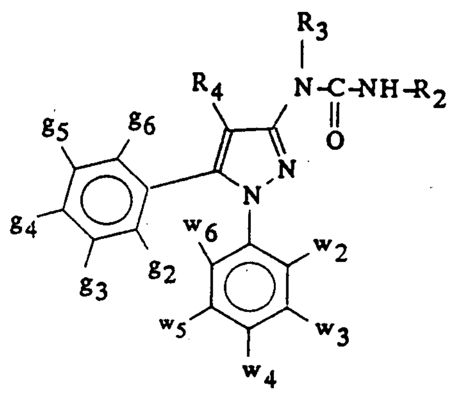

(Ie)

,

worin $w_2$, $w_3$, $w_4$, $w_5$, $w_6$, $g_2$, $g_3$, $g_4$, $g_5$, $g_6$, $R_2$ und $R_4$ wie für (I) definiert sind, und $R_3$ Wasserstoff bedeutet, wobei die gleichen Verbindungen, für die $R_3$ von Wasserstoff verschieden ist, aus dem obigen primären Amin (VII) hergestellt werden, das in ein sekundäres Amin der Formel transformiert wird:

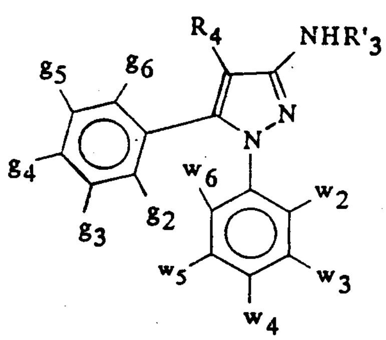

(VIIb),

worin $w_2$, $w_3$, $w_4$, $w_5$, $w_6$, $g_2$, $g_3$, $g_4$, $g_5$, $g_6$ und $R_4$ wie für (I) definiert sind, und $R'_3$ $C_1$-$C_2$-Alkyl bedeutet, die anschließend der Einwirkung eines Säurechlorids $R_2COCl$ oder eines Isocyanats $R_2$-N=C=O unterworfen werden, wobei Amide und Harnstoffe der Formel (Id) und (Ie), wie oben definiert, worin $R_3$ von Wasserstoff verschieden ist, erhalten werden,

- oder einem Organomangan-Reagens $R_5MnX_1$, worin $R_5$ wie für (I) in Anspruch 1 definiert ist, und $X_1$ Halogen bedeutet, wobei Keton-Derivate der Formel erhalten werden:

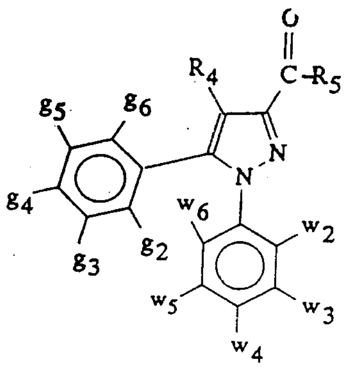

(If),

und wobei die so erhaltenen Verbindungen anschließend gegebenenfalls in eines ihrer Salze transformiert werden.

**15.** Verbindungen der Formel:

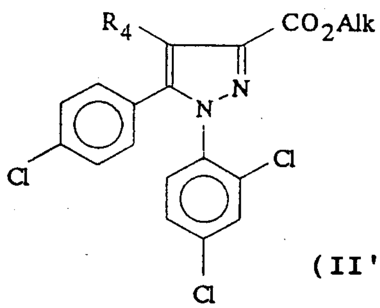

(II'a)

oder

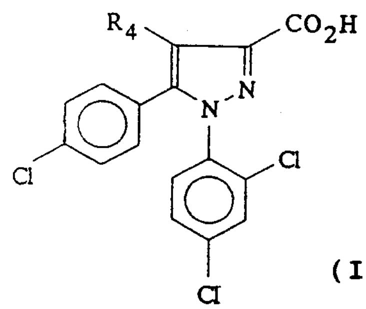

(II'b)

,

worin $R_4$ wie in Anspruch 1 definiert ist, und Alk $C_1$-$C_5$-Alkyl bedeutet.

**16.** Pharmazeutische Zusammensetzung, welche als aktiven Bestandteil eine Verbindung der Formel (I) nach einem der Ansprüche 1 bis 13 enthält.

**17.** Pharmazeutische Zusammensetzung nach Anspruch 16 in Einheitsdosierungsform, worin der aktive Bestandteil mit zumindest einem pharmazeutischen Exzipienten gemischt ist.

**18.** Pharmazeutische Zusammensetzung nach Anspruch 17, welche 0,5 bis 1000 mg aktiven Bestandteil enthält.